# EUROPEAN PATENT APPLICATION

(11) **EP 2 465 529 A2**
(43) Date of publication of application: **20.06.2012**
(21) Application number: 11152812.1
(22) Date of filing: 20.09.2004
(51) Int. Cl.: A61K 39/00

(54) **Educated NKT cells and their uses in the treatment of immune-related disorders.**

(30) Priority: 30.09.2003 US 676045
(62) Divisional of application: 04788871.4
(71) Applicant: Enzo Therapeutics, Inc. a fully owned subsidiary of Enzo Biochem, Inc., New York, NY 10022 (US)
(72) Inventor: Ilan, Yaron, 96400, Jerusalem (IL); Margalit, Maya, 93721, Jerusalem (IL); Elinav, Eran, 93715, Jerusalem (IL)
(74) Representative: Vossius & Partner

(57) **Abstract**

The present invention relates to a method for the treatment of immune-related or immune-mediated disorders in a mammalian subject in need of such treatment. This method comprises the step of manipulating the NKT cell population in said subject by suitable means, said manipulation of the NKT cell population resulting in modulation of the Th1/Th2 balance toward anti-inflammatory cytokine producing cells. Manipulation of the NKT cell population may be performed either by depletion of said cells by a suitable means or alternatively by *ex vivo* education of the NKT cells, such that the educated NKT cells have the capability to modulate the Th1/Th2 balance toward anti-inflammatory cytokine producing cells. The invention further relates to pharmaceutical compositions for the treatment of immune-related or immune-mediated disorders in a mammalian subject. These compositions comprising as an effective ingredient an *ex vivo* educated NKT cell. The invention further provides for an *ex vivo* educated NKT cell and uses thereof in the treatment of immune-related or immune-mediated disorders.

## Description

### REFERENCE TO RELATED PATENT APPLICATIONS

This application is a continuation-in-part of U.S. Patent Application entitled "Educated NKT Cells and Their Uses in the Treatment of Immune-Related Disorder" by Yaron Ilan, et al., (Application No. not yet assigned) filed on June 25, 2003, which is a 371 of international application No. PCT/ILO1 /01197, filed on December 24, 2001. The contents of the aforementioned patent applications are hereby incorporated by reference, in their entirety.

### FIELD OF THE INVENTION

The present invention relates to the field of therapeutic methods, compositions and uses thereof, in the treatment of immune-related or immune-mediated disorders in mammalian subjects. More particularly, the methods and the compositions of the invention are directed to manipulation of the NKT cell population in a subject, which results in modulation of the Th1/Th2 cell balance toward anti-inflammatory or pro-inflammatory cytokine producing cells, and to their use in the treatment of immune related disorders.

All patents, patent applications, patent publications, scientific articles, and the like, cited or identified in this application are hereby incorporated by reference in their entirety in order to describe more fully the state of the art to which the present invention pertains.

### BACKGROUND

The immune system is responsible for a major part of the defense against potentially harmful agents. However, this system may turn against self-antigens and bring about autoimmune disorders such as inflammatory bowel disease. These disorders can be perceived as a dysbalance between pro-inflammatory (Th1) and anti-inflammatory (Th2) cytokines.

Overcoming the immune response tends to involve generalized immunosuppression which can often lead to undesirable side effects. Thus, there is a need for an alternative strategy for induction of antigen-specific immune suppression. Immune tolerance can be induced by two types of mechanisms. The first, termed "recessive", involves clonal anergy or deletion of the vast majority of the immunocytes that are capable of responding to the antigen [Matzinger, P. et al., Ann. Rev. Immunol. 12:991-1045 (1994); Qin, S., et al., Science 259:974-977 (1993)]. Alternatively, in a "dominant" type of tolerance, negative immunoregulatory lymphocytes may emerge as a result of tolerization procedures. In contrast to clonal deletion or anergy, the presence of a limited number of these lymphocytes may down regulate a much larger number of effector cells.

### The Role of the Immune System in the Pathogenesis of Inflammatory Bowel Disease

Inflammatory bowel diseases (IBD) are common gastrointestinal disorders that can be perceived as being the result of a dysbalance between Th1-pro-inflammatory and Th2-anti-inflammatory subtypes of immune responses [Strober, W., et al., Immunol Today 18:61-64 (1997); Neurath, M., et al., J. Exp. Med. 183:2605-2616 (1996)].

There are several extra-intestinal manifestations that accompany IBD. For example, autoimmune phenomena, the formation of immune complexes having a role in target organ damage and immunosuppressive agents such as glucocorticoids, azathioprine, methotrexate and cyclosporine, which are used to alleviate the disease [Podolsky, D.K., et al., New Engl. J. Med., 325:928-935(1991); Strober, W., et al., In Clinical Immunology, Mosby, St. Louis. R.R. Rich, Editor, 1401-14281-2 (1995)]. Patients with IBD have antibodies against components of colon cells and several different bacterial antigens. These antigens gain access to the immune system as a consequence of epithelial damage [Hibi, S., et al., Clin. Exp. Immunol. 54:163-168 (1983); Das, K.M., et al., Gastroenterology 98:464-69 (1990)]. Abnormalities of T cell-mediated immunity, including coetaneous anergy and diminished responsiveness to T cell stimuli, have also been described in these patients [Chiba, M., et al. Gut, 22:177-182 (1981); Raedler, A., et al., Clin. Exp. Immunol. 60:518-526 (1985)]. In addition, changes in mucosal cell mediated immunity were identified, including increased concentrations of mucosal IgG cells and changes in T cell subsets, suggesting antigen stimulation [Dasgupta, A., et al., Gut 35:1712-17 (1994); Takahashi, F., et al., J. Clin. Invest. 76:31 1-318 (1985)]. Exposure of target antigens after infectious, immune, or toxic damage leads to activation of mucosal immune cells resulting in cytokines that lead to mucosal inflammatory response [Neurath, M., et al., J. Exp. Med., 183:2605-2616 (1996)]. The secretion of pro-inflammatory cytokines such as IFNγ contribute to an increase in mucosal permeability, and has been described in animal models of IBD [Strober, W., et al., Immunol. Today 18:61-64. (1997)]. Similarly, an increase in collagen synthesis mediated by IL1 and IL6 can be detected in these animals [Strober, W., *et al., ibid.*]. A Th1-mediated granulomatous colitis model has been established by the adoptive transfer of normal CD45RB T cells from Balb/C mice into CB-17 scid mice. CD4 cells from CD45RB were shown to prevent the disease when injected together with the CD45RB population. This prevention could be reversed by adding antibodies to TGFβ1 [Sadlack, B., et al., Cell 75:253-261 (1993); Powrie, F., et al., Immunity 1 :553-562 (1994)].

### The Th1/Th2 Dysbalance in Inflammatory Bowel Disease

Both CD4 and CD8 lymphocytes can be typed as either Th1 cells that produce IL-2 and IFNγ, or Th2 cells that produce IL-4 and IL-10. The way the immune system responds to foreign and self-antigens is the result of a balance between the two subtypes of responses [Weiner, H.L., et al., Immunol. Today 18: 335-343 (1997); Adorini, L., et al., Immunol. Today 18:209-211 (1997); Rabbani, E. *et al.,* European Patent Publication No. EP1149586A1 (filed on April 27, 2001), herein incorporated by reference]. A Th1 type response is involved in the pathogenesis of several autoimmune and chronic inflammatory disorders such as IBD [Adorini, L., *et al.,* (1997) *ibid.;* Mizoguchi, A., et al., J. Exp. Med. 183:847-856, (1996)]. Thus, experimental colitis and IBD in humans can be perceived as a dysbalance between pro-inflammatory Th1-type and anti-inflammatory Th2-type cytokines. It has been recently shown, in both animals and humans, that anti-inflammatory cytokines such as IL10 can downregulate the pro-inflammatory effects of Th1-mediated cytokines, thereby alleviating immune-mediated disorders [Mizoguchi, A., *et al.,* (1996) *ibid.;* Madsen, K.L., et al., Gastroenterology 113:151-159 (1997); Van Deventer Sander, J., et al., Gastroenterology 113:383-389 (1997)].

### Oral Tolerance Induction (Oral Immune Regulation) for the Amelioration of Immune-Mediated Disorders

Oral tolerance (Oral Immune Regulation) is a recognized procedure for the induction of antigen-specific peripheral immune hyporesponsiveness [Weiner, H.L., *et al.,* (1997) *ibid.;* Weiner, H., Proc. Natl. Acad. Sci. USA 91:10762-10765 (1994); Roy-Chowdury, *et al.,* International Publication No. WO 98/37917 (filed February 26, 1998), herein incorporated by reference]. Oral administration of antigens has been shown, both in animals and humans, to prevent or alleviate several autoimmune disorders such as collagen-induced arthritis, uveitis, diabetes, and experimental allergic encephalomyelitis [Esbjorn, T., et al., Int. Arch. Allergy Immunol. 113:219-223 (1997);, Von Herrath, M.G., et al., J. Clin. Inves. 98:1324-1331 (1996); Hancock, W., et al., Am. J. Path. 147:1193-1197 (1993); Weiner, H.L., et al., Science 261 :1321-1 324 (1993)].

Enteral exposure to high doses of the antigens induces tolerance by clonal inactivation of antigen specific T cells, while the feeding of low doses of the antigens leads to induction of regulatory cell secreting factors that suppress the generation of antigen-specific effector cells [Weiner, H.L., *et al.,* (1997) *ibid.].* Both in animals and humans, tolerance induction is associated with a Th2/Th3 type immune response leading to the secretion of immunosuppressive cytokines such as IL10, IL4 and TGFβ1 [Weiner, H.L., *et al.,* (1997) *ibid.].* A bystander effect involving reactivity to multiple closely-related-antigens was shown to play a role in oral tolerance induction in several models [Weiner, H.L., *et al.,* (1997) *ibid.;* Carvalho, B.A., et al., Scand J. Immunol. 45: 276-281 (1997)]. As regulatory cells secrete non-antigen specific cytokines after being triggered by a fed antigen, they can suppress inflammation in the microenvironment where the fed antigen is localized. Although the procedure is well established as a method for immune tolerance induction, the exact mechanism has yet to be discovered. Conflicting results have been published as to whether an antigen has to be processed and/or absorbed, and whether protein denaturation is necessary for tolerance induction [Carvalho, B.A., *et al.,* (1997) *ibid.;* Blanas, E., et al., Science 274:1707-1709 (1996)].

Antigen presentation may require whole proteins to be presented into the bowel. However, protein processing and absorption may also be involved in tolerance induction or in its maintenance through post-gut mechanisms [Carvalho, B.A., *et al.,* (1997) *ibid.*]. Gut wall epithelial cells, Peyer's patches, mesenteric lymph node, or extraintestinal cells have been suggested as mediating immune tolerance induction [Strober, W., *et al.,* (1997) *ibid.].* However, oral administration of an antigen can also elicit an epitope-specific immunity [Carvalho., B.A., *et al.,* (1997) *ibid.;* Blanas, E., *et al* (1996) *ibid.].* Indeed, side by side with immunosuppressive-cytokine-secreting cells (e.g. Th3 cells secreting TGFβ) that appear after oral tolerization, a second population of cells, secreting pro-inflammatory-cytokines (e.g. IFNγ,) can be found in the gut wall, mainly in Peyer's patches [Weiner, H.L., *et al.,* (1997) *ibid.].* Orally administered antigen elicits a local pro-inflammatory response, IFNγ-mediated, in the gut mucosa, along with a systemic TGFβ and IL4-mediated anti-inflammatory response. In contrast to splenocytes from orally tolerized animals, gut extracted lymphocytes have been unable to transfer the tolerance into naive animals [Strober, W., *et al.,* (1997) *ibid.;* Weiner, H.L,. *et al.,* (1997) *ibid.*]. Thus, induction of oral tolerance requires a balance between an immunogenic and a tolerogenic cell population, with a shift from a Th1 (and secretion of pro-inflammatory cytokines), to a Th2 (and secretion of anti-inflammatory cytokines) immune response.

It has been shown by others and the present inventors that oral tolerance can be used to prevent or alleviate experimental colitis in a model system that employs mice treated with 2,4,6-trinitrobenzenesulfonic acid (TNBS) [Madsen, K.L., et al., Gastroenterology 113:151-159 (1997); Trop, S., et al., Hepatology 27:746-755 (1999)]. Induction of oral tolerance to colitis extracted proteins downregulates the anti-colon immune response, thereby ameliorating the immune-mediated-colitis. Suppressor lymphocytes mediate the tolerance by induction of a shift from a pro-inflammatory to an anti-inflammatory immune response [Madsen, K.L., *et al.,* (1997) *ibid.;* Trop, S., *et al., ibid.*].

### The Role of the Liver in Immune Tolerance Induction

The liver has long been suggested to be involved in immunoregulatory functions. It is the largest reticuloendothelial organ in the body, and several subpopulations of its cells are involved in antigen presentation and/or processing [Callery, M.P., et al., J. Surg. Res. 46:391-394 (1989); Nakano, Y., et al., Surgery 1 11:668-676 (1 992); Yu, S.Y., et al., Surgery 1 16:229-234 (1 994)].

Portocaval shunts, or blockage of Kupffer cell functions have precluded induction of oral tolerance in several animal, models [Callery, M.P., *et al.,* (1989) *ibid.;* Nakano, Y., *et al.,* (1992) *ibid.;* Yu, S.Y., *et al.,* (1994) *ibid*.]. Antibody titers to intestinal flora were found to be elevated in humans with chronic liver diseases that underwent portocaval shunts [Crispe, N., et al., Immun. Today 11:236-245 (1996); Ilan, Y., et al., Gastro 114:260 (1998)]. Portal vein administration of donor cells has been shown to promote allo-specific hyporesponsiveness [Crispe, N., *et al.,* (1996) *ibid.].* Thus, the liver may be necessary for peripheral immune tolerance induction through first pass clearance of specific subpopulations of cells or peptides.

The present examples show that oral immune regulation induction by continuous feeding with mice liver extract ameliorates ob/ob mouse glucose intolerance, reduces their hepatic fat content, while renering them vulnerable to hepatic injury mediated by Concanavalin-A. This mechanism involves a Th1/Th2 shift response.

### Liver-Associated Lymphocytes

The adult liver contains several subpopulations of cells involved in its immunomodulatory functions. Kupffer cells were found important in front line defense against antigens entering the liver through portal circulation. Antigen-activated Kupffer cells have antigen presentation, phagocytosis, and have exhibited killing properties via secretion of cytokines. These cells also induce chemotaxis and lymphocyte aggregation [Crispe, N., *et al.,* (1996) *ibid.].* In addition, the adult liver contains pluripotent stem cells, giving rise to multiple cell lineages including thymic and extrathymic T cells, granulocytes, and erythroid lineage cells [Crispe N, *et al.,* (1996) *ibid.].* Indeed, T cells can differentiate extrathymically in an adult liver [Collins C., et al., Eur. J. Immunol. 26:3114-3118 (1996)].

The liver appears to be the meeting place for two populations of T cells consisting of thymus derived T cells with high TCR (TCR^{high}) and extrathymic T cells with intermediate TCR (TCR^{int}). The first set of T cells, also known as mainstream T cells, contains a mixture of minor populations of CD4⁺ and CD8⁺ cells, and a large population of CD4⁻CD8⁻ double negative (DN) cells, that do not express NK cell markers or IL2Rβ, and which are closely linked to the circulating T cell pool [Crispe, N., *et al.,* (1996) *ibid*.]. Many of the DN cells express a B cell marker, B220, the induction of which leads to the trafficking of apoptosing T cells to the liver [Crispe, N., *et al.,* (1996) *ibid.;* Ilan, Y., *et al.,* (1998) *ibid.;* Collins, C., *et al.,* (1996) *ibid.;* Garcia-Barcina, M., et al., Immunol 82:95-8 (1994); MacDonald, R.H., et al., J. Exp. Med. 182:633-638 (1995)]. The second subset of liver T cells, known as alternative T cells, are CD4⁺, or CD4-8⁻ and CD16⁻, express αβTCR^{int}; and known NK receptors including NKR-P1, Ly-49A, and IL2 receptor β-chain [Garcia-Barcina, M., *et al.,* (1994) *ibid.,* MacDonald, R.H., *et al.,* (1995) *ibid.;* Bendelac, A., et al., Curr. Opin. in Immunol. 7:367-374. (1995)]. The majority of the liver IL2Rβ⁺ TCR^{int} cells are NK1.1⁺. These cells are rare in the pool that circulates through the peripheral lymphoid organs. A small population of these cells, however, is present in the thymic medulla, spleen, and bone marrow. TCR^{int} IL2Rβ⁺ NK1.1 + cells differentiate through primordial pathway, thymic and extrathymic alternative pathways, rather than through the conventional thymic pathway, and can develop in livers of thymectomized animals [MacDonald, R.H., *et al.,* (1995) *ibid.;* Bendelac, A., *et al.,* (1995) *ibid.;* Takahashi, M., et al., J. Immunol. 156: 2436-2442 (1996); Doherty, D.G., et al., Hepatology 26:445A (1997)]. Their functions are not characteristic of those of any subset of conventional T cells, but include elements of cytotoxicity and B cell help. Upon primary activation they release a large variety of cytokines of both Th1 and Th2 origin [MacDonald, R.H., *et al.,* (1995) *ibid.;* Bendelac, A., *et al.,* (1995) *ibid.;* Takahashi, M., *et al.,* (1996) *ibid.;* Doherty, D.G., *et al.,* (1997) *ibid.].* They also respond to IL12 and produce IFNγ, both of which are Th1 cytokines, inducing anti-tumor and anti-microbial effector cells [Takahashi, M., *et al.,* (1996) *ibid.*; Doherty, D.G., *et al.,* (1997) *ibid.].* In addition, in the liver these cells multiply in response to IL12 and TNFα, and may be actively involved in lethal hit to mainstream T cells during peripheral deletion [Takahashi, M., *et al.,* (1996) *ibid.*; Doherty, D.G., *et al.,* (1997) *ibid.*].

One of the objects of the present invention is to determine the role of NK1.1⁺ lymphocytes in peripheral immune tolerance induction, in induction of tolerance and/or inflammation via adoptive transfer of splenocytes, specifically in keeping the balance between immunogenic and tolerogenic subsets of lymphocytes. The results of the present study show for the first time, that NK1.1⁺ lymphocytes may play a dual role in immune mediated disorders. In a "tolerized environment", they induce and/or maintain immune hyporesponsiveness via alteration of the Th1/Th2 paradigm in the anti-inflammatory direction. On the other hand, in a "non-tolerized environment", they support a pro-inflammatory paradigm. This and other objects of the invention will become clearer as the description proceeds.

Various methods have been described for the treatment of immune-related or immune mediated disorders or diseases, infectious diseases, metabolic disorders and different types of cancer in mammalian subjects. One of these methods involves the modulation of immune responses in a subject. This includes the down regulation of the immune response system using procedures or combinations of procedures for producing and applying a new and unexpected immune modulation termed selective immune down regulation (SIDR). Immunological modulation is an artificially induced variation in a subject's immune system in response to the introduction of reagents, procedures and processes. These procedures have been described in detail in U.S. Patent Application No. 08/808,629, filed on February 28, 1997, U.S. Patent Application No. 10/377,628, filed on March 4, 2003, U.S. Application No. 10/377, 603, filed on March 4, 2003, U.S. Patent Application No. 09/447,704, filed on February 28, 1997, U.S. Application No. 10/385,440, filed on May 9, 2001, and U.S. Application No. 09/356,294, filed on July 16, 1999. Each of the foregoing patents are incorporated by reference in their entirety in the present application and may further be used in conjunction with the present invention.

### SUMMARY OF THE INVENTION

In a first aspect, the invention relates to a method for the treatment of immune-related or immune-mediated disorders in a mammalian subject in need of such treatment, by manipulating the NKT cell population in said subject by suitable means, said manipulation of the NKT cell population resulting in modulation of the Th1/Th2 cell balance toward anti-inflammatory or pro-inflammatory cytokine producing cells.

In a preferred embodiment, the invention relates to method of manipulating the NKT cell population by depletion of said cells. As a specifically preferred embodiment, depletion of the NKT cell population may be performed by administering to the subject a therapeutically effective amount of a composition comprising as the effective ingredient an antibody that specifically recognizes the NKT cells. Alternatively, depletion of the NKT cell population may be performed by *ex vivo* pheresis, using beads coated with an antibody that specifically recognizes the NKT cells.

In an alternatively preferred embodiment, the invention relates to a method for the treatment of immune-related or immune-mediated disorders in a mammalian subject, this method involving manipulation of NKT cell population by *ex vivo* education of said NKT cells, such that the educated NKT cells have the capability to modulate the Th1/Th2 balance toward anti-inflammatory or pro-inflammatory cytokine producing cells.

A specifically preferred embodiment relates to a method for the treatment of immune-related or immune-mediated disorders in a mammalian subject comprising the steps of:
a. obtaining NKT cells from said subject or another subject;
b. *ex vivo* educating the NKT cells obtained in step (a) such that the resulting educated NKT cells have the capability of modulating the Th1/Th2 cell balance toward anti-inflammatory or pro-inflammatory cytokine producing cells; and
c. re-introducing to the subject the educated NKT cells that were obtained in step (b). Modulation of the Th1/Th2 cell balance toward anti-inflammatory cytokine producing cells results in an increase of the quantitative ratio between any one of IL4 and IL10 to IFNγ. Modulation of the Th1/Th2 cell balance toward pro-inflammatory cytokine producing cells results in a decrease of the quantitative ratio between any one of IL4 and IL10 to IFNγ.

More specifically, *ex vivo* education of the NKT cells may be performed by culturing these cells in the presence of any one of:
a. antigens associated with the immune-related or immune-mediated disorder to be treated or any combination thereof;
b. at least one liver-associated cell of tolerized or non-tolerized patients suffering from the same immune-related or immune-mediated disorder or from said subject;
c. at least one cytokine or adhesion molecule; and
d. a combination of any of (a), (b) and (c) above.

The *ex vivo* educated NKT cells according to the method of the invention are re-introduced by adoptive transfer to the treated subject.

Another preferred embodiment relates to the method of the invention wherein the immune-related or immune-mediated disorder is an inflammatory bowel disease (IBD). More particularly, said disease may be Crohn's disease.

In another specifically preferred embodiment, the method of the invention is intended for the treatment of a malignancy selected from the group consisting of melanomas, carcinomas, lymphomas and sarcomas. For this purpose, NKT cells may be manipulated in the direction of enhancing the immune response in a pro-inflammatory direction, in order to augment the favorable anti-tumor immunity.

A preferred embodiment relates to the method of the invention wherein the immune-related or immune-mediated disorder is Non-Alcoholic Steatohepatitis.

In another preferred embodiment, the method of the invention is intended for the treatment of obesity.

Another preferred embodiment of the invention is a method for the treatment of diabetes mellitus or glucose intolerance.

In yet another preferred embodiment, the method of the invention is for the treatment of Graft Versus Host Disease.

Another preferred embodiment relates to the method of the invention for the treatment of an immune-related or immune-mediated disorder or disease comprising Osteoporosis, Multiple Sclerosis, SLE, Rheumatoid Arthritis, JRA, Eye Disease, Skin Disease, Renal Disease, Hematologic Disease, ITP, PA, Autoimmune Liver Disease, Other Rheumatologic Disease, Endocrine Disease (not including Diabetes), Vasculitis, Scleroderma, CREST, Neurologic Disease, Lung Disease, Myositis, Ear Disease, or Myasthenia Gravis.

A preferred embodiment relates to the method of immune modulation through oral tolerance induction or oral immune regulation for the treatment of an immune-related or immune-mediated disorder or disease.

A preferred embodiment relates to the method of immune modulation through oral tolerance induction or oral immune regulation for the treatment of Non-Alcoholic Steatohepatitis.

Another preferred embodiment relates to the method of immune modulation through oral tolerance induction or oral immune regulation for the treatment of diabetes mellitus or glucose intolerance.

In yet another preferred embodiment, the invention relates to the method of immune modulation through oral tolerance induction or oral immune regulation for the treatment of obesity.

The method of the invention may optionally further comprise the step of eliciting in the subject up or down regulation of the immune response to the immune-related or immune-mediated disorder, preferably by oral tolerization.

Yet another preferred embodiment relates to the method of immune modulation through oral tolerance induction or oral immune regulation for the treatment of an immune-related or immune-mediated disorder or disease comprising Osteoporosis, GVHD, Multiple Sclerosis, SLE, Rheumatoid Arthritis, JRA, Eye Disease, Skin Disease, Renal Disease, Hematologic Disease, ITP, PA, Autoimmune Liver Disease, Other Rheumatologic Disease, Endocrine Disease (not including Diabetes), Vasculitis, Scleroderma, CREST, Neurologic Disease, Lung Disease, Myositis, Ear Disease, or Myasthenia Gravis.

In yet another specifically preferred embodiment, the method of the invention is intended for the treatment of human patients.

A second aspect of the present invention relates to a therapeutic composition for the treatment of immune-related or immune-mediated disorder in a mammalian subject. The composition of the invention comprises as an effective ingredient *ex vivo* educated autologous or non-autologous NKT cells capable of modulating the Th1/Th2 cell balance toward anti-inflammatory or pro-inflammatory cytokine producing cells. These educated NKT cells mediate an increase or a decrease in the quantitative ratio between any one of IL4 and IL10 to IFNγ. The composition of the invention may optionally further comprise pharmaceutically acceptable carrier, dilluent, excipient and/or additive.

In a preferred embodiment, the educated NKT cells comprised in the therapeutic composition of the invention are cultured *ex vivo,* prior to its use in the composition of the invention, in the presence of any one of:
a. antigens associated with said immune-related or immune-mediated disorder or any combination thereof;
b. at least one liver-associated cell of tolerized or non-tolerized patients suffering from said immune-related or immune-mediated disorder or from the subject to be treated;
c. at least one cytokine, or adhesion molecule; and
d. a combination of any of (a), (b) and (c) above;.

In one preferred embodiment the therapeutic composition of the invention is intended for the treatment of intestinal inflammatory disease in a mammalian subject, particularly humans, and more specifically for the treatment of Crohn's disease.

In another preferred embodiment the therapeutic composition of the invention is intended for the treatment of a malignancy selected from the group consisting of melanomas, carcinomas, lymphomas and sarcomas.

In a preferred embodiment, the therapeutic composition of the invention is intended for the treatment of Non-Alcoholic Steatohepatitis.

In another preferred embodiment, the invention relates to a therapeutic composition for the treatment of obesity.

Another preferred embodiment of the invention is a therapeutic composition for the treatment of diabetes mellitus or glucose intolerance.

In yet another preferred embodiment, the therapeutic composition of the invention may be used for the treatment of Graft Versus Host Disease.

In yet another preferred embodiment the invention relates to a therapeutic composition for the treatment of an immune-related or immune-mediated disorder. This composition comprises as an effective ingredient, an antibody that specifically recognizes the NKT cells.

In one embodiment, the therapeutic composition of the invention may be used for the treatment of an intestinal inflammatory disease such as Crohn's disease.

In another embodiment the therapeutic composition of the invention may be used for the treatment of a malignancy selected from the group consisting of melanomas, carcinomas, lymphomas and sarcomas. For this purpose, NKT cells may be manipulated in the direction of enhancing the immune response in a pro-inflammatory direction, in order to augment the favorable anti-tumor immunity.

Another preferred embodiment relates to the method of the invention for the treatment of an immune-related or immune-mediated disorder or disease comprising Osteoporosis, Multiple Sclerosis, SLE, Rheumatoid Arthritis, JRA, Eye Disease, Skin Disease, Renal Disease, Hematologic Disease, ITP, PA, Autoimmune Liver Disease, Other Rheumatologic Disease, Endocrine Disease (not including Diabetes), Vasculitis, Scleroderma, CREST, Neurologic Disease, Lung Disease, Myositis, Ear Disease, or Myasthenia Gravis.

A preferred emobidment relates to the use of oral antigens in the manufacture of a therapeutic composition for the treatment of an immune-related or immune-mediated disorder or disease.

A preferred embodiment relates to the use of oral antigens in the manufacture of a therapeutic composition for the treatment of Non-Alcoholic Steatohepatitis.

Another preferred embodiment relates to the use of oral antigens in the manufacture of a therapeutic composition for the treatment of diabetes mellitus or glucose intolerance.

An additional preferred embodiment relates to the use of oral antigens in the manufacture of a therapeutic composition for the treatment of obesity.

In another preferred emobidiment the oral antigens are used in the manufacute of a therapeutic composition for the treatment of an immune-related or immune-mediated or immune-mediate disorder or disease comprising Osteoporosis, Multiple Sclerosis, SLE, Rheumatoid Arthritis, JRA, Eye Disease, Skin Disease, Renal Disease, Hematologic Disease, ITP, PA, Autoimmune Liver Disease, Other Rheumatologic Disease, Endocrine Disease (not including Diabetes), Vasculitis, Scleroderma, CREST, Neurologic Disease, Lung Disease, Myositis, Ear Disease, or Myasthenia Gravis.

As a third aspect, the present invention relates to the use of educated autologous or non-autoloaous NKT cells in the manufacture of a therapeutic composition for modulating the Th1/Th2 balance toward anti-inflammatory cytokine producing cells, in a mammalian subject suffering of an immune-related or immune-mediated disorder.

In a specifically preferred embodiment, the invention relates to the use of *ex vivo* educated autologous or non-autologous NKT cells in the manufacture of a therapeutic composition for the treatment of an immune-related or immune-mediated disorder in a mammalian subject. The educated NKT cells are capable of modulating the Th1/Th2 cell balance toward anti-inflammatory cytokine producing cells, and thus mediate an increase in the quantitative ratio between any one of IL4 and IL10 to IFNγ.

In one specifically preferred embodiment the invention relates to the use of *ex vivo* educated autologous or non-autologous NKT cells in the manufacture of a therapeutic composition for the treatment of intestinal inflammatory disease in a mammalian subject, particularly human patients and especially for the treatment of Crohn's disease

In another specifically preferred embodiment the invention relates to use of *ex vivo* educated autologous or non-autologous NKT cells in the manufacture of a therapeutic composition for the treatment of a malignancy, more specifically, for the treatment of melanomas, carcinomas, lymphomas and sarcomas.

In a preferred embodiment, the invention relates to the use of ex vivo educated autologous or non-autologous NKT cells in the manufacture of a therapeutic composition for the treatment of Non-Alcoholic Steatohepatitis.

In antoher preferred embodiment, the invention relates to the use of ex vivo educated autologous or non-autologous NKT cells in the manufacture of a therapeutic composition for the treatment of obesity.

Another preferred embodiment of the invention relates to the use of ex vivo educated autologous or non-autologous NKT cells in the manufacture of a therapeutic composition for the treatment of diabetes mellitus or glucose intolerance.

In yet another preferred embodiment, the invention relates to the use of ex vivo educated autologous or non-autologous NKT cells in the manufacture of a therapeutic composition for the treatment of Graft Versus Host Disease.

Another preferred embodiment relates to the method of the invention for the treatment of an immune-related or immune-mediated disorder or disease comprising Osteoporosis, Multiple Sclerosis, SLE, Rheumatoid Arthritis, JRA, Eye Disease, Skin Disease, Renal Disease, Hematologic Disease, ITP, PA, Autoimmune Liver Disease, Other Rheumatologic Disease, Endocrine Disease (not including Diabetes), Vasculitis, Scleroderma, CREST, Neurologic Disease, Lung Disease, Myositis, Ear Disease, or Myasthenia Gravis.

The present invention further provides for *ex vivo* educated autologous NKT cells for use in the treatment of immune-related or immune-mediated disorders in a mammalian subject in need of such treatment. The educated NKT cell has been *ex vivo* cultured in the presence of any one of:
a. antigens associated with said immune-related or immune-mediated disorder or any combination therof;
b. at least one liver-associated cell of tolerized or non-tolerized patients suffering from said immune-related or immune-mediated disorder or of said subject;
c. at least one cytokine or adhesion molecule; and
d. a combination of any of (a), (b) and (c) above.

In another embodiment of the present aspect, the invention relates to the use of *ex vivo* educated autologous or non-autologous NKT cells in the treatment of immune-related or immune-mediated disorders in a mammalian subject in need of such treatment.

In yet another preferred embodiment the present invention relates to the use of an antibody that specifically recognizes NKT cells, in the manufacture of a therapeutic composition for the manipulation of the NKT cell population in a mammalian subject suffering of a immune-related or immune-mediated disorder. Specifically, the depletion of said NKT cell population.

The depletion of the NKT cells population results in modulating the Th1/Th2 cell balance toward anti-inflammatory cytokine producing cells.

In a specifically preferred embodiment, the invention relates to the use of an antibody that specifically recognizes the NKT cells, in the manufacture of a therapeutic composition for the treatment of an immune-related or immune-mediated disorder in a mammalian subject.

In one specific embodiment the immune related disorder may be an intestinal inflammatory disease, such as Crohn's disease. In another specific embodiment, the immune-related or immune-mediated disorder may be a malignancy, such as melanoma, carcinoma, lymphoma and sarcoma.

### BRIEF DESCRIPTION OF THE FIGURES

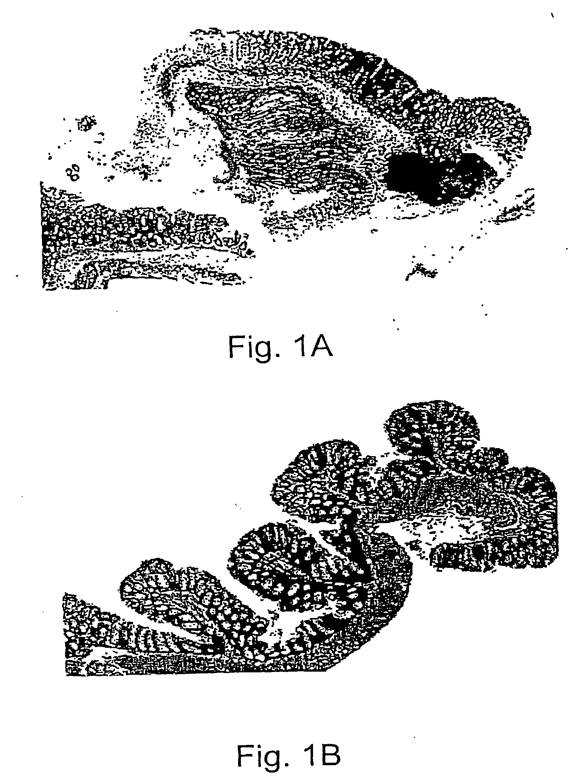

**Figure 1A-1B****:** *Effect of tolerization on histologic evaluation of bowel mucosa in experimental colitis.*
   **Fig. 1A****:** shows paraffin sections from distal colonic tissue (last 10 cm) of non-tolerized mice.
   **Fig. 1B****:** shows paraffin sections from distal colonic tissue (last 10 cm) of tolerized mice.
      Sections were stained with hematoxylin-eosin. Feeding of mouse-derived CEP led to marked alleviation of experimental colitis, manifested by marked reduction in _ inflammatory response and mucosal damage (group B, Fig. 1B). In contrast, severe colitis was observed in non-tolerized mice fed with BSA (group A, Fig. 1 A).

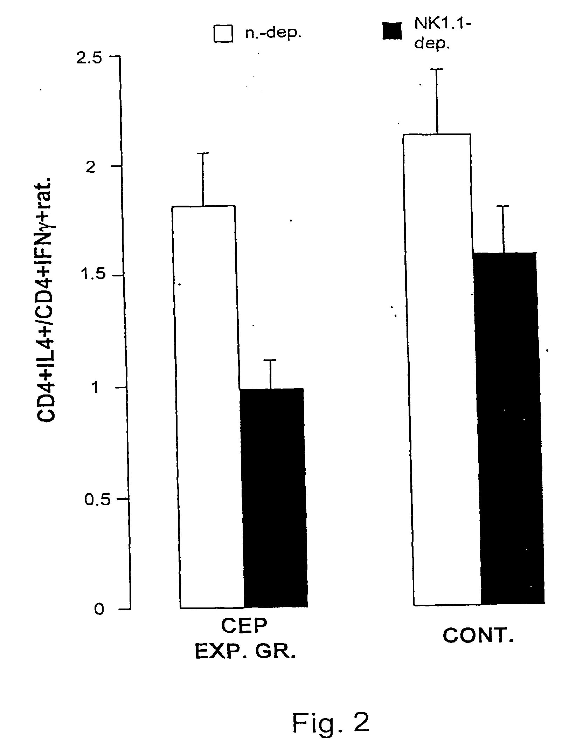

**Figure 2****:** *NK1.1*+ *lymphocytes increase the CD4+IL4+*/*CD4+IFNγ+ ratio in tolerized mice.*
   Splenocytes and liver-associated-lymphocytes (LAL) (2.5 x 10⁶ splenocytes and 0.5 x10⁶ LAL) were harvested from mice in all groups and cultured for 72 hours in the presence of CEP and APC. Flow cytometry analysis summarized in the following histogram has shown that NK1.1-LAL depletion following oral tolerance induction decreased the CD4+IL4+/CD4+IFNγ+ ratio (group B, black bar) in comparison with the non-NK1.1 depleted tolerized mice (group E, white bar). Control NK1.1-depleted group (group F, black bar) had a decrease in CD4+IL4+/CD4+IFNγ+ ratio compared with non-NK1.1-depleted group (group C, white bar). Abbreviations: EXP. GR. = Experimental groups, rat. = ratio, CEP = colitis extracted protein, n-dep. = none-depleted, NK1.1-dep. (NK1.1-depleted), cont. = control.

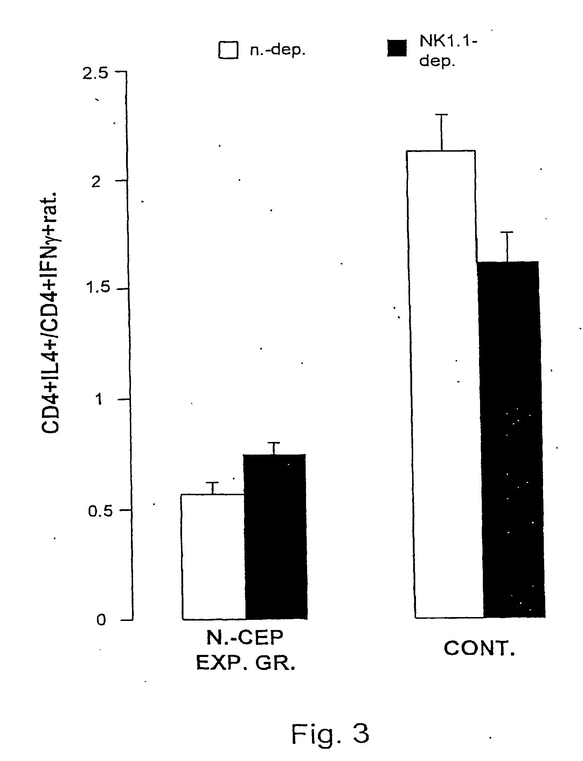

**Figure 3****:** *NK1.1+ lymphocytes decreased the CD4 + IL4 +* /*CD4 + IFNγ+ ratio in non-tolerized mice with experimental colitis.*
   In contrast to tolerized groups, NK1.1-depletion had an opposite effect in non-tolerized mice with experimental colitis (N-CEP). The CD4+IL4+/CD4+IFNγ+ ratio increased in NK1.1-depleted non toterized group (group D, black bar), in comparison with the non-NK1.1 depleted non-tolerized group (groups A, white bar). Abbreviations: EXP. GR. = Experimental groups, rat. = ratio, CEP = colitis extracted protein, n-dep. = none-depleted, NK1.1-dep. (NK1.1-depleted), cont.=control.

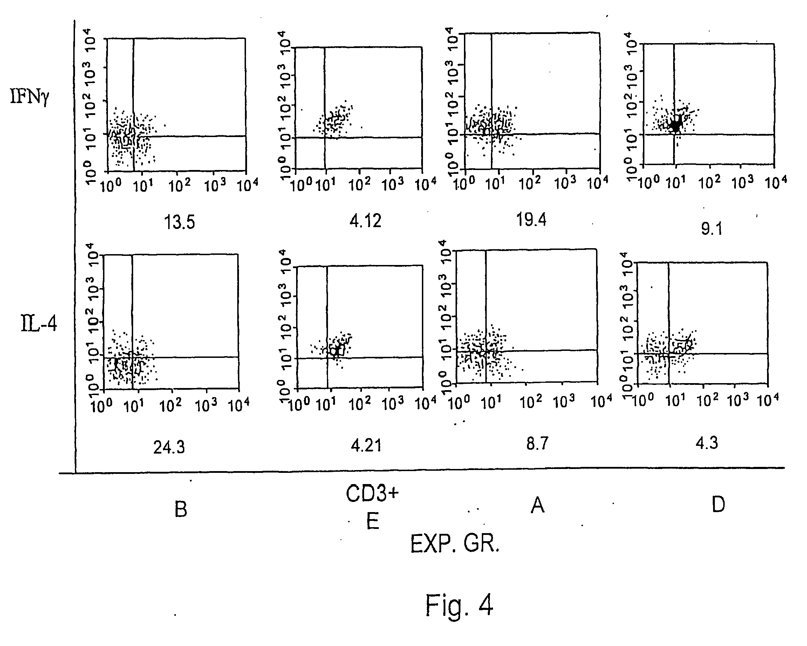

**Figure 4****:** *Expression of IL4 and IFNγ on isolated lymphocytes from different experimental groups.*
   The figure shows representative results of flow cytometry analysis for determination of IL4 and IFNγ expression. Expression of IL4 and IFNγ in isolated lymphocytes from tolerized NK1.1 non-depleted and depleted mice from groups B and E, and non-tolerized NK1.1 non-depleted and depleted mice from groups A and D, respectively. Data are displayed as dot plots after gating of 5x10⁴ small lymphocytes. Numbers below dot plots represent the percentages of stained cells.
   The different experimental groups are indicated by B, E, A and D. Abbreviations: EXP. GR. = Experimental groups.

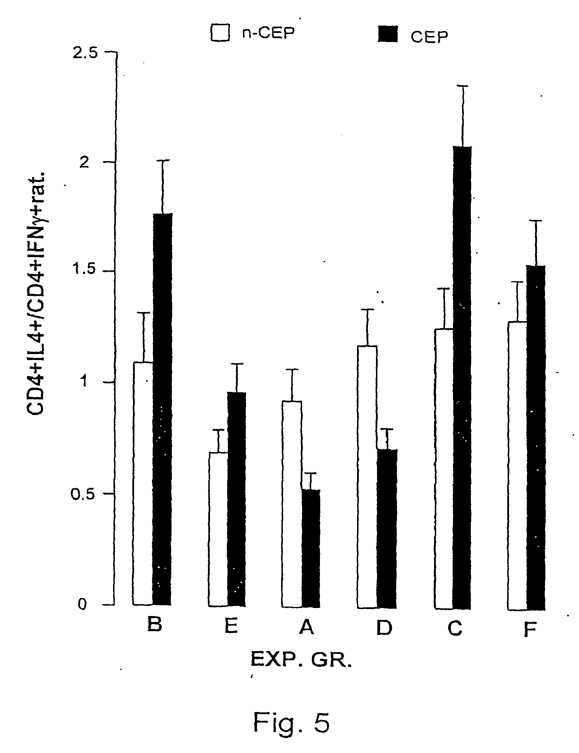

**Figure 5****:** *The effect of in-vitro antigen exposure on CD4+IL4+*/*CD4+IFNγ+ ratio in tolerized and non-tolerized mice with experimental colitis.*
   For evaluation of the effect of disease-target antigen on the CD4+IL4+/ CD4+IFNγ+ ratio, splenocytes and liver-associated-lymphocytes (2.5x10⁶ splenocytes and 0.5 x10⁶ LAL) were harvested from mice of all groups (B, E, A, D, C, F) and cultured for 1 2 hours in the presence of Con A (concavaline-A) and in the absence of CEP and APC (white bars). Flow cytometry analysis have shown that the CD4+IL4+/CD4+IFNγ+ ratio decreased significantly in tolerized mice in groups B and E and in the control groups C and F, and increased significantly in non-tolerized (n-CEP) mice in groups A and D.
   Evaluation of the effect of NK1.1 depletion in the absence of the antigen showed a similar effect to the one described in the presence of antigen (black bars). Lymphocytes harvested from tolerized mice in group B revealed significantly higher CD4+IL4+/CD4+IFNγ+ ratio compared with NK1.1-depleted mice in tolerized group E. In contrast, NK1.1 depletion induced an increase in the CD4+IL4+/CD4+IFNγ+ ratio in non-tolerized mice from groups A and D in the absence of the disease target antigen. Abbreviations: EXP. GR. = Experimental groups, rat. = ratio, CEP=colitis extracted protein, n-CEP. = non-tolerized.

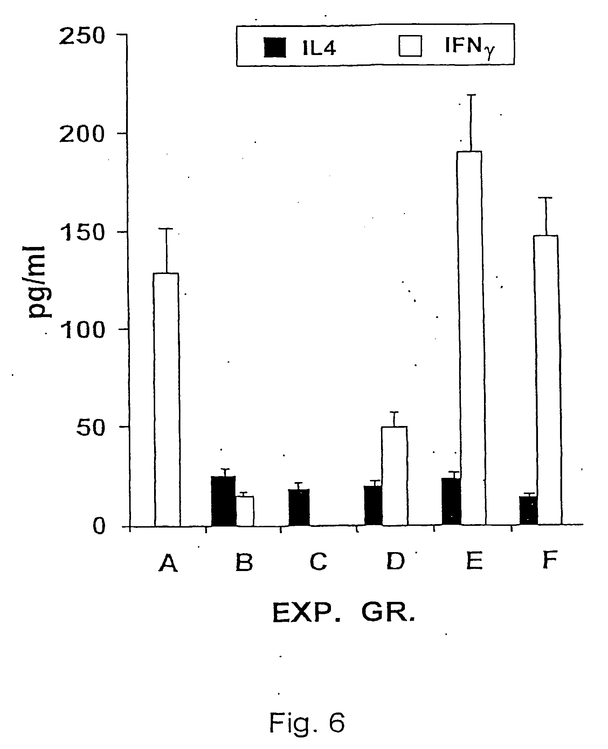

**Figure 6****:** *IL4 and IFNγ levels in the different experimental groups.*
   Supernatant fluids were collected from both sets of triplicates and cytokine levels were measured for all mice from all tolerized and non-tolerized groups (different groups are indicated by A, B, C, D, E, F). IL4 and IFNγ levels were measured by a "sandwich" ELISA. Tolerized mice manifested a shift from Th1 to Th2 immune response cytokine secretion. These mice (group B) manifested an increase in IL4 levels and a decrease in IFNγ levels. In contrast, mice from non-tolerized groups (groups A, E and F) exhibited high IFNγ and low IL4 levels. Abbreviations: EXP. GR. = Experimental groups.

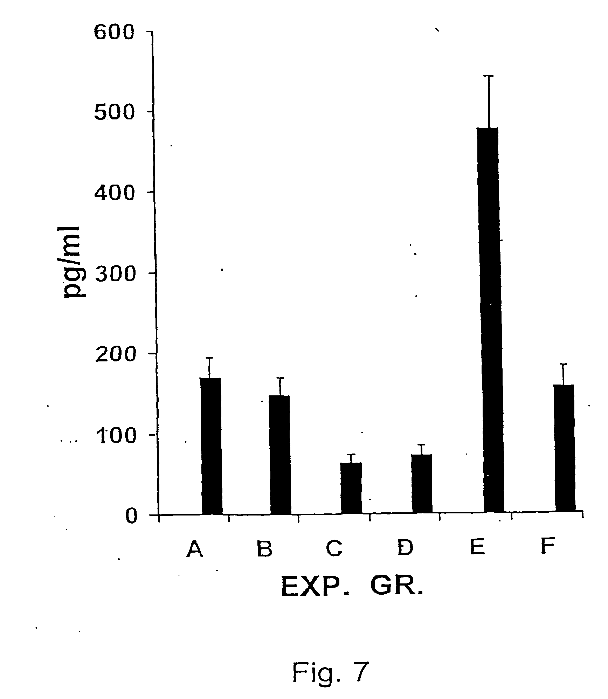

**Figure 7****:** *Effect of NK1. 1- depletion on IL12 levels.*
   Supernatant fluids were collected from both sets of triplicates and cytokine levels were measured for all mice from all tolerized and non-tolerized groups (different groups are indicated by A, B, C, D, E, F). NK1.1 depletion led to an increase in IL12 levels in the CEP-fed groups (groups E and B, respectively) but had an opposite effect in the non-CEP fed groups (groups A and D). Abbreviations: EXP. GR. = Experimental groups.

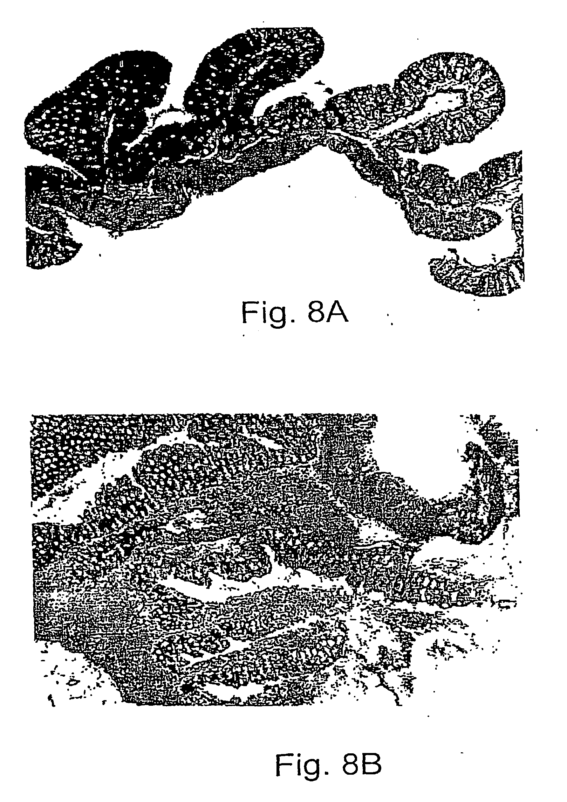

**Figure 8A-8B****:** *Effect of tolerization on histologic evaluation of bowel mucosa in*
   *experimental colitis.*
   **Fig. 8A****:** shows paraffin sections from distal colonic tissue (last 10 cm) of non-tolerized mice.
   **Fig. 8B****:** shows paraffin sections from distal colonic tissue (last 10 cm) of tolerized mice.
   Sections were stained with hematoxylin-eosin. Feeding of mouse-derived CEP led to marked alleviation of experimental colitis, manifested by marked reduction in inflammatory response and mucosal damage (group H, Fig. 8B). In contrast, severe colitis was observed in non-tolerized mice fed with BSA (group G, Fig. 8A).

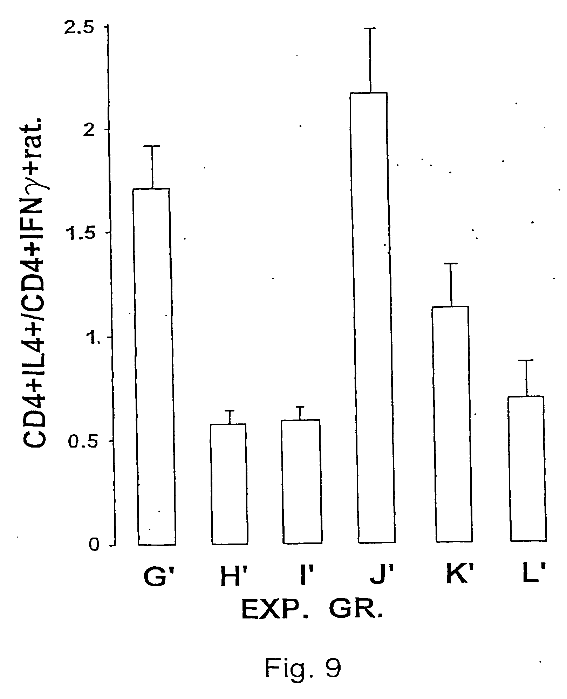

**Figure 9****:** *NK1.1+ lymphocytes increase the CD4+IL4+*/*CD4+IFNγ+ ratio in tolerized mice.*
   Splenocytes and liver-associated-lymphocytes (2.5x10⁶ splenocytes and 0.5x10⁶ LAL) were harvested from mice in all groups and cultured for 72 hours in the presence of CEP and APC. The different experimental groups are indicated by G', H', I', J', K' and L'. Flow cytometry analysis have shown that NK1.1-LAL depletion following oral tolerance induction decreased the CD4+IL4+/CD4+IFNγ+ ratio (group H') in comparison with the non-NK1.1 depleted tolerized mice (group K'). Control NK1.1-depleted group (group L') had a decrease in CD4 + IL4 + /CD4 + IFNγ + ratio compared with non-NK 1 .1-depleted group (group I'). NK1.1+ lymphocytes decreased the CD4 + IL4 + /CD4 + IFNγ + ratio in non-tolerized mice with experimental colitis. In contrast to tolerized groups, NK1.1-depletion had an opposite effect in non-tolerized mice with experimental colitis The CD4+IL4+/CD4+IFNγ+ ratio increased in NK1.1-depleted non tolerized group (group J'), in comparison with the non-NK1.1 depleted non-tolerized group (groups G'). Abbreviations: EXP. GR. = Experimental groups, rat. = ratio.

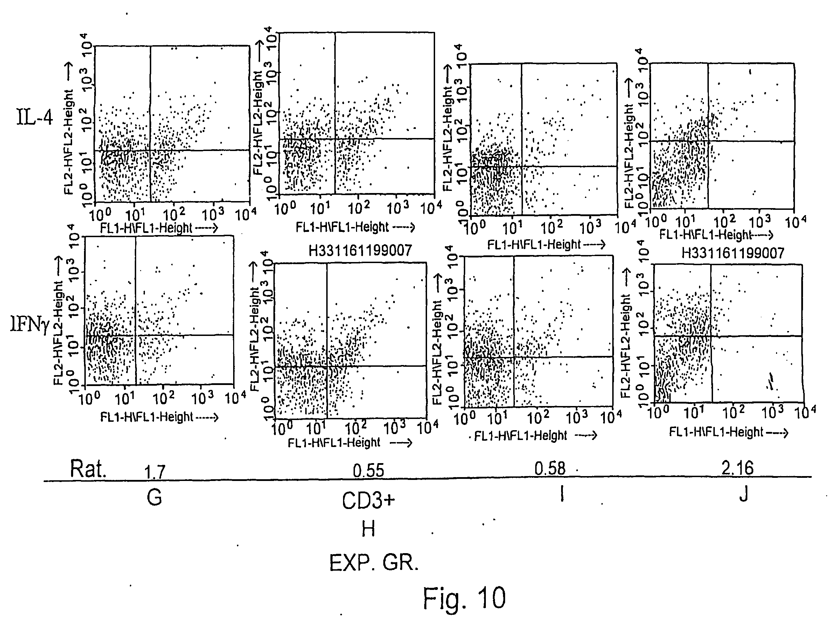

**Figure 10****:** *Expression of IL4 and IFNγ on isolated lymphocytes from different experimental groups.*
   The figure shows representative results of flow cytometry analysis for determination of IL4 and IFNγ expression. Expression of IL4 and IFNγ on isolated lymphocytes from tolerized NK1.1 non-depleted and depleted mice, and non-tolerized NK1.1 non-depleted and depleted mice. Data are displayed as dot plots after gating of 5x10⁴ small lymphocytes. Numbers below dot plots represent the percentages of stained cells. Representative results are shown. Experimental groups (EXP GR).
   The different experimental groups are indicated by G, H, I and J.Abbreviations: EXP. GR. =Experimental groups, rat. = ratio.

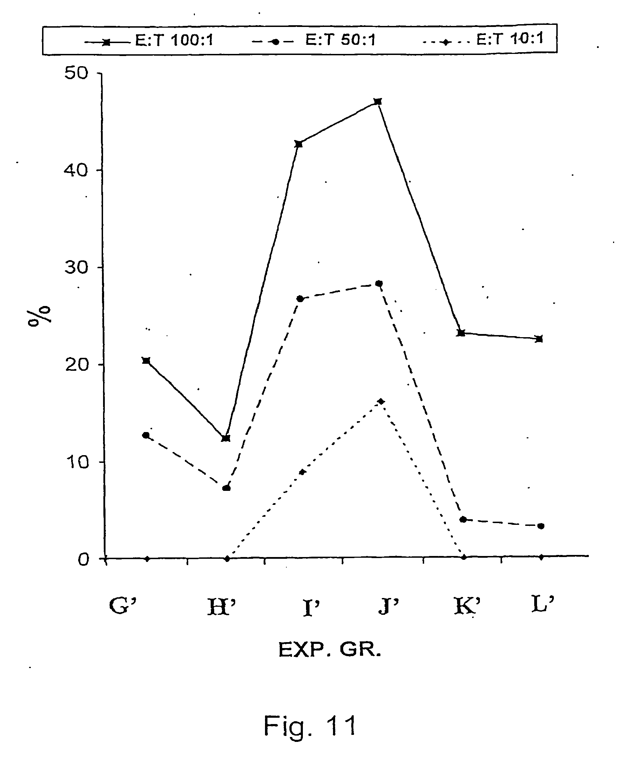

**Figure 11****:** *Liver lymphocyte cytotoxicity by NK1.1.*
   YAC-1 cells were used as target cells in these studies at an E:T ratio of from 100:1 to 10:1. Recipients from non-tolerized non-NK1.1 depleted mice (group H') showed almost no lysis compared to the other groups. Recipients from non-tolerized NK1.1-depleted mice in group G' showed higher lysis then group H', respectively. Recipients from NK1.1- depleted CEP fed mice from group I' showed lower lysis then non NK1.1 depleted mice in group J'. Recipients from control groups had 23% vs. 22.47% cytotoxicity, for mice in group K' compared with group L' respectively. The different experimental groups are indicated by G', H', I', J', K' and L'. Abbreviations: EXP. GR. = Experimental groups.

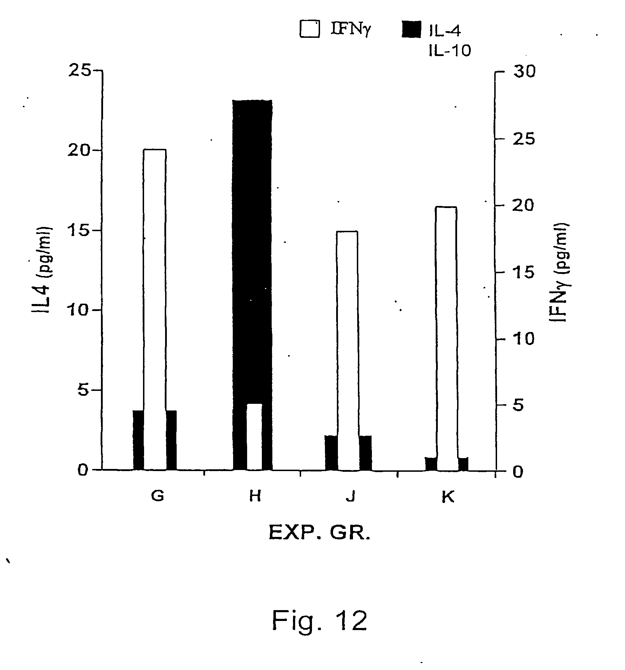

**Figure 12****:** *Cytokine levels in different experimental groups.*
   Supernatant fluids were collected from both sets of triplicates and cytokine levels were measured for all mice from all tolerized and non-tolerized groups. IL4, IL10, and IFNγ levels were measured by a "sandwich" ELISA. Tolerized mice manifested a shift from Th1 to Th2 immune response cytokine secretion. These mice (group H) manifested an increase in IL4, IL10 levels and a decrease in IFNγ levels. In contrast, mice from non-tolerized groups (groups G, J, K) and control group I, exhibited high IFNγ and low IL10 levels. Lymphocytes harvested from tolerized mice in group H revealed significantly higher IL4, IL10, and lower IFNγ levels compared with NK1.1-depleted mice in tolerized group K. In contrast, NK1.1 depletion induced an increase in IFNγ and a decrease in IL4, IL10 levels in non-tolerized mice from groups G and J in the absence of antigen. The different experimental groups are indicated by G, H, J and K. Abbreviations: EXP. GR. = Experimental groups. IL4 and IL10 are indicated by black bars and IFNγ by white bars.

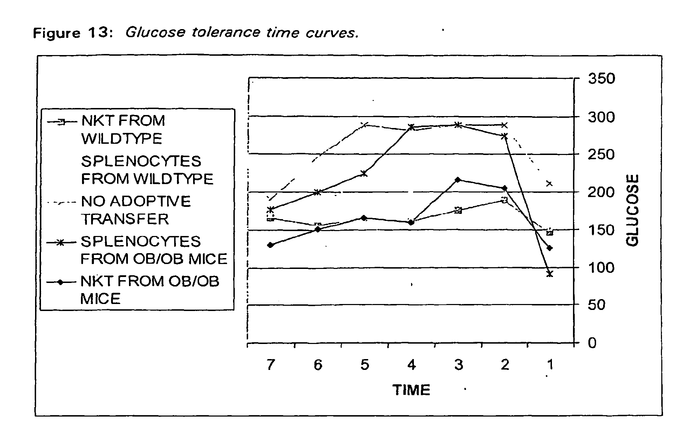

**Figure 13****:** *Glucose tolerance time curves.*

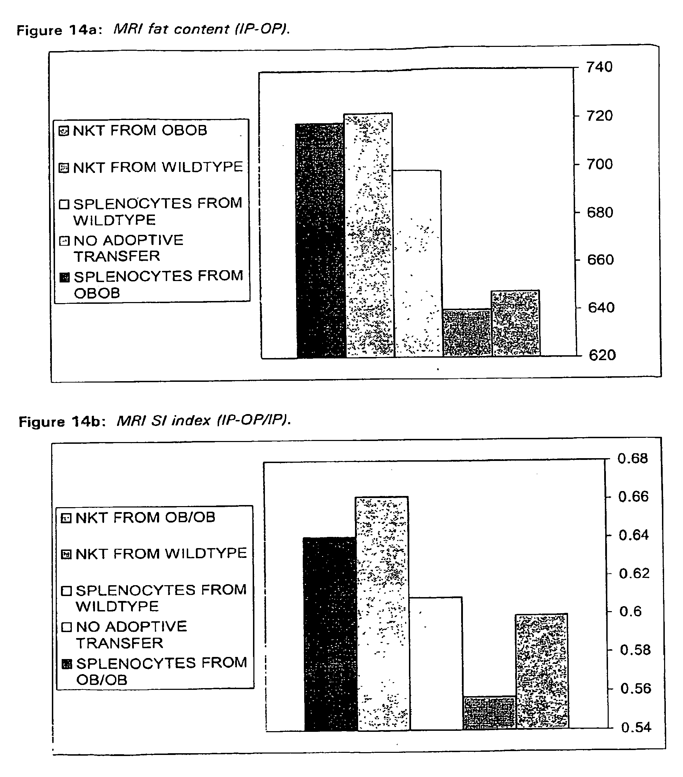

**Figure 14a****:** *MRI fat content (IP-OP).*
**Figure 14b****:** *MRI SI index (IP-OP*/*IP).*

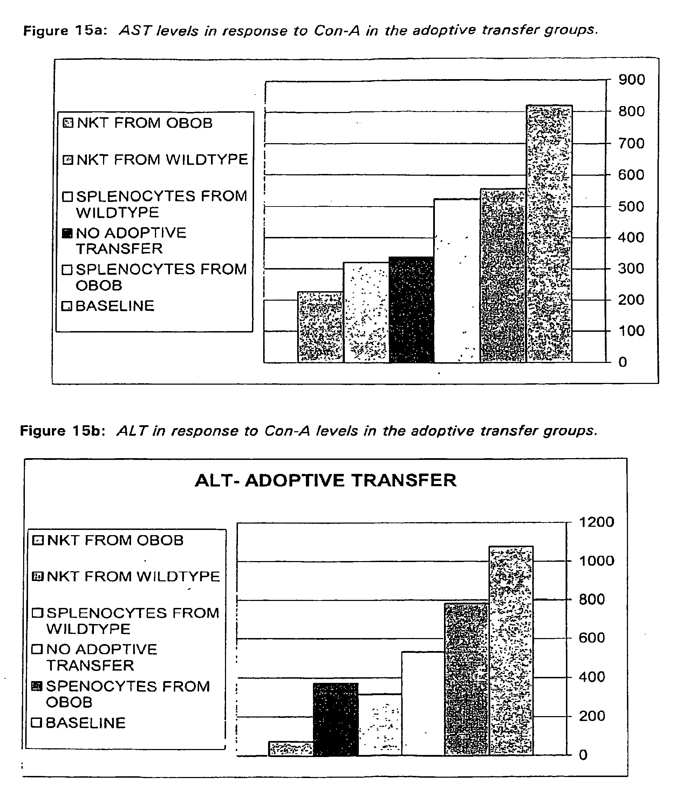

**Figure 15a****:** *AST levels in response to Con-A in the adoptive transfer groups.*
**Figure 15b****:** *ALT in response to Con-A levels in the adoptive transfer groups.*

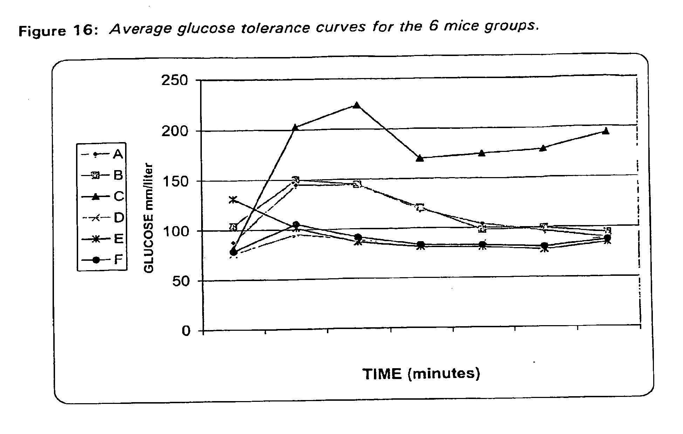

**Figure 16****:** *Average glucose tolerance curves for the 6 mice groups.*

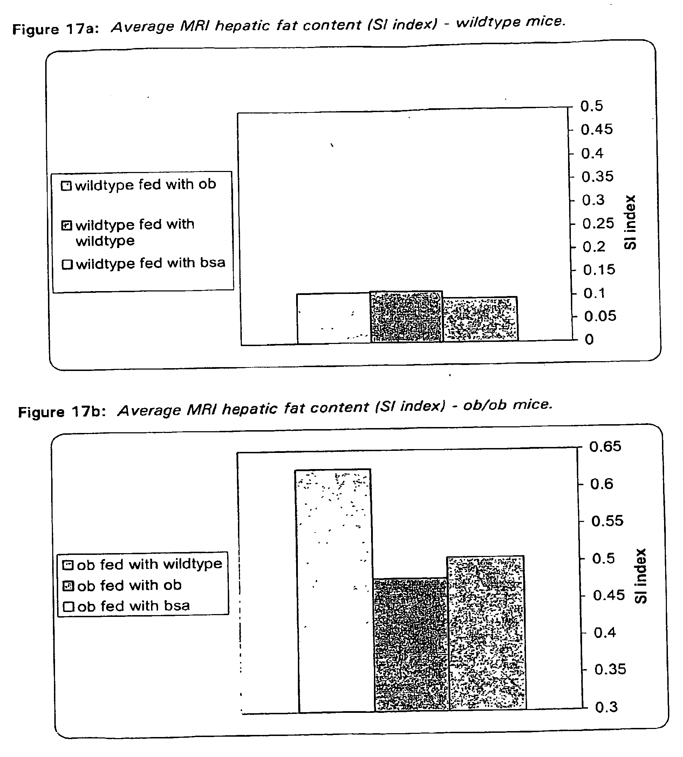

**Figure 17a****:** *Average MRI hepatic fat content (SI index) - wildtype mice.*
**Figure 17b****:** *Average MRI hepatic fat content (SI index) - ob*/*ob mice.*

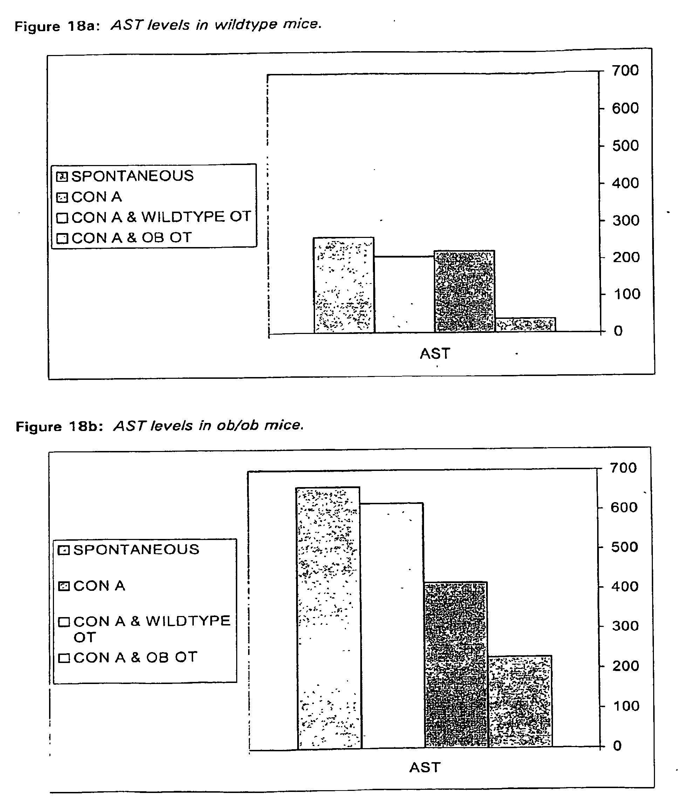

**Figure 18a****:** *AST levels in wildtype mice.*
**Figure 18b****:** *AST levels in ob*/*ob mice.*

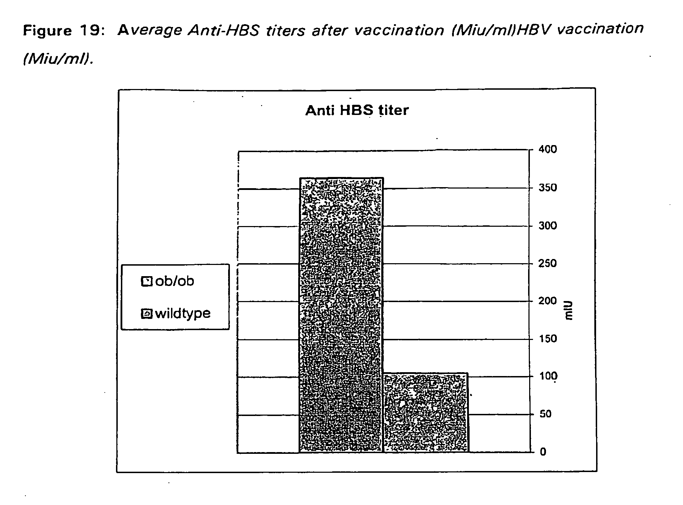

**Figure 19****:** *Average Anti-HBS titers after vaccination (Miu*/*ml)HBV vaccination (Miu*/*ml).*

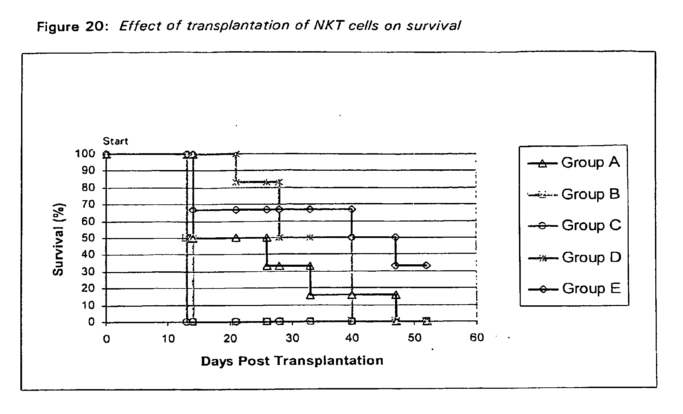

**Figure 20****:** *Effect of transplantation of NKT cells on survival*

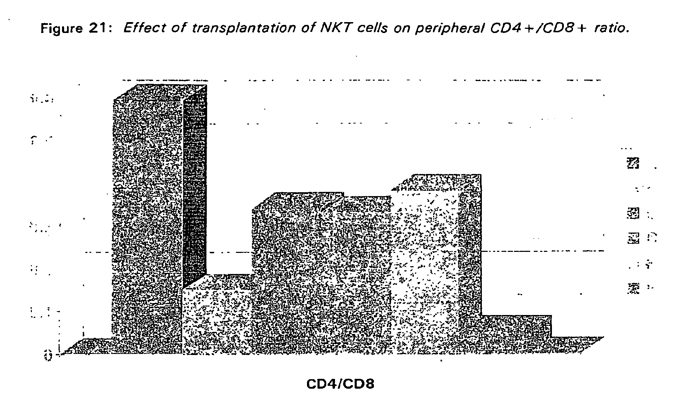

**Figure 21****:** *Effect of transplantation of NKT cells on peripheral CD4*+/*CD8*+ *ratio.*

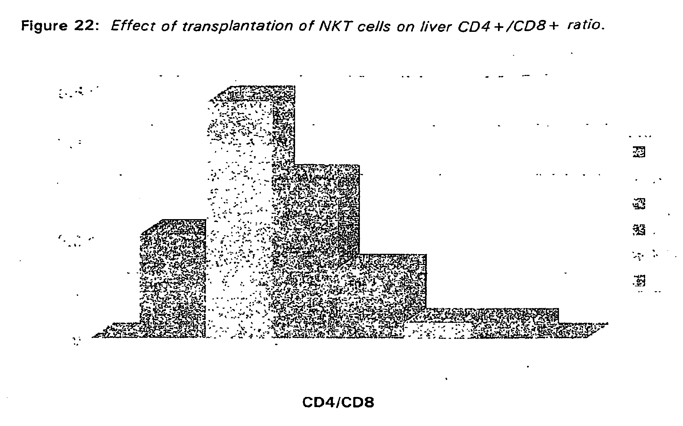

**Figure 22****:** *Effect of transplantation of NKT cells on liver CD4*+/*CD8*+ *ratio.*

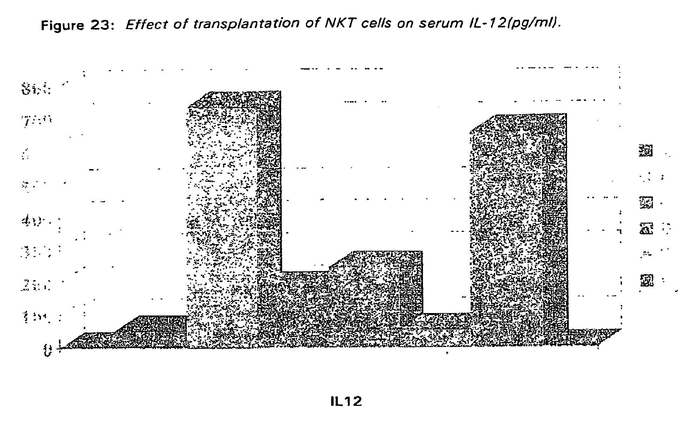

**Figure 23****:** *Effect of transplantation of NKT cells on serum IL-12(pg*/*ml).*

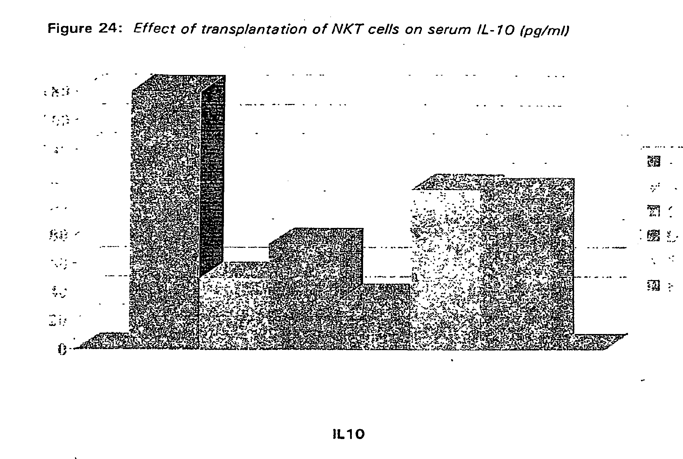

**Figure 24****:** *Effect of transplantation of NKT cells on serum IL-10 (pg*/*ml).*

### DETAILED DESCRIPTION OF THE INVENTION

NK1.1 T cells may be involved in keeping a balance between anti-inflammatory and pro-inflammatory lymphocytes via cytokine secretion, and/or killing, and may be involved in the determination of T helper cell differentiation [Arase, H., et al., Eur. J. Immunol. 23: 307-310 (1993); Yoshimoto, T., et al., J. Exp. Med. 179:1285-1295 (1994), MacDonald, H.R., et al., J. Exp. Med. 182:633-638 (1995), Seder, R.A. et al., Annu. Rev. Immuno. 12:635-673 (1994), Yoshimoto, T., et al., Science 270:1845-1847 (1995)]. Multiple signaling pathways were identified for NK1.1 T cell activation. It is assumed that NK1.1⁺ T cells are not stably polarized, and upon different triggers, TCR engagement triggers both Th1 and Th2 cytokine secretion from these cells [Bendelac, A., et al., Annu. Rev. Immunol. 15:535-562 (1997); Arase, H., et al., J. Immunol. 151 :546 (1993); Kawamura, T., et al., J. Immunol. 160:16-19 (1998), Chen, H., et al., J. Immonol., 159:2240-2249 (1997); Arase, H., et al., Eur. J. Immunol. 23: 307-310 (1998);Yoshimoto, T., J. Exp. Med. 179: 1285-1295 (1994); MacDonald, H.R., J. ibid., (1995)]. NK1.1R or IL12R engagement may selectively promote the Th1 secretion paradigm [Bendelac, *et al.* (1997) *ibid.;* Arase, H., et al., J. Exp. Med. 183:2391-2396 (1996); Hayakawa, T., et al., J. Exp. Med. 176:269-274 (1992)].

As described above, NK1.1⁺ T lymphocytes play a complicated role in immunoregulation. The results described in the present invention show that NK1.1 T lymphocytes have a dual role in regulating immune-mediated experimental colitis. The depletion of NK1.1 T lymphocytes following oral tolerance induction prevented the adoptive transfer of tolerance while significantly decreasing the quantitative ratio between IL4 to IFNγ secreted by CD4⁺ cells. In contrast, the depletion of NK1.1 T lymphocyte in non-tolerized mice alleviated colitis and significantly increased the quantitative ratio between IL4 secreted by CD4⁺ to IFNγ secreted by CD4⁻.

In a first aspect, the invention thus relates to a method for the treatment of immune-related or immune-mediated disorders in a mammalian subject in need of such treatment. The method of the invention comprises the step of manipulating the NKT cell population in a subject by suitable means. The manipulation of the NKT cell population results in modulation of the Th1/Th2 cell balance and shifts it toward the production of anti-inflammatory or pro-inflammatory cytokine producing cells. It should be emphasized that any immune-modulation can down or up regulate the immune response. This modulation is further mediated by different components of the subject's or another subject's immune system. Such components are, for example, cellular immune reaction elements, humoral immune reaction elements and cytokines.

In a preferred embodiment, manipulating the NKT cell population is by depletion of this cell population. Depletion of the NKT cell population may be performed, for example, by administering to the subject a therapeutically effective amount of a composition comprising as the effective ingredient an antibody that specifically recognizes the NKT cells. This specific method encompasses the use of polyclonal as well as, preferably monoclonal antibodies.

The generation of polyclonal antibodies against proteins is described in Chapter 2 of Current Protocols in Immunology, Wiley and Sons Inc. Monoclonal antibodies may be prepared from B cells taken from the spleen or lymph nodes of immunized animals, particularly rats or mice, by fusion with immortalized B cells under conditions which favor the growth of hybrid cells. For fusion of murine B cells, the cell line Ag-8 is preferred. The technique of generating monoclonal antibodies is described in many articles and textbooks, such as the above-noted Chapter 2 of Current Protocols in Immunology. Spleen or lymph node cells of these animals may be used in the same way as spleen or lymph node cells of protein-immunized animals, for the generation of monoclonal antibodies as described in Chapter 2 therein. The techniques used in generating monoclonal antibodies are further described by Kohler and Milstein, Nature 256:495-497, (1975), and in United States Patent No. 4,376,110.

The term "antibody" is meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less non- specific tissue binding than an intact antibody [WahI et al., J. Nucl. Med. 24: 316-325, (1983)]. It will be appreciated that Fab and F(ab')₂ and other fragments of the antibodies useful in the present invention may be used for the selective depletion of the NKT cells, according to the methods disclosed herein for intact antibody molecules. Such fragments are typically produced by proteolytic cleavage, using enzymes such as papain (to produce Fab fragments) or pepsin (to produce F(ab')₂ fragments).

An antibody is said to be "capable of specifically recognizing" a certain cell if it is capable of specifically reacting with an antigen which is in this particular example an extracellular marker molecule expressed by said cell, to thereby bind the molecule to the antibody.

An "antigen" is a molecule or a portion of a molecule capable of being bound by an antibody, which is additionally capable of inducing an animal to produce antibody capable of binding to an epitope of that antigen. An antigen may have one or more than one epitope. The term "epitope" is meant to refer to that portion of any molecule capable of being bound by an antibody that can also be recognized by that antibody. Epitopes or "antigenic determinants" usually consist of chemically active surface groupings of molecules such as amino acids or sugar side chains, and have specific three-dimensional structural characteristics as well as specific charge characteristics.

As an alternative, depletion of the NKT cell population may be performed by an *ex vivo* pheresis, using beads coated with an antibody that specifically recognizes the NKT cells. In a pheresis procedure the whole blood is drawn from the treated subject, and is immediately separated into plasma, red cells and white cells. The NKT cells are depleted from the white cell population by using a specific antibody to the NKT cell markers while other blood components are being simultaneously transferred back to the treated subject.

NK1.1 molecules on NK1.1⁺ T cells serve as receptors leading to IFNγ and not to IL4 production [Arase, H., et al., J. Exp. Med. 183:2391-2396 (1996); Seder, R.A., et al., Proc. Natl. Acad. Sci. USA 90:10188-92 (1993)]. Upon stimulation with glycosylposphatidylinositol-anchored protein or LPS ligand, NK1.1 T cells become IFNγ producing cells, inhibit Th2 cell differentiation and suppress IgE response [Cui, J., et al., J. Exp. Med. 190(N-6): 783-792 (1999)]. Exogenous IL2 increases IFNγ production upon NK1.1R-P1 cross-linking [Arase, H., *et al.,* (1996) *ibid.].* NK1.1⁺ T cells are involved in CD4⁺ T cell differentiation via the secretion of large amount of IL4 promptly upon *in vivo* stimulation with anti-CD3 [Yoshimoto, T., et al., Science 270:1845-7 (1995)]. A CD1-restricted NK1.1 T cell population is essential for anti-CD3-induced early IL4 burst [Seder, R.A., Ann. Rev. Imm. 12:635-673 (1994)]. Bacterial LPS has been shown to activate NK1.1⁺ cells via IL-12 production from Kupffer cells and subsequently induces IFNγ production [Ma, X., et al., J. Exp. Med. 183:147-157(1996)]. Cell to cell contact between dendritic cells and NK and/or T lymphocytes resulted in a substantial increase in both cell cytolytic activity and IFNγ production [De-Moraes, L., et al., Eur. J. Immunol. 28:1507-1515 (1998)]. IL18 and leukocyte function-associated antigen-1 may play a role in the accumulation of NK1.1⁺ T cells in the liver and in their cytotoxic activity [Sakamoto, Y. et al., J. Immunol., 103(5 pt 2):445-51 (1999)]. NK1.1+ T cells have been suggested as playing a role in antigen presentation, which may be another pathway by which they influence T cell response [Seki. S., et al., J. Immunol. V 147:1214-1221 (1991)]. This subtype of cells was previously shown to have a high level of autologous killing [Crispe, N., et al., Immun. Today 11:236-245 (1996); Kawamura, T., et al., J. Immunol. 160:16-19 (1998); Dohert, D.G., et al., J. Hepatology 28:59A. (1998)]. Fas expression by LAL resulted in death of activated Fas expressing T cells [Dohert, D.G., *et al.,* (1998) *ibid.;* Jonsson, J.R., et al., Hepatology 26:269A(1997); Doherty, D.G., et al., Hepatology 26: 445A (1997)]. Thus it is possible that in a tolerized environment NK1.1 T cells may be involved in killing sensitized pro-inflammatory cells in addition to their IL4-mediated anti-inflammatory cytokines secretion, whereas in a non-tolerized environment they may be involved in killing anti-inflammatory cells in addition to their IFNγ secretion. Both IL4 and IL12 increase the cytotoxic potential of NK1.1 T cells [Hashimoto, W., et al., J. Immonol. 154: 4333-4340 (1995); Ballas, Z.K., et al., J. Immonol. 150:17-30 (1993)]. During inflammation there is an IL12/IFNγ loop which plays a role in balancing the immune response [Ma, *et al.,* (1996) *ibid.*]. IL12 augments IFNγ secretion, as well as the cytolytic activity and proliferation of NK1.1 + T cells [Cui, *et al.,* (1999) *ibid.;* Bendelac *et al.,* (1997) *ibid.;* Arase, *et al.,* (1996) *ibid.,* De-Moraes, *et al.,* (1998) *ibid.;* Neurath, M.F., et al., J. Exp. Med., 182:1281-1290 (1995)].

Therefore, as an alternative preferred embodiment, the invention relates to a method for treatment of immune-related or immune-mediated disorders in a mammalian subject. This method involves manipulation of NKT cell population by the *ex vivo* education of said NKT cells, such that the educated NKT cells have the ability of modulating the Th1/Th2 balance and shifting it toward the production of anti-inflammatory cytokine producing cells and administration of the educated cells into said subject. This modulation results in an increase in the quantitative ratio between any one of IL4 and IL10 to IFNγ (may also be referred to throughout the entire specification as CD4⁺IL4, IL10 /CD4⁺IFNγ ratio). In immune-mediated disorders the ratio decreases according to severity of the disease and it may increase during recovery. Therefore, it is to be appreciated that the change in the quantitative ratio between any one of IL4 and IL10 to IFNγ should be related to the pre-treatment level.

In yet another preferred embodiment, the invention relates to a method for treatment of immune-related or immune-mediated disorders in a mammalian subject. This method involves manipulation of NKT cell population by the *ex vivo* education of said NKT cells, such that the educated NKT cells have the ability of modulating the Th1/Th2 balance and shifting it toward the production of pro-inflammatory cytokine producing cells and administration of the educated cells into said subject. This modulation results in a decrease in the quantitative ratio between any one of IL4 and IL10 to IFNγ (may also be referred to throughout the specification as CD4⁺ IL4, and IL10/CD4⁺ IFNγ ratio).

The term "CD4⁺IL4" means the IL4 produced by CD4⁺ cells, "CD4⁺IL10" means the IL10 produced by CD4⁺ cells and "CD4⁺IFNγ" means the IFNγ produced by CD4⁺ cells. The term "CD4⁺IL4 IL10 /CD4⁺IFNγ ratio" used in the present invention, means the quantitative ratio between any one of IL4 and IL10 preferably produced by CD4⁺ cells, and between the IFNγ preferably produced by CD4⁺ cells. Quantitative measurements for defining the quantity of each of these cytokines were performed as described in the examples (experimental procedures) described herein.

A specifically preferred embodiment relates to a method for the treatment of immune-related or immune-mediated disorders in a mammalian subject. This method of treatment comprises the steps of:
a. obtaining NKT cells from said subject or another subject;
b. *ex-vivo* educating the NKT cells obtained in step (a) such that the resulting educated NKT cells have the capability of modulating the Th1/Th2 cell balance toward anti-inflammatory or pro-inflammatory cytokine producing cells; and
c. re-introducing to said subject the educated NKT cells that were obtained in step (b). Modulation of the Th1/Th2 balance toward anti-inflammatory cytokine producing cells results in an increase in the quantitative ratio between any one of IL4 and IL10 to IFNγ. Modulation of the Th1/Th2 balance toward pro-inflammatory cytokine producing cells results in a decrease in the quantitative ratio between any one of IL4 and IL10 to IFNγ.

NKT cells can be obtained from bone marrow, liver, spleen, or uterus, but can also be obtained from the peripheral blood, by cytopheresis methods described above.

More specifically, *ex-vivo* education of the NKT cells may be performed by culturing these cells in the presence of any one of:
a. at least one antigen associated with bystander epitopes to the immune-related or immune-mediated disorder to be treated, or any combination thereof;
b. at least one liver-associated cell of tolerized or non-tolerized patients suffering from the same immune disorder or from the subject to be treated, or any combination thereof;
c. at least one cytokine or adhesion molecule or any combination thereof; and
d. a combination of any of (a), (b) and (c) above.

It is to be appreciated that the NKT cells may be educated *in vivo* as well via any of the methods described above. They can be modulated prior to, or at any point in time following exposure to the allogeneic epitopes or antigens.

In one particular embodiment, the *ex vivo* education of the NKT cells may be performed by culturing these cells in the presence of antigens associated with the immune-related or immune-mediated disorder to be treated. These antigens may be allogeneic antigens taken from donor patients suffering from said immune-related or immune-mediated disorder, xenogeneic antigens, autologous antigens, recombinantly prepared antigens, or any combination thereof.

These antigens may be native or non-native with regards to the subject. They can be natural or synthetic, modified or unmodified, whole or fragments thereof. Fragments can be derived from synthesis as fragments or by digestion or other means of modification to create fragments from larger entities. Such antigen or antigens comprise but are not limited to proteins, glycoproteins, enzymes, antibodies, histocompatibility determinants, ligands, receptors, hormones, cytokines, cell membranes, cell components, viruses, viral components, viral vectors, non-viral vectors, whole cells, tissues or organs. The antigen can consist of single molecules or mixtures of diverse individual molecules. The antigen can present itself within the context of viral surface, cellular surface, membrane, matrix, or complex or conjugated with a receptor, ligand, antibody or any other binding partner. Such antigen may be introduced to the subject alone or with an agent or agents that could further contribute to uptake, stability, reactivity or targeting.

Polymerization and degradation, fractionation and chemical modification are all capable of altering the properties of a particular antigen in terms of potential immune responses. These small segments, fragments or epitopes can either be isolated or synthesized. As a non-limiting example, such antigen may be a combination of different antigens derived from body extracts, such as the CEP used for *ex vivo* education in Example 7.

The method of the present invention further encompasses recombinantly prepared antigens. Preparation of recombinant antigens involves the use of general molecular biology techniques that are well known in the art. Such techniques include for example, cloning of a desired antigen to a suitable expression vector.

"Vectors", as used herein, encompass plasmids, viruses, bacteriophage, integratable DNA fragments, and other vehicles, which enable the integration of DNA fragments into the genome of the host. Expression vectors are typically self-replicating DNA or RNA constructs containing the desired gene or its fragments, and operably linked genetic control elements that are recognized in a suitable host cell and effect expression of the desired genes. These control elements are capable of effecting expression within a suitable host. Generally, the genetic control elements can include a prokaryotic promoter system or an eukaryotic promoter expression control system. This typically includes a transcriptional promoter, an optional operator to control the onset of transcription, transcription enhancers to elevate the level of RNA expression, a sequence that encodes a suitable ribosome binding site, RNA splice junctions, sequences that terminate transcription and translation and so forth. Expression vectors usually contain an origin of replication that allows the vector to replicate independently of the host cell.

A vector may additionally include appropriate restriction sites, antibiotic resistance or other markers for selection of vector-containing cells. Plasmids are the most commonly used form of vector but other forms of vectors which serve an equivalent function and are, or become known in the art are suitable for use herein. See, e.g., Pouwels et al., Cloning Vectors: a Laboratory Manual (1985 and supplements), Elsevier, N.Y.; and Rodriquez, et al. (eds.) Vectors: a Survey of Molecular Cloning Vectors and their Uses, Buttersworth, Boston, Mass (1988), which are incorporated herein by reference.

It has been recently proposed that the liver is a major site of T cell destruction and that in the liver of autoimmune mice Ipr/Ipr, there is a failure of this process with leakage of T cells from the liver to peripheral lymphoid tissues [Crispe, N., et al., Immunol. Review, 174:47-62 (2000)]. The liver was shown to play a role in T cell differentiation. CD3⁻CD4⁺/CD8⁺TCRβ cells and CD3-4-TCRβ⁺ cells can be generated from CD4⁻8⁻TCRβ athymic nude bone marrow cells by culture with liver parenchymal cells [Mabuchi, A., et al., J. Leukocyte Biology, 63:575-583 (1998)]. Therefore, in another particular embodiment, the *ex vivo* education of the NKT cells may be performed by culturing these cells in the presence of liver-associated cells. These cells may be for example Kupffer cells, Stellate cells, liver endothelial cells, liver associated stem cells, or any other liver-related lymphocytes.

Co-culturing of the NKT cells in the presence of peripheral lymphocytes from tolerized or non-tolerized patients suffering from the same immune-related or immune-mediated disorder or from the treated subject is also contemplated in the present invention. In order to obtain lymphocytes from a subject, particularly a human subject, blood is drawn from the patient by cytopheresis, a procedure by which a large number of white cells are obtained, while other blood components are simultaneously transferred back to the subject.

As described in Example 7, *ex vivo* education of NKT cells may be preformed by the co-culturing of NKT cells with CD4 or CD8 cells. These cells are preferably obtained from a tolerized subject (mice received CEP as oral tolarization).

In another particular embodiment, the *ex-vivo* education of the NKT cells may be performed by culturing the cells in the presence of cytokines such as IL4, IL10, TGFβ, IFNγ, IL12 and IL15, or in the presence of adhesion molecules such as Integrins, Selectin and ICAM.

While IL12 exerts an effect of IFNγ induction by NK1.1 T cells, IFNγ may in turn contribute to its regulation [Ma *et al.,* (1996) *ibid*.]. Cytolytic activity of thymocytes from mice undergoing acute GVHD decreased significantly following NK1.1⁺ cell depletion [Neurath et al., (1995) *ibid.].* The increase in NK1.1⁺ T cells in the thymus of mice suffering from acute GVHD, was preceded by a transient increase of IL12 production in the thymus [Neurath *et al.,* (1995) *ibid.].* IL12 was reported to induce an increase of NK1.1 + T cells in the thymus of mice suffering from acute GVHD [Onoe, Y., et al., Immunology 95:248-256. (1998)]. It was recently shown that anti-IL12 antibodies enhance oral tolerance in transgenic animals and was associated with increased TGFβ secretion [Marth, T., et al., J. lmmunol. 157:2348-2357 (1996)]. Both IL12 and TNFα were shown to have an important role in the immunepathogenesis of experimental colitis [Bragger, M.S.H., et al., Gut 34:1705 (1998); Parronchi, P., et al., Am. J. Pathol. 150:823 (1997)]. IL12 production by monocytes/macrophages was essential in maintaining TNBS induced colitis and was required for the Th1-mediated inflammatory response [Kuhn, R., et al., Cell 75:263-274, (1993); Sellon, R.K., et al., Immun. 66:5224-5231 (1998); Neurath *et al* (1995) *ibid.;* Marth, T., et al., J. Immunol. 157:2348-2357 (1996)].

Antibodies to IL12 abrogated chronic TNBS induced colitis [Neurath *et al.,* (1995) *ibid.].* Therefore, IL12 may have a dominant role in disease pathogenesis via NK1.1⁺ T cell activation. It is possible that activation of this subset of lymphocytes induces IFNγ secretion, followed by a Th1 immune shift in non-tolerized mice [Arase, *et al.,* (1996) *ibid.;* Bleicher, P.A., et al., Science 250:679-682 (1990); Kitamura, H., et al., J. Exp. Med. 189:1121-1127 (1999)].

NK1.1⁺ T cells may be potent IFNγ producers in the presence of IL12 in the experimental colitis [Cui *et al.,* (1999) *ibid.;* Bendelac *et al.,* (1997) *ibid.;* Arase et a/., (1996) *ibid*.; De-Moraes *et al.,* (1998) *ibid.].* The results of the present invention suggest that IFNγ was secreted by NK1.1 T cells in the inflammatory state via NK1.1R, independent of the IL12 pathway. This may have been followed by IFNγ triggered-IL12 production, with IL12 induced-IFNγ secretion via the IL12R. In contrast, in the anti-inflammatory tolerized state, NK1.1 T cells are activated with increased IL4 secretion. Indeed, adoptive transfer of lymphocytes from non-tolerized NK1.1-depleted mice upregulated the anti-inflammatory Th2 cytokines. It is possible that different stimuli determine the type of cytokine response.

Thus, chemokines or other mediators may determine NK1.1 + T cell function and the way in which they influence the Th1/Th2 paradigm in different immunological environments.

In a specifically preferred embodiment, the NKT cell that has been *ex vivo* educated as described above may be re-introduced to the treated subject. This can be carried out by a process that has been termed adoptive transfer. The particular educated NKT cells used for the transfer may preferably originate from the subject (autologous transfer). A syngeneic or non-syngeneic donor (non-autologous transfer) is not excluded. The storage, growth or expansion of the transferred cells may have taken place *in vivo, ex vivo* or *in vitro.*

Methods for *in vitro* storage, growth or expansion of cells prior to transfer are well known to practitioners of the art. When the educated NKT cells intended for use in a transfer are derived from a donor, these cells may also undergo storage, growth or expansion *in vivo* or *in vitro* as described above.

Cell therapy may be by injection, e.g., intravenously, or by any of the means described herein above. Neither the time nor the mode of administration is a limitation on the present invention. Cell therapy regimens may be readily adjusted taking into account such factors as the possible cytotoxicity of the educated cells, the stage of the disease and the condition of the patient, among other considerations known to those of skill in the art.

The method of the invention may optionally further comprise the step of eliciting in the treated subject up or down regulation of the immune response to the immune-related or immune-mediated disorder. A down regulation response may be achieved by administering to said subject components, cells, tissues or organs derived from any allogeneic donor suffering from said immune-related or immune-mediated disorder, xenogenic sources, autologous sources, immunological equivalents, or any combination thereof.

The present invention provides for the administration of non-native active compounds without the risk of an immune response that could diminish the effectiveness of such treatment, whether such treatment is transient or whether such treatment is made repeatedly over a prolonged period. The present invention thus provides for the effective biological function of these non-native active compounds without interference by the body's immune response. This can be achieved by the use of the immune modulation as provided in this invention wherein it can be used as general immune suppression for transient or short term treatment and/or by tolerization provided by modulation of the immune response for prolonged treatment. In some cases, a combination of two or more such immune-modulating regimens may be advantageous. Such treatments may be applied prior to and/or during the course of administration of non-native active compounds.

In a specifically preferred embodiment, said components, cells, tissues or organs may by administered in a single dose or in multiple doses. These components, cells, tissues or organs may be administered by a single route of administration or by at least two different routes of administration.

The components may be administered directly to the subject to be treated or, depending on the size of the compound, it may be desirable to conjugate them to a carrier prior to their administration. Therapeutic formulations may be administered in any conventional dosage formulation. Formulations typically comprise at least one active ingredient, as defined above, together with one or more acceptable carriers thereof.

Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. The nature, availability and sources, and the administration of all such compounds including the effective amounts necessary to produce desirable effects in a subject are well known in the art and need not be further described herein.

Specifically, said components, cells, tissues or organs may be administered by a route selected from oral, intravenous, parenteral, transdermal, subcutaneous, intravaginal, intranasal, mucosal, sublingual, topical and rectal administration and any combination thereof. Preferably, these components, cells, tissues or organs are administered orally as an oral tolerization.

Another preferred embodiment of the method of the invention relates to the treatment of an inflammatory bowel disease (IBD), particularly Crohn's disease. The treatment of Crohn's disease in a mammalian, particularly human subject, comprises the steps of:
a. obtaining NKT cells from said subject;
b. *ex vivo* educating the NKT cells obtained in step (a) such that the resulting educated NKT cells have the capability of modulating the Th1/Th2 cell balance toward anti-inflammatory cytokine producing cells; and
c. re-introducing to said subject the educated NKT cells that were obtained in step (b). Modulation of the Th1/Th2 balance toward the production of anti-inflammatory cytokine producing cells results in increase in the quantitative ratio between any one of IL4 and IL10 to IFNγ.

Although the method of the invention is particularly intended for the treatment of immune-related or immune-mediated disorders in humans, other mammals are included. By way of non-limiting examples, mammalian subjects include monkeys, equines, cattle, canines, felines, mice, rats and pigs.

For treating a human patient, the method of the invention for *ex vivo* education may utilize a specific subtype of NKT cells which are the NKT cells that express the CD56 marker. For mice, the method of the present invention for *ex vivo* education may utilize the specific subtype of NK 1.1⁺ T cells. The examples of the present invention disclose experiments using the NK 1.1 ⁺ cells of a mouse model. It is to be appreciated that these results are also applicable to the NKT cells that express the CD56 marker in humans. The *ex vivo* educating of the CD56 marker-expressing NKT cells according to the invention is by culturing these cells in the presence of any one of:
a. at least one antigen associated with Crohn's disease; these antigens include, but are not limited to allogeneic antigens from donors suffering of Crohn's disease, xenogenic antigens, autologous antigens from the patient itself, and recombinantly prepared antigens, or any combination thereof;
b. at least one liver-associated cell from tolerized or non-tolerized patients suffering from Crohn's disease or from the treated patient; these cells include, but are not limited to Kupffer cells, Stellate cells, liver endothelial cells, liver associated stem cells, and any other liver-related lymphocytes, or any combination thereof;
c. at least one cytokine such as IL4, IL10, TGFβ IFNγ IL12 and IL15, or adhesion molecules such as Integrins, Selectin and ICAM, or any combination thereof; and
d. a combination of any of (a), (b) and (c) above.

The educated NKT cell according to the method of the invention is re-introduced by adoptive transfer to the treated subject.

The method of the invention may optionally further comprise the step of eliciting in the subject up or down regulation of the immune response to inflamed intestine. The elicitation of a down regulation response may be induced by administering to the subject components that may be proteins extracted from inflamed intestines of a subject suffering from Crohn's disease, or from the intestines of the treated subject.

The components may be cells, tissues, organs or parts thereof, and they may be administered in a single dose, or in multiple doses. These components may be administered by a single route of administration or by at least two different routes of administration. Specifically, said components may be administered by a route selected from oral, intravenous, parenteral, transdermal, subcutaneous, intravaginal, intranasal, mucosal, sublingual, topical and rectal administration, or any combination thereof. Preferably, the components are administered orally as oral tolarization (oral introduction of CEP) as described in the examples.

In another specifically preferred embodiment, the method of the invention is intended for the treatment of a malignancy. In cancerous situations, modulation of the NKT cells may be in the direction of inducing a pro-inflammatory response or in augmenting the anti-tumor associated antigen immunity. As used herein to describe the present invention, "cancer", "tumor" and "malignancy" all relate equivalently to a hyperplasia of a tissue or organ. If the tissue is a part of the lymphatic or immune systems, malignant cells may include non-solid tumors of circulating cells. Malignancies of other tissues or organs may produce solid tumors. In general, the methods and compositions of the present invention may be used in the treatment of non-solid and solid tumors.

Malignancy, as contemplated in the present invention, may be selected from the group consisting of melanomas, carcinomas, lymphomas and sarcomas. Malignancies that may find utility in the present invention can comprise but are not limited to hematological malignancies (including leukemia, lymphoma and myeloproliferative disorders), hypoplastic and aplastic anemia (both virally induced and idiopathic), myelodysplastic syndromes, all types of paraneoplastic syndromes (both immune mediated and idiopathic) and solid tumors (including lung, liver, breast, colon, prostate Gl tract, pancreas and Karposi).

For treating a mammalian subject suffering of cancer, the educated NKT cell used by the method of the invention can be administered in a variety of ways. By way of a non-limiting example, the educated cells may be delivered intravenously, or into a body cavity adjacent to the location of a solid tumor, such as the intraperitoneal cavity, or injected directly into or adjacent to a solid tumor.

Still further, the present invention provides for a method for the education of NKT cells. This education may be performed by culturing these cells in the presence of any one of:
a. at least one antigen associated with bystander epitopes to the immune-related or immune-mediated disorder to be treated, or any combination thereof;
b. at least one liver-associated cell of tolerized or non-tolerized patients suffering from the same immune disorder or from the subject to be treated, or any combination thereof;
c. at least one cytokine or adhesion molecule or any combination thereof; and
d. a combination of any of (a), (b) and (c) above.

The methods of the invention may be combined with other therapies useful in the treatment of cancer. It is also anticipated that this treatment may be given to a mammalian subject that is already immuno-suppressed due to disease. The evaluation of the immune status of the human or veterinary patient may be readily determined by one of skill in the art.

As a second aspect, the present invention relates to therapeutic composition for the treatment of an immune-related or immune-mediated disorder in a mammalian subject. The composition of the invention comprises as an effective ingredient *ex vivo* educated autologous NKT cells capable of modulating the Th1/Th2 balance toward anti-inflammatory cytokine producing cells. These educated autologous NKT cells mediate increase in the quantitative ratio between any one of IL4 and IL10 to IFNγ.

The compositions of the invention may further contain a pharmaceutically acceptable carrier, additive, diluent or excipient. Suitable carriers include, e.g., saline phosphate buffered saline, and saline with 5% HSA or PPF. Other suitable carriers are well known to those of skill in the art and are not a limitation on the present invention. Similarly, one of skill in the art may readily select other desired components for inclusion in a pharmaceutical composition of the invention, and such components are not a limitation of the present invention.

In a preferred embodiment, the educated autologous NKT cells of the therapeutic composition of the invention are *ex vivo* cultured in the presence of any one of:
a. at least one antigen associated with the immune-related or immune-mediated disorder to be treated; these antigens may be any one of allogeneic antigens from donors suffering from the same immune-related or immune-mediated disorder, xenogenic antigens, autologous antigens from the treated patient and recombinantly prepared antigens, or any combination thereof;
b. at least one liver-associated cell of tolerized or non-tolerized patients suffering from said immune-related or immune-mediated disorder or from the treated subject; these cells include, but are not limited to Kupffer cells, Stellate cells, liver endothelial cells, and any other liver-related lymphocytes, or any combination thereof;
c. at least one cytokine such as IL4, IL10, TGFβ IFNγ IL12 and IL15, or adhesion molecules such as Integrins, Selectin and ICAM; and
d. a combination of any of (a), (b) and (c) above.

In one preferred embodiment, the therapeutic composition of the invention is intended for the treatment of intestinal inflammatory disease in a mammalian subject, and more specifically for the treatment of Crohn's disease. This composition comprises as an effective ingredient educated autologous NKT cells, which have been rendered capable of modulating the Th1/Th2 balance toward the production of anti-inflammatory cytokine producing cells.

The educated autologous NKT cell contained in the therapeutic composition of the invention is capable of modulating the Th1/Th2 balance and shift it towards the production of anti-inflammatory cytokine producing cells. The result of this balance shift is an increase in the CD4+IL4+/CD4+IFNγ ratio (the quantitative ratio between any one of IL4 and IL10 to IFNγ). These modulation processes are further mediated by different components of the subject's immune system, such as cellular immune reaction elements, humoral immune reaction elements and cytokines.

The education of the autologous NKT cells contained in the compositions is preferably performed as described above.

In another preferred embodiment, the therapeutic composition of the invention is intended for the treatment of a malignancy such as melanoma, carcinoma, lymphoma and/or sarcoma. In cancerous situations, modulation of the NKT cells contained in the compositions of the invention may be in the direction of inducing a pro-inflammatory response or in augmentation of the anti-tumor associated antigens immunity.

In yet another preferred embodiment, the invention relates to a therapeutic composition for the treatment of immune-related or immune-mediated disorders. This composition comprises as an effective ingredient an antibody that specifically recognizes the NKT cells. The compositions of the invention may further contain a pharmaceutically acceptable carrier. Suitable carriers include, e.g., saline phosphate buffered saline, and saline with 5% HSA or PPF. Other suitable carriers are well known to those of skill in the art and are not a limitation on the present invention. Similarly, one of skill in the art may readily select other desired components for inclusion in a pharmaceutical composition of the invention, and such components are not a limitation of the present invention.

In one embodiment, this therapeutic composition of the invention may be used for the treatment of an intestinal inflammatory disease, such as Crohn's disease. For the treatment of intestinal inflammatory diseases, and particularly Crohn's disease, oral pharmaceutical compositions may advantageous. Oral administration will permit amelioration of the patient's condition, without the need for systemic immunosuppression or invasive procedures.

In another embodiment, the therapeutic composition of the invention may be used for the treatment of a malignancy selected from the group consisting of melanomas, carcinomas, lymphomas and sarcomas.

Composition dosages may be in any amount sufficient to modulate the Th1/Th2 balance. It is understood by the skilled artisan that the preferred dosage would be individualized to the patient following good laboratory practices and standard medical practices.

As used herein, "an amount sufficient to modulate the Th1/Th2 balance" means an amount necessary to achieve a selected result. For example, an effective amount of the composition of the invention will modulate the Th1/Th2 balance toward anti-inflammatory cytokine producing cells.

The compositions and methods of the present invention may further provide for the treatment of autoimmune diseases such as insulin-dependent diabetes mellitus (IDDM).

The compositions of the invention can be administered in a variety of ways. By way of non-limiting example, the composition may be delivered intravenously.

The pharmaceutical forms suitable for injection use include sterile aqueous solutions or dispersions and sterile powders for the extemporaneous preparation of sterile injectable solutions or dispersions. In all cases the form must be sterile and must be fluid to the extent that easy syringeability exists. It must be stable under the conditions of manufacture and storage and must be preserved against the contaminating action of microorganisms, such as bacteria and fungi.

The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterilized active ingredients into a sterile vehicle which contains the basic dispersion medium and the other required ingredients from those enumerated above.

In the case of sterile powders for the preparation of the sterile injectable solutions, the preferred method of preparation are vacuum and freeze drying techniques which yield a powder of the active ingredient plus any additional desired ingredient from a previously sterile-filtered solution thereof.

The pharmaceutical compositions of the invention generally comprise a buffering agent, an agent which adjusts the osmolarity thereof, and optionally, one or more pharmaceutically acceptable carriers, excipients and/or additives as known in the art. Supplementary active ingredients can also be incorporated into the compositions. The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As used herein "pharmaceutically acceptable carrier" includes any and all solvents, dispersion media, coatings, antibacterial and antifungal agents and the like. The use of such media and agents for pharmaceutically active substances is well known in the art. Except when any conventional media or agent is incompatible with the active ingredient, its use in the therapeutic composition is contemplated.

As a third aspect, the present invention relates to the use of an educated autologous NKT cell, in the manufacture of therapeutic pharmaceutical compositions for modulating the Th1/Th2 cell balance toward the production of anti-inflammatory cytokine producing cells, in a mammalian subject suffering of a immune-related or immune-mediated disorder. Preferred use is the manufacture of compositions for the treatment of intestinal inflammatory disease in a mammalian subject, specifically, Crohn's disease in human subjects. Alternatively, the educated autologous NKT cells may be used in the preparation of therapeutic pharmaceutical compositions for the treatment of a malignancy, such as melanoma, carcinoma, lymphoma and sarcoma. In cancerous situation, modulation of the NKT cells of the invention may be in the direction of inducing a pro-inflammatory response or in augmentation of the anti-tumor associated antigens immunity towards a favorable direction.

The present invention further provides for *ex vivo* educated autologous NKT cells. The educated NKT cell has been *ex vivo* cultured in the presence of any one of:
a. at least one antigen associated with said immune-related or immune-mediated disorder, or any combination thereof;
b. at least one liver-associated cell of tolerized or non-tolerized patients suffering from said immune-related or immune-mediated disorder or of said subject or any combination thereof;
c. at least one cytokine, or adhesion molecule; and
d. a combination of any of (a), (b) and (c) above.

Still further, the invention provides for an *ex vivo* educated autologous NKT cell of the invention for use in the treatment of immune-related or immune-mediated disorders in a mammalian subject in need of such treatment.

In another embodiment of the present aspect, the invention relates to the use of an *ex vivo* educated autologous N KT cell in the treatment of immune-related or immune-mediated disorders in a mammalian subject in need of such treatment.

In yet another preferred embodiment, the present invention relates to the use of an antibody, that specifically recognizes the NKT cells, in the manufacture of a therapeutic pharmaceutical composition for manipulation of the NKT cell population in a mammalian subject suffering of a immune-related or immune-mediated disorder. Specifically, the depletion of said NKT cell population in said subject. It is to be appreciated that the depletion of the NKT cell population results in modulating the Th1/Th2 balance toward the preferred production of anti-inflammatory cytokine producing cells. The antibodies may be particularly used for the preparation of a therapeutic pharmaceutical composition for the treatment of immune-related or immune-mediated disorders in mammalian subjects, specifically intestinal inflammatory disease, such as Crohn's disease in a human subject.

In another specific embodiment, the immune-related or immune-mediated disorder may be a malignancy such as melanomas, carcinomas, lymphomas and sarcomas.

### The Role of the Immune System in the Pathogenesis of Non-Alcoholic Steatohepatitis

Non-alcoholic steatohepatitis (NASH) is a clinico-pathological entity consisting of hepatic fat accumulation, inflammation and fibrosis in patients who have no history of alcohol consumption. It will progress to cirrhosis in 20% of cases and is considered the most common cause of cryptogenic cirrhosis in the western world (Caldwell SH, et al, Hepatology 29:664 (1999); Matteoni CA, et al., Gastroenterology 116:1413 (1999). NASH is common in patients who suffer of other metabolic disturbances, which are suggested to play a contributing role in the pathogenesis of the disorder. These include insulin resistance (Sanyal AJ, et al., Gastroenterology 120:1183 (2001), obesity-related ATP depletion (Cortez-Pinto H et al., Jama 282:1659 (1999), increased free-fatty-acid beta peroxidation (Hruszkewycz AM, Biochem Biophys Res Commun 153:191 (1988), iron accumulation (George DK, et al., gastroenterology 114:311 (1998), antioxidant depletion (Harrison SA et al, gastroenterology 123:M1332 (2002), and leptin deficiency (Cohen B et all., Science 274:1185 (1996). Yet no therapeutic intervention, including weight loss, tight diabetic control, normalization of lipid levels and antioxidant treatment have consistently shown an alteration in the natural progression of the disorder (Angulo P. New England Journal of Medicine 346:1221-1231 (2002).

Most information about NASH has been derived from two mammalian models: leptin-deficient ob/ob mice and leptin-receptor deficient fa/fa Zucker rats. Leptin is a protein that is involved with the regulation of body weight (Zhang Y et al., Nature 372:425-432 (1994). Its deficiency in rodents and humans results in a severe form of 'metabolic syndrome' (formerly termed syndrome X) consisting of morbid obesity, glucose intolerance, hyperlipidemia, and severe hepatic steatosis (Pelleymounter MA et al., Science 269:540-543 (1995). Yet, as mentioned above, no intervention aimed at correcting some of these metabolic disturbances have resulted in an amelioration of the hepatic steatosis, fibrosis, and inflammation.

Recent evidence suggests that the immune system may play a pivotal role in the pathogenesis of NASH in the leptin deficient models. In leptin deficient mice, defective hepatic macrophage (Kupffer cell) response has been observed after liver injury induction by lipopolysaccharide (Diehl AM. J Physiol Gastrointest liver Physiol 282:G1-G5 (2002). In similar models, LPS induction of IL6 was greatly enhanced, while that of IL10 was inhibited (Loffreda S, et al., FASEB J 12:57-65 (1998). Ob/ob mice hepatic macrophages were observed to produce more IL12 and less IL15 than control mice in response to LPS challenge, which may explain the significant reduction in the number and function of NKT lymphocytes observed in these mice (Yang et al., Proc Natl Acad Sci USA 94:2557-2562 (1997). Other observations have shown a reduction in the number of CD4 T lymphocytes in the blood and liver of leptin-deficient ob/ob mice (Howard JK et al, J Clin Invest 104:1051-1059 (1999) and Lord et al., Nature 394:897-901 (1998). This may explain the relative resistance of leptin-deficient mice to Concanavalin A hepatitis, which is mediated by CD4 T lymphocytes (Faggioni R et al., Proc Natl Acad Sci USA 97:2367-2372 (2000).

### The Th1/Th2 Dysbalance in Non-Alcoholic Steatohepatitis

CD4 and CD8 lymphocytes are classified as either Th1 cells that produce IL-2 and IFNγ, or Th2 cells that produce IL-4 and IL-10. The immune system responds to foreign and self-antigens by a shift in balance between the two subtypes of responses [Weiner, H.L., et al., Immunol. Today 18: 335-343 (1997); Adorini, L., et al., Immunol. Today 18:209-211 (1997)]. Usually the Th1 type response causes a pro-inflammatory reaction [Adorini, L., *et al.,* (1997) *ibid.;* Mizoguchi, A., et al., J. Exp. Med. 183:847-856, (1996)], while anti-inflammatory cytokines such as IL10 shift the balance towards an anti-inflammatory Th2 reaction, thereby alleviating immune-mediated disorders [Mizoguchi, A., *et al.,* (1996) *ibid.;* Madsen, K.L., et al., Gastroenterology 113:151-159 (1997); Van Deventer Sander, J., et al., Gastroenterology 113:383-389 (1997)]. NKT cells, in response to different endogenous and exogenous stimuli, are believed to play a major role in the direction of the immune system towards either the Th1 or Th2 pathways.

Leptin has been shown to play a major role in the immune regulation of the balance between Th1 & Th2 response (Lord GM et al., Nature 394:897-901 (1998). In the leptin-deficient ob/ob mouse NASH model an alteration of the number and function of NKT cells has been suggested to tilt the immune system towards the Th1 response. This is suggested to result in an increased sensitivity to LPS induced hepatotoxicity and a unique resistance to the hepatotoxic effects of Concanavalin A. The difference may be in their different pathogenic mechanisms. The former depends upon the action of the innate hepatic immune system, which is hyperactive in the leptin-deficient mice, while the latter is dependent upon the activation of NKT-lymphocytes, which are suppressed and defective in the leptin deficient mice (Faggioni R et al., PNAS 97:2367-2372 (2000), Zhiping LI et al., Gastroenterology 123:1304-1310 (2002).

### The Immune System and Obesity

The immune system and the regulation of adipose tissue metabolism appear to be closely interlinked. Up to fifty percent of cells within adipose tissues are composed of non-adipose cells, including many immunocytes (Montague CT et al., Diabetes 47:1384-91 (1998)). Most research has been focused on the immunological consequences of morbid obesity. Immunological alterations which are known to exist in obese animals and humans include reduced DTH and mitogen-stimulated lymphocyte proliferation responses (Chandra RK et al., Acta paediatr Scand 69:25-30 (1980)), impaired phagocyte number and function (Krishnan EC et al., J Surg Res 33:89-97 (1982)), attenuation of insulin induced lymphocyte cytotoxicity (Koffler M et al., Diabetes 40:364-360 (1991)), and changes in the CD4 / CD8 ratio, especially during weight loss attempts (Field CJ et al., Am J Clin Nutr 54:123-129 (1991)).

Adipose cells are known to secrete pro-inflammatory cytokines including TNF-α (Hotamisligil GS et al., Science 259:87-91 (1993)) and IL6 (Purohit A. et al., Journal of Clinical Endocrinology and Metabolism 80:3052-58 (1995)), which are both related to the level of adiposity (Hotamisligil GS et al., Journal of Internal Medicine 245:621-625 (1999)). Some of these cytokines are considered to have metabolic effects such as insulin resistance mediated by TNF-α (Ogawa H et al., Biochimica et biophysica acta 1003:131-135 (1989)) and lipoprotein lipase inhibition mediated by IL6 (Feingold et al., Diabetes 41:97s-101s (1992)). TNF-α knockout mice have higher insulin sensitivity and improved lipid profile than their normal littermates (Uysal et al., Nature 389:610-614 (1997)). Other components of the immune system, which are produced by adipose cells, include the protein adipsin, which is an integral part of the alternative complement system, and functions identically to human complement factor D (Rosen BS et al., Science 244:1483-7 (1989)).

Little information is known about the role of the immune system as a mediator of obesity, but several recent studies suggest that the immune system may have an important contributory role in the development of obesity. Several cytokines are known to act as adipose tissue regulators. TNF-α suppresses the expression of ß₃ adreno-receptors on adipose cells, which are involved in sympathetically mediated lipolysis, while IL1 stimulates adipose leptin secretion (Sarraf et al., Journal of experimental medicine 185:171-175 (1997)). The metabolic activity rate of adipose cells has been observed to be closely correlated to their distance from the closest lymph node (Pond CM et al., Proceedings of the nutrition society 60:365-374 (2001)), through a mechanism which is partly mediated by IL4, IL6 and TNF-α (Mattacks CA et al., Cytokine 11:334-346 (1 999)).

These observations, which point to the fact that obese animals and humans may also be suffering of various alterations in the different arms of the immune system, suggest that modulation of the immune system may change some of the pathogenic mechanisms responsible for the development of morbid obesity.

### The Role of the Immune System in the Pathogenesis of Graft Versus Host Disease

Graft Versus Host Disease (GVHD) is a major obstacle to successful bone marrow transplantation. GVHD is a multi organ disorder that develops following Stem Cell Transplantation (SCT) [Ferrara JLM, Deeg HJ. Graft versus host disease. New Eng J of Med 1991; 324:667-72]. The pathogenesis involves recognition of alloreactive antigens and activation of T cells and other immunocompetent effector cells, resulting in tissue destruction [Vogelsang GB. Graft versust host disease: Implications from basic immunology for prophylaxis and treatment. Cancer Treat and Res 1997; 77:87-97]. Liver involvement in GVHD is a result of an immune attack by transplated donor lymphocytes on recipient bile ducts.

Several studies have shown the importance of regulatory T cell subsets in GVHD. For example, infusion of CD4+CD25+ regulatory T cells has recently been shown to inhibit GVHD lethality [Taylor PA, Lees CJ, Blazar B. The infusion of ex-vivo activated and expanded CD4 + CD25 + immune regulatory cells inhibits graft-versus-host-disease lethality. Blood 2002; 99: 3493-99]. NKT cells are a unique subset of regulatory T lymphocytes with important immune modulatory effects. These cells have previously been shown to be of critical importance in a variety of immune mediated disorders, including various infectious, inflammatory and neoplastic processes. NKT cells may be involved in both Th1 and Th2 type immunity via the secretion of different cytokines (i.e. IFN gamma or IL-4), or by activation of different immune cell subsets [Godfrey DJ, Hammond KJ, Poulon LD, Smyth MJ, Baxter AG. NKT cells: facts, functions and fallacies. Imunol Today 200, 21:573-83]. Recently, we have shown that NKT cells have a critical role in oral immune regulation-induced anti-inflammatory and anti-neoplastic effects in murine models of colitis [Trop S. Ilan Y. NK 1.1 + T cell: A two-faced lymphocyte in immune modulation of the IL4/IFN paradigm? J of Clinical Immunology, 22:270-80, 2002] and hepatoma [Shibolet O, Alper R, Zlotogarov L, Thalenfeld B, Engelhardt D, Rabanni E, Ilan Y. NKT and CD8 lymphocytes mediate suppression of hepatocellular carcinoma growth via tumor antigen-pulsed dendritic cells. Int J Cancer. 20;106:236-43, 2003], respectively. Previous studies have demonstrated a role for NKT cells in acute and chronic GVHD. For example, NK1.1 positive T cell subsets were shown to suppress GVHD, while NK1.1 negative T lymphocytes aggravated GVHD, an effect that was associated with differential cytokine production [Zeng D, Lewis D, Dejbakhsh-Jones S, Lan F, Garcia-Ojeda M, Sibley R, Strober S. Bone marrow NK1.1- and NK1.1 + T cells reciprocally regulate acute graft versus host disease. J Exp Mes 1999; 189: 1073-81].

Acute GVHD is the major complication of post allogeneic SCT, and continues to be a major obstacle to successful SCT even when modern methods of transplantation, including transplantation, post low intensity conditioning or non-myeloablative regimens, are employed. One experimental model for studying acute GVHD is the semi-allogeneic C57BL/6 to (C57BL/6xBalb/c)F1 mouse model, in which GVHD can be generated by infusing 2x10⁷ splenocytes from C57BL/6 donor mice into (C57BL/6 x Balb/c)F1 recipient mice that received 7Gy ⁶⁰Co total body irradiation (TBI) prior to transplantation [Nagler A, Ohana M, Alper R, Doviner V, Sherman Y, Rabbani E, Engelhardt D, and Ilan Y. Induction of oral tolerance in bone marrow transplantation recipients suppresses graft versus host disease in a semi allogeneic mouse model. Bone Marrow Transplantation, 2003, (in press)]. We have recently shown in this model that induction of oral immuneregulation by pre-transplantation feeding of the donor with recipient splenocytes alleviates acute GVHD, manifested by suppression of in vitro alloreactivity, improved survival, reduced lymphocytic infiltration and other typical histopathological GVHD manifestations in target organs.

Disclosed and described, it is to be understood that this invention is not limited to the particular examples, methods steps, and compositions disclosed herein as such methods, steps and compositions may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following examples are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### EXAMPLES

### I.

### Materials and Methods

### Animals

Normal inbred 2 to 4 month old C57BL male mice were obtained from Harlan and maintained in the Animal Core of the Hadassah-Hebrew University Medical School. Mice were maintained on standard laboratory chow and kept in 12-hour light/dark cycles.

### Induction of Colitis

TNBS-colitis was induced by rectal instillation of TNBS, 1 mg/mouse, dissolved in 100 ml of 50% ethanol as described. [Collins, C., et al., Eur. J. Immunol. 26:3114-3118 (1996)].

### Preparation and Administration of the Oral Antigen

Colons were removed from TNBS-induced-colitis mice, cut into small strips, and mechanically homogenized. After filtration through a 40 mm nylon cell strainer, intact cells were spun down and removed. Proteins were quantified by using a protein assay kit (Biorad, Munich, Germany). Colitis extracted proteins (CEP) were introduced into the experimental groups described below by using a feeding atraumatic-needle every other day for 11 days (a total of 5 doses).

### NK1.1 Cell Depletion

Depletion of NK1.1 + cells was performed by using mouse anti-mouse NK1.1 monoclonal antibody (Serotec, Oxford, UK) as previously described [Kawamura, T., et al., J. Immunol. 160:16-19 (1998)]. Mice were injected with 50 µg/day IP 36 hours before splenocyte harvesting from donor mice.

### Adoptive Transfer of Lymphocytes

Donor mice from all groups were sacrificed 14 days after induction of colitis and single suspensions of lymphocytes derived from spleens were prepared as described [Weiner, H., et al., Annu. Rev. Immunol. 12:809-837 (1994)]. Cells were re-suspended in PBS before transplantation. Splenic lymphocytes from all groups were transplanted into naive recipient mice, followed 24 hours later by rectal challenge with TNBS.

### Evaluation of the Effect of Tolerance Induction on Experimental Colitis

The effect of tolerance induction was evaluated by monitoring the following parameters for colitis:

### Clinical Assessment of Colitis:

Diarrhea was followed daily throughout the study.

### Macroscopic Score of Colitis

Colitis assessment was performed 14 days following colitis induction using standard parameters [Madsen, K.L., et al., Gastroenterology 113:151-159 (1997); Trop, S., et al., Hepatology 27:746-755 (1999)].

Four macroscopic parameters were determined, namely: degree of colonic ulcerations; intestinal and peritoneal adhesions; wall thickness; and degree of mucosal edema. Each parameter was graded on a scale from 0 (completely normal) to 4 (most severe) by two experienced blinded examiners.

### Grading of Histological Lesions

For histological evaluation of inflammation, distal colonic tissue (last 10 cm) was removed and fixed in 10% formaldehyde. Five paraffin sections from each mouse were then stained with hematoxylin-eosin by using standard techniques. The degree of inflammation on microscopic cross sections of the colon was graded semiquantitatively from O to 4 [Madsen *et al.,* (1997) *ibid.;* Trop et al., Hepatology 27:746-755 (1999)]. Grade 0: normal with no signs of inflammation; Grade 1: very low level of leukocyte infiltration; Grade 2: low level of leukocyte infiltration; and Grade 3: high level of infiltration with high vascular density, and bowel wall thickening; Grade 4: transmural infiltrates with loss of goblet cells, high vascular density, wall thickening, and disruption of normal bowel architecture. The grading was performed by two experienced blinded examiners.

### Evaluation of the Role of NK1.1 Lymphocyte on Tolerance Induction in the Experimental Colitis Model

### Liver and Spleen Lymphocyte isolation

Splenocytes were isolated and red blood cells removed as previously described [Vicari, A.P., et al., Immunology Today 17(2):71 (1996)]. Intrahepatic lymphocytes were isolated from all groups of mice at the end of the study, as previously described, with some modifications [Vicari *et al.,* (1996) *ibid.;* Bleicher, P.A., et al., Science 250:679-682 (1990)]. The inferior vena cava was cut above the diaphragm and the liver was flushed with 5 ml of cold PBS until it became pale. The connective tissue and the gal Ibladder were removed, and livers were place in a 10-ml dish in cold sterile PBS. Livers and spleens were crushed through a stainless mesh (size 60, Sigma Chemical Co., St. Louis MO). Cell suspension was placed in a 50 ml tube for 3 minutes and washed twice in cold PBS (1,250xrpm for 10 minutes), and debris was removed. Cells were re-suspended in PBS, cell suspension was placed through a nylon mesh presoaked in PBS, and unbound cells were collected. Cells were washed twice in 45 ml PBS (1,250xrpm in room temperature). For liver and spleen lymphocyte isolation 20 ml of histopague 1077 (Sigma Diagnostics, St. Louis, MO) were slowly placed underneath the cells suspended in 7 ml of PBS, in a 50-ml tube. The tube was centrifuged at 1,640 rpm for 1 5 minutes at room temperature. Cells at the interface were collected, diluted in a 50-ml tube, and washed twice with ice-cold PBS (1,250 rpm for 10 minutes). Approximately 1x10⁶ cells/mouse liver were recovered. The viability by trypan blue staining was more than 95%. Both splenocytes and liver-associated lymphocytes were isolated from all animals in all experimental groups.

### Flow Cytometry Analysis for Determination of NK1.1⁺ Lymphocyte Depletion

Immediately following lymphocyte isolation, triplicates of 2-5x10⁴ cells/500µl) PBS were put into Falcon 2052 tubes incubated with 4 ml of 1% BSA for 10 minutes, and centrifuged at 1400 rpm for 5 minutes. Cells were resuspended in 10µl FCS with 1 :20 FITC-anti mouse NK1.1 antibody (NKR-P1C, Pharmingen, USA), and mixed every 10 minutes for 30 minutes. Cells were washed twice in 1% BSA, and kept in 4°C until reading. For the control group, only 5µl of 1% BSA was added. Analytical cell sorting was performed on 1x10⁴ cells from each group with a fluorescence-activated cell sorter (FACSTAR plus, Becton Dickinson). Only live cells were counted, and background fluorescence from non-antibody-treated lymphocytes were deducted from the levels obtained. Gates were set on forward-and side-scatters to exclude dead cells and red blood cells. The data were analyzed with Consort 30 two-color contour plot program (Becton Dickinson, Oxnard, CA), or the CELLQuest program.

### Splenocyte and Liver-Associated Lymphocyte Cultures

Splenocytes and liver-associated-lymphocytes were harvested from mice in all groups (A' to F') and cultured in 24 well tissue culture plates. Triplicates were prepared from each animal in all study groups and cultured for 12 hours. Lymphocytes were activated in cell dishes 1 x 10⁶ splenocytes/ml RPMI 1640 with Con A 2 µg/ml and 2 µM monensin (Biosource, CA) required to prevent cytokines from being released from cells for 12 h at 37°C in 5%. The RPMI medium contains: 10% FCS, 200 mM Hepes, 100 U of penicillin and 100 Mg of streptomycin/ml, 10 mM Hepes IL2-10 U/ml, CEP-50 Mg/ml. Cells included 2.5x10⁶ splenocytes and 0.5x10⁶ LAL, with Monensin 2µM (Biosource, CA). Supernatant fluids were collected from both sets for cytokine measurements by ELISA, and lymphocytes were analyzed by flow cytometry as described [Collins, C., et al., Eur. J. Immunol. 26:3114-3118 (1996)].

### Intracellular Staining and Flow Cytometry

Cells were harvested from all wells and double stained. Extracellular and intracellular staining to detect CD4⁺ T-cell populations (Th1 and Th2 cells) were used as previously described using the following antibodies: FITC conjugated anti CD4, and PE-conjugated anti IL4 mAb were used for detection of CD4 + IL4+ cells (PharMingen, San Diego, CA). FITC conjugated anti CD4 and PE-conjugated anti IFNγ mAb were used for detection of CD4+ IFNγ cells (PharMingen, San Diego, CA). All was done according to the manufacturer's instructions (IC screen, Biosource intracellular staining kit, CA). Lymphocytes were analyzed by flow cytometry.

### Liver Lymphocyte Cytotoxicity Assays

The target cells used in these studies were YAC-1 cells, a lymphoma cell line adapted to continuous growth in tissue culture by employing supplemented RPMI with 10% FCS. YAC-1 cells were prepared for NK assay by seeding them at a density of 2x10⁵ cells/ml in 25 ml flasks with RPMI 10% FCS, and collecting them 24 hours later. Cells were suspended and collected in a 50 ml tube and washed twice with medium by centrifugation (1250 rpm) for 10 minutes. This procedure ensured efficient labeling with ⁵¹Cr and high sensitivity of lysis by NK cells. Target cells were labeled with ⁵¹Cr (New Life Science, Boston MA, Gamidor, Israel) and incubated for 90 minutes at 37°C (200mCi/2x10⁶ cells in 300µl RPMI medium). Cells were manually mixed every 10 minutes. Following incubation, 3ml of 20% FCS RPMI were added, and reincubated for 30 minutes at 37°C. Cells were washed three times in RPMI 10% FCS and counted. For determination of degree of labeling efficiency, 100µl of cells were counted, and a minimum of 0.6 cpm/cell were measured. Effector cells were liver lymphocytes isolated from livers from groups AH described above. The ⁵¹Cr-release assay was performed in Costar 96-well plates. A graded number of effector cells in 100µl were mixed with 5000 labeled target cells in 100µl, with effector to target ratios (E:T ratio) of 100:1, 50:1, and 10:1. Each well contained target and effector cells in a total volume of 200µl. Five wells were tested for each ratio from each sample. For determination of spontaneous release, 6 wells of a similar number of target cells were plated with 100µl RPMI 10% FCS. For determination of maximum release, 6 wells of target cells in 100µl medium were mixed with 100µl TritonX. The plate was centrifuged for 2 minutes (500 rpm) followed by 4 hours of incubation in 5% C02 at 37°C. The plate was than centrifuged again for 2 minutes (500 rpm), and supernatants were harvested and counted using a gamma counter. Results were expressed as percent specific lysis of target cells calculated by using the equation: % cytotoxicity = mean cpm of assay-cpm from spontaneous release/cpm from targets lysed with TritonX-cpm from spontaneous release x100.

### Cytokine Secretion

Supernatant fluids were collected from both sets of triplicates and cytokine levels were measured for all mice from all tolerized and non-tolerized groups, NK1.1 depleted and non-deleted mice. IL4, IL10, IL12, and IFNγ levels were measured by a "sandwich" ELISA, using Genzyme Diagnostics kits (Genzyme Diagnostics, MA, USA) according to the manufacturer's instructions. Serum levels were measured in 5 mice from tolerized and non-tolerized NK1.1 depleted and non-depleted mice, 10 days after colitis induction.

### In vitro Education Experiments

### Isolation and Separation of Lymphocytes

Splenocytes were prepared and separated into four subsets of lymphocytes, CD4⁺, CD8⁺, NK, and Dendritic cells. Cell separation was done using Magnetic Cell Sorting (MACS). Specific microbeads were used for each subset of lymphocytes: CD4 and CD8 microbeads, and anti-NK beads (Miltenyl Biotec, Germany). Immediately following lymphocyte isolation, triplicates of 2-5x10⁴ cells/5000l PBS were put into Falcon 2052 tubes incubated with 4 ml of 1% BSA for 10 minutes, and centrifuged at 1400 rpm for 5 minutes. Cells were re-suspended in 10µl FCS with 1 :20 FITC-anti mouse NK1.1 antibody (NKR-P1C, Pharmingen, USA), and mixed every 10 minutes for 30 minutes. Cells were washed twice in 1% BSA, and kept in 4°C until reading. For the control group, only 5µl of 1% BSA was added. Analytical cell sorting was performed on 1x10⁴ cells from each group with a fluorescence-activated cell sorter (FACSTAR plus, Becton Dickinson). Only live cells were counted, and background fluorescence from non-antibody-treated lymphocytes was deducted from the levels obtained. Gates were set on forward-and side-scatters to exclude dead cells and red blood cells. The data was analyzed with the Consort 30 two-color contour plot program (Becton Dickinson, Oxnard, CA), or the CELLQuest program.

### Splenocyte and Liver-Associated Lymphocyte Cultures

Splenocytes were harvested from mice in all groups and cultured in 24 well tissue culture plates. Triplicates were prepared from each animal in all study groups and cultured for 12 hours. Supernatant fluids were collected from both sets for cytokine measurements by ELISA.

### Example 1

### The Effect of Tolerance Induction in Experimental Colitis

To evaluate the effect of tolerance induction in the experimental colitis model, six groups of mice, consisting of 20 animals each, were studied (Table 1). All mice were challenged with rectal TNBS (groups A, B, D, and E), or with normal saline (control groups C and F) on day 1 of the study. Mice in all groups were fed (50µg/mouse) every other day for 11 days beginning with the day of colitis induction. Groups B and E included mice fed with colitis extracted proteins (CEP). Mice in groups A, C, D, and F, were fed with bovine serum albumin (BSA, 50µg/mouse). Mice in all groups were sacrificed 14 days following colitis induction. Mice in groups D to F were treated with anti-NK1.1 anti-mouse monoclonal antibodies 36 hours before termination of the study, as described above. Mice in groups A to C were not NK1.1-depleted.

**Table 1**

| **Experimental and Control Groups** | | | |
|---|---|---|---|
| **Group** | **NK1.1 depletion** | **Antigen fed** | **Rectal challenge** |
| A | - | BSA | TNBS |
| B | - | CEP | TNBS |
| C | - | BSA | NS |
| D | + | BSA | TNBS |
| E | + | CEP | TNBS |
| F | + | BSA | NS |

BSA: Bovine Serum Albumin
CEP: Colitis Extracted Protein
TNBS: 2,4,6,-Trinitrobenzene Sulfonic Acid

### Clinical Assessment of Colitis

A marked decrease in diarrhea was observed in tolerized mice from groups B and D fed with mouse-CEP or NK1.1-depleted respectively. In contrast, mice from groups A and E, fed with BSA or fed with mouse-CEP and NK1.1-depleted, suffered severe diarrhea. A follow up of mice body weight disclosed a statistically significant increase in body weight among tolerized mice in groups B and D, as compared with mice in groups A and E (13.5% and 11.65% vs. 3.2% and 4.8%, respectively, p<0.005).

### Macroscopic Grading of Colitis

Induction of oral tolerance by the feeding of mouse extracted colitis-derived proteins or NK1.1-depletion (groups B and D), markedly alleviated the macroscopic grading of colitis. The scores for tested macroscopic parameters of colitis were: degree of colonic ulceration, intestinal and peritoneal adhesions, wall thickness, and degree of mucosal edema. The total macroscopic score was 0.35±0.01 and 0.63±0.03 in groups B and D mice respectively, compared with 3.1 ± 0.54 and 3.05 ± 0.67 in the non-treated control and CEP-fed-NK1.1-depleted groups A and E respectively (p < 0.005).

### Grading of Histological Lesions

Histologic evaluation of bowel tissue showed a marked reduction in inflammatory response and mucosal ulcerations in tolerized or NK1 .1-depleted mice in groups B and D, as compared with non-tolerized mice in groups A and E. In mice in groups B and D, almost normal sections, or only minimal lymphocytic infiltration, was detected. In contrast, severe inflammatory reaction (grade 3-4) was observed in bowel specimens taken from non-tolerized mice (Fig. 1).

### Example 2

### NK1.1 + Lymphocytes Increase the CD4+IL4+/CD4+IFNγ+ Ratio in Tolerized Mice and Decreased the CD4+IL4+/CD4+IFNγ+ Ratio in Non-Tolerized Mice With Experimental Colitis

### Tolerized Mice

To study the effect of NK1.1 + lymphocytes in tolerized mice, splenocytes and liver-associated-lymphocytes (2.5x10⁶ splenocytes and 0.5x10⁶ LAL) were harvested from mice in all groups and cultured for 72 hours in the presence of CEP and APC. Flow cytometry analysis have shown that NK1.1-depletion following oral tolerance induction decreased the CD4+IL4+/CD4+IFNγ+ ratio in comparison with the non-NK1.1 LAL depleted tolerized mice (0.99 ± 0.03 vs. 1.8 ± 0.35 CD4+IL4+/CD4+IFNγ+, in groups E and B respectively, p < 0.005, Fig. 2). The control NK1.1-depleted group (group F) disclosed a decrease in CD4+IL4+/CD4+IFNγ+ ratio compared with non-NK1.1-depleted group C (2.13 ± 0.36 vs. 1.6 ± 0.29, for groups C and F respectively).

### Non-Tolerized Mice

In contrast to tolerized groups, NK1.1-depletion had an opposite effect on non-tolerized mice with experimental colitis The CD4⁺IL4/CD4⁺ IFNγ ratio increased in NK1.1-depleted non-tolerized groups, as compared with the non-NK1.1 depleted non-tolerized group (0.74 ± 0.06 vs. 0.56 ± 0.05 in groups A and D respectively, p < 0.005, Fig. 3).

A comparison of the CD4 + IL4 + /CD4 + IFNγ + ratio between tolerized and non-tolerized mice revealed a higher ratio in all tolerized groups. Mice treated with TNBS and orally fed with CEP (group B) showed a significantly higher ratio, as compared with non-tolerized mice fed with BSA (group A). CD4 + IL4 + /CD4 + IFNγ + ratio in groups A, B, and C were: 0.56 ± 0.05, 1.8 ± 0.35 and 2.13 ± 0.36 respectively (p<0.005). Fig. 4 shows the representative results of expression of IL4 and IFNγ on isolated lymphocytes from tolerized NK1.1 non-depleted and depleted mice from groups B and E, and non-tolerized NK1.1 non-depleted and depleted mice from groups A and D, respectively.

### Example 3

### The Role of In-Vitro Sensitization and the Effect of Disease-Target-Antigen on CD4+IL4+/CD4+IFNγ+ Ratio in Tolerized and Non-Tolerized Mice with Experimental Colitis

For evaluation of the effect of *in vitro* exposure to the disease-target antigen on the CD4+IL4+/CD4+IFNγ+ ratio Splenocytes and liver-associated-lymphocytes (2.5x10⁶) splenocytes and (0.5x10⁶) LAL were harvested from mice in all groups (listed in Table 1), and cultured for 12 hours, in the presence of Con A and in the absence of CEP and APC. An evaluation of the effect of NK1.1 depletion in the absence of antigen was similar to that found in the presence of antigen. Lymphocytes harvested from tolerized mice in group B revealed a significantly higher CD4 + IL4 + /CD4 + IFNγ + ratio, as compared with NK1.1-depleted mice in tolerized group E (0.7±0.02 vs. 1.1 ± 0.02, respectively, p<0.005). In contrast, NK1.1 depletion induced an increase in the CD4 + IL4 + /CD4 + IFNγ + ratio in non-tolerized mice from groups A and D in the absence of antigen (1.21 ± 0.03 vs. 0.96 ± 0.01, respectively, p < 0.005, Table 2, Fig. 5). These results suggest that immune education was achieved *in vivo* and was not affected by cell-antigen incubation *in vitro.*

Similarly, flow cytometry analysis has shown that the CD4+IL4+/CD4+IFNγ+ ratio decreased significantly in tolerized mice in groups B and E and in control groups C and F, and increased significantly in non-tolerized mice in groups A and D (p<0.005, Fig. 5).

**Table 2**

| **Effect of NK1.1 Depletion and of Disease-Target-Antigen on CD4+IL4+/CD4+IFNγ+ Ratio** | | | | |
|---|---|---|---|---|
| **Group** | **Tolerized** | **NK1.1 Depletion** | **CD4+IL4+/CD4+IF Ny + (with tolerizing antigen)** | **CD4+IL4+/CD4+IF Nγ + (without tolerizing antigen)** |
| A | - | - | 0.56 ±0.05 | 0.96±0.01 |
| B | + | - | 1.8±0.35 | 1.1±0.02 |
| C | Naive | - | 2.13±0.36 | 1.3±0.21 |
| D | - | + | 0.74±0.06 | 1.21±0.03 |
| E | + | + | 0.99±0.03 | 0.7±0.02 |
| F | Naive | + | 1.6±0.29 | 1.33±0.27 |

### Change in Cytokine Levels in Tolerized and Non-Tolerized Mice

Supernatant fluids were collected from both sets of triplicates and cytokine levels were measured for all mice from all tolerized and non-tolerized groups. IL4, and IFNγ levels were measured by a "sandwich" ELISA. Tolerized mice manifested a shift from Th1 to Th2 immune response cytokine secretion. These mice (group B) manifested an increase in IL4 levels and a decrease in IFNγ levels. In contrast, mice from non-tolerized groups (groups A, E) exhibited high IFNγ and low IL4 levels. Lymphocytes harvested from tolerized mice in group B revealed significantly higher IL4, and lower IFNγ levels, as compared with NK1.1-depleted mice in tolerized group E (24.4 ± 1.4 and 14.1 ± 0.4 vs. 22.6 ± 0.7 and 189.8 ± 8.4, respectively, Fig. 6). In contrast, NK1.1 depletion induced an increase in IFNγ and a decrease in IL4 levels in non-tolerized mice from groups A and D, in the absence of antigen (128.3 ± 3.7 and 0.6 ± 0.01 vs. 48.3 ± 4.1 and 19.1 ± 0.4, respectively, Fig. 6). NK1.1 depletion led to an increase in IL12 levels in the CEP-fed groups (475 ± 23.3 vs. 145 ± 5.7 and, for groups E, respectively, Fig. 7) but had an opposite effect in the non-CEP fed groups (165 ± 7.4 and 74 ± 3.3, for groups A and D respectively).

### Example 4

### The Effect of Tolerance Induction on Adoptive Transfer of Splenocytes in Experimental Colitis

To evaluate the effect of tolerance induction in the experimental colitis model, six groups of donor mice consisting of 10 animals each were studied (the different groups are listed in Table 3). Colitis was induced in mice from groups G to J by rectal challenge with TNBS. Control mice in groups K and L were challenged with normal saline. Mice in all groups were fed with 50 µg/mouse every other day for 11 days starting on day of colitis induction. Groups I and J included mice fed with colitis extracted protein (CEP). Mice in groups G, H, K and L were fed with bovine serum albumin (BSA 50 µg/mouse). NK1.1 depletion was performed as described above in mice from groups G, I and K 36 hours prior to splenocyte harvesting. Mice in all groups were sacrificed 14 days following colitis induction.

The recipient mice groups G'-L', consisting of 10 animals each were studied as well. Recipient mice were sublethally irradiated with 300 rad total body irradiation, 24 hours before intravenous injection of 1x10⁶ donor cells in 0.5 ml PBS. All mice were treated with TNBS enemas, 24 hours following cell transplantation. Clinical, macroscopic and histological parameters for colitis were determined 14 days following colitis induction as described below.

**Table 3**

| **Experimental and Control Groups** | | | | |
|---|---|---|---|---|
| **GROUP** | **NK1.1 DEPLETION** | **ANTIGEN FED** | **RECTAL CHALLENGE** | **SPLENOCYTE DONORS** |
| **DONORS:** | | | | |
| G | + | BSA | TNBS | - |
| H | - | BSA | TNBS | - |
| I | + | CEP | TNBS | - |
| J | - | CEP | TNBS | - |
| K | + | BSA | NS | - |
| L | - | BSA | NS | - |
| G' | - | - | TNBS' | G |
| H' | - | - | TNBS | H |
| I' | - | - | TNBS | I |
| J' | - | - | TNBS | J |
| K' | - | - | TNBS | K |
| L' | - | - | TNBS | L |

| | | | | |
|---|---|---|---|---|
| BSA: Bovine Serum Albumin CEP: Colitis Extracted Protein TNBS: 2,4,6,-Trinitrobenzene Sulfonic Acid | | | | |

### Clinical Assessment of Colitis

A marked decrease in diarrhea was observed in recipients of tolerized cells from tolerized mice from group J' fed with mouse CEP, as well as in the tolerized mice of group J. In contrast recipients of non-tolerized splenocytes from group H' and mice fed with BSA from group H, suffered severe diarrhea. Follow up of mice body weight disclosed a statistically significant increase in body weights among tolerized mice in groups J and J' compared with non-tolerized mice in groups H and H' (10.8% and 11.2% vs. 5.7 and 5.5%, respectively, p<0.005).

Recipients of splenocytes from NK1.1 depleted-mice from group G' and their donors from group G suffered less diarrhea compared with non-tolerized mice in groups H and H'. Mice from both groups (G and G') showed increase in body weights (9.9% and 10.2 % vs.5.7% and 5.5% respectively, p<0.005). In contrast, recipients of splenocytes from NK1.1-depleted mice from group I' led to loss of the tolerizing effect. A similar effect was observed in their donors (group I). These mice disclosed less diarrhea when compared with groups H and H', however were worse than non-NK1.1 depleted controls. Similarly, no significant increase in body weights was observed in mice in both groups (6.0% and 5.1%, for mice in groups I and I', respectively, p < 0.005, compared with tolerized mice in groups J and J').

Mice from groups K and L were not challenged with TNBS and did not show clinical evidence of disease. Their body weights increased by 11.4% and 12.3% respectively. In contrast, mice from groups K' and L' developed severe diarrhea and their body weights increased only by 4.5% and 5.2% respectively.

### Macroscopic Grading of Colitis

Induction of oral tolerance by the feeding of mouse extracted colitis-derived proteins (group J), and adoptive transfer of tolerized lymphocytes (group J') markedly alleviated the macroscopic grading of colitis. The scores for tested macroscopic parameters of colitis were: degree of colonic ulceration, intestinal and peritoneal adhesions, wall thickness, and degree of mucosal edema. The total macroscopic score was 0.31 ± 0.24 and 0.3 ± 0.25 in groups J and J' respectively, compared with 3.22 ± 0.15 and 3.32 ± 0.26 in non-tolerized mice in groups H and H', respectively. NK1.1 depleted mice from group G and recipients of their lymphocytes from group G' manifested alleviation of disease (0.8 ± 0.4 and 0.85 ± 0.5 respectively). In contrast, NK1.1-depleted mice from group I and recipients of their lymphocytes (group I') manifested severe colitis (3.72 ± 0.22 and 3.77 ± 0.6 respectively, p<0.005). Mice form groups K' and L' showed evidence of severe colitis (3.4 + 0.29 and 3.27 + 0.22, respectively).

### Grading of Histological Lesions

Histologic evaluation of bowel tissue showed a marked reduction in inflammatory response and mucosal ulcerations in tolerized mice in groups J and J', with histological scores of 1.8 and 1.7 respectively. In these mice almost normal sections, or only minimal lymphocytic infiltration, was detected. In contrast, severe inflammatory reaction was observed in bowel specimens taken from non-tolerized mice in groups H and H' with histological scores of 3.3 and 3.08 (groups H and H', respectively, p < 0.005, Fig. 8). A marked reduction in inflammatory response and mucosal ulcerations was detected in non-tolerized NK1.1-depleted mice in group G and recipients of their splenocytes (group G'). The histological scores for groups G and G' were 2.08 and 2 respectively. NK1.1-depleted mice from group I and recipients of their lymphocytes (group I') manifested severe colitis. Scores for mice in groups I and I' were 2.9 and 2.5 respectively. Groups K and L were not rectal challenge with TNBS. Mice form groups K' and L' showed evidence of severe colitis with scores of 3.1 and 3, respectively.

### Example 5

### NK1.1 + Lymphocytes Increase the CD4 + IL4 + /CD4 + IFNγ + Ratio in Tolerized Mice and Decreased the CD4 + IL4 + /CD4 + IFNγ + Ratio in Non-Tolerized Mice with Experimental Colitis.

### Tolerized Mice

A comparison of the CD4+IL4+/CD4+IFNγ+ ratio between tolerized and non-tolerized recipient mice revealed a higher ratio in all tolerized groups. Tolerized recipient mice from group J' showed a significantly higher ratio, as compared with non-tolerized mice in group H'. CD4+IL4+/CD4+IFNγ+ ratio were: 2.16 and 0.55 respectively (p<0.005).

Adoptive transfer of lymphocytes from tolerized mice increased the CD4+IL4/CD4+IFNγ+ ratio in recipients mice. Flow cytometry analysis have shown that adoptive transfer of splenocytes from NK1.1-depleted CEP fed donor mice decreased the CD4+IL4/CD4+IFNγ+ ratio, compared to splenocytes harvested from tolerized non- depleted mice (0.58 vs. 2.16, for groups I' and J' respectively, p < 0.005, Fig. 9).

### Non-Tolerized Mice

Adoptive transfer of non-tolerized lymphocytes decreased the CD4+IL4/CD4+IFNγ+ ratio in recipient mice. In contrast to tolerized groups, NK1.1-depletion had an opposite effect on non-tolerized mice with experimental colitis. Flow cytometry analyses have shown that adoptive transfer of splenocytes from NK1.1-depleted non-tolerized donor mice increased the CD4+IL4/CD4+IFNγ+ ratio compared with splenocytes from non-tolerized non-NK1.1 depleted mice. (1.7 vs. 0.55, in groups G' and H', respectively, p < 0.005, Fig. 9 and Table 4). Fig. 10 shows representative results of expression of IL4 and IFNγ on isolated lymphocytes from recipients of tolerized NK1.1 non-depleted and depleted donors, and from recipients from non-tolerized NK1.1 non-depleted and depleted donors (groups G'-J').

**Table 4**

| **Effect of Adoptive Transfer of Tolerized and Non-Tolerized, NK1.1-Depleted and Non-Depleted Splenocytes CD4 + IL4 + /CD4 + IFNγ + Ratio** | | |
|---|---|---|
| **Recipients:** | **Donors:** | **CD4 + IL4 + /CD4 + IFNγ + ratio** |
| G' | G | 1.7 |
| H' | H | 0.55 |
| I' | I | 0.58 |
| J' | J | 2.16 |
| K' | K | 1.13 |
| L' | L | 0.69 |

### Adoptive Transfer from Control Lymphocytes z

Flow cytometry analyses have shown that adoptive transfer of splenocytes from control NK1.1-depleted mice increased the CD4+IL4/CD4+IFNγ + ratio compared to non NK1.1-depleted donor mice. (1.13 vs. 0.69, groups K' and L' respectively, p < 0.005).

### Example 6

### Liver Lymphocytes Cytotoxicity by NK1.1

YAC-1 cells were used as target cells in these studies at an E: T ratio of 100:1, 50:1 lysis and 10:1. Studies were performed using liver lymphocytes isolated from recipients of NK1.1 depleted and non-depleted tolerized and non-tolerized mice. Recipients from non-tolerized non-NK1.1 depleted mice (group H') showed almost no lysis compared to the other groups 12.37% cytotoxicity (100:1 E: T, Fig. 11). Recipients from non-tolerized NK1.1-depleted mice in group G' showed higher lysis than group H' 20.4% vs. 12.37% of cytotoxicity, respectively. Recipients from NK1.1-depleted CEP fed mice from group I' showed lower lysis than non NK1.1 depleted mice in group J' (42.58% vs. 46.98% cytotoxicity, respectively). Recipients from control groups had 23.1% vs. 22.47% cytotoxicity, for mice in group K' compared with Group L' respectively (p < 0.005, Fig. 11).

### Cytokine Assay

Supernatant fluids were collected from both sets of triplicates and cytokine levels were measured for all mice from all tolerized and non-tolerized groups. IL4, IL10, and IFNγ levels were measured by a "sandwich" ELISA. Tolerized mice manifested a shift from Th1 to Th2 immune response cytokine secretion. These mice (group H) manifested an increase in IL4, IL10 levels and a decrease in IFNγ levels. In contrast, mice from non-tolerized groups (groups G, J, K) exhibited high IFNγ and low IL10 levels. Lymphocytes harvested from tolerized mice in group H revealed significantly higher IL4, IL10, and lower IFNγ levels, as compared with NK1.1-depleted mice in tolerized group K (18.4 ± 3.7, 23.1 + 2.9 and 5.1 ± 0.4 vs. 2.9 ± 0.6, 0.8 ± 0.1 and 19.8 ± 3.8, respectively, Fig. 12). In contrast, NK1.1 depletion induced an increase in IFNγ and a decrease in IL4, IL10 levels in non-tolerized mice from groups G and J, in the absence of antigen (24.3 ± 3.7, 3.1 ± 0.9, and 4.6 ± 0.4 vs. 18.3 ± 1.1, 3.2 ± 0.1 and 2.1 ± 0.4, respectively, Fig. 12).

### Example 7

### Ex-vivo Immune Programming of NKT Lymphocytes

As shown by the preceding examples, induction of oral tolerance by feeding of mouse extracted colitis-derived proteins markedly alleviated different symptoms of colitis (macroscopic grading of colitis, severe diarrhea, inflammatory response and mucosal ulcerations) as compared with non-tolerized mice.

Therefore, the present inventors have preformed the following experiment in order to determine the possibility of *in vitrolex-vivo* immune programming of NKT cells by examining whether an *ex-vivo* education of cells, particularly NK cells, may ameliorate different colitis symptoms in animals suffering from induced colitis that were not subjected to any oral tolerance treatment.

Different cell subgroups in eight different combinations (CD4, CD8, splenocytes and Dendritic cells, as listed in Table 5) were prepared from each of the following six experimental groups:
1. Cells harvested from control animals without colitis and without treatment (oral tolerization). These cells were incubated *ex-vivo* with BSA.
2. Cells harvested from control animals with colitis and without treatment (oral tolerization). These cells were incubated *ex-vivo* with BSA.
3. Cells harvested from animals with colitis and with treatment via oral toerization. Cells were incubated *in vitro* with BSA.
4. Cells harvested from control animals without colitis and without treatment (oral tolerization). These cells were incubated *ex-vivo* with CEP.
5. Cells harvested from control animals with colitis and without treatment (oral tolerization). Cells were incubated *ex-vivo* with CEP.
6. Cells harvested from animals with colitis and with treatment (oral tolerization) via oral toerization. Cells were incubated *ex-vivo* with CEP.

**Table 5**

| **Different Experimental Subgroups of Cell Type or Combination** | |
|---|---|
| **Different subgroups** | **Cell type or cell combination** |
| Group A" | CD4 cells |
| Group B" | CD8 cells |
| Group C" | Splenocytes |
| Group D" | Dendritic cells (DC) |
| Group E" | NKT cells |
| Group F" | NKT+ CD4 |
| Group G" | NKT + CD8 |
| Group H" | NKT + DC |

It should be noted that cells from experimental groups 1, 2 and 3 were incubated *in vitro* in the presence of BSA and therefore served as control, whereas cells of experimental groups 4, 5 and 6 were incubated in vitro in the presence of the antigen (CEP) and therefore served as test groups. *ex-vivo* education was examined by measuring secretion of IL10 (as compared to IFN□ secretion) by the different treated cells.

It is to be appreciated that different cell types or cell combinations (subgroups A" to H") which were prepared from animals suffering from colitis that were not treated (oral tolerization) but were incubated *in vitro* in the presence of CEP (subgroups 5A" to 5H"), are the main tested groups indicating the feasibility of *ex-vivo* education by incubation with antigen. As shown by Table 6, culturing NK1.1 + T cells in the presence of disease associated antigens (subgroup E"5) leads to cytokine pattern that is similar to that of tolerized cells as manifested by increase IL10 secretion.

A similar pattern was observed for culturing of CD4 cells and antigen (subgroup A"5). These results indicate successful *ex-vivo* education by exposing cells to antigen associated with the disease. However combining of more than one cell type in the presence of antigen diminished this desired effect, as NKT education by antigen was prevented by the addition of CD4, CD8, or DC (subgroups F"5, G"5 and H"5, respectively).

In addition to feasibility of *ex-vivo* education of NKT cells by incubation with antigen associated with the disease, the inventors have examined whether co-culturing of NKT cells with other cell types may result in the desired *ex-vivo* education as reflected by IL10 elevated secretion. As shown by Table 6, only combination of NKT cells and CD4 or CD8 cells that were obtained from tolerated mice resulted in IL10 elevated secretion (subgroups F3 and G3, respectively). NKT and CD4 cells obtained from tolerated mice combined with *ex-vivo* exposure to antigen had a similar effect (subgroup F6), whereas the antigen presence significantly reduced IL10 secretion when the NKT CD8 from tolerized mice, combination was examined (subgroup G6).

However, co-culturing of NKT cells with dendritic cells failed to induce IL10 secretion in any combination examined (subgroups H3 to H6).

**Table 6**

| **Experimental and Control Groups** | | | | | | |
|---|---|---|---|---|---|---|
| **GROUP** | **TNBS COLITIS** | **ISOLATED LYMPHOCYTES** | **ANTIGEN FED** | **ANTIGEN IN PLATE** | ***IFNγ*** | ***IL10*** |
| A"1 | - | CD4 | BSA | BSA | 4000 | 1450 |
| A"2 | + | CD4 | BSA | BSA | 36 | 200 |
| A"3 | + | CD4 | CEP | BSA | 0 | 53 |
| A"4 | - | CD4 | BSA | CEP | 0 | 0 |
| A"5 | + | CD4 | BSA | CEP | 0 | 270 |
| A"6 | + | CD4 | CEP | CEP | 0 | 66 |
| B"1 | - | CD8 | BSA | BSA | 4000 | 1500 |
| B"2 | + | CD8 | BSA | BSA | 0 | 305 |
| B"3 | + | CD8 | CEP | BSA | 50 | 165 |
| B"4 | - | CD8 | BSA | CEP | 0 | 0 |
| B"5 | + | CD8 | BSA | CEP | 0 | 54 |
| B"6 | + | CD8 | CEP | CEP | 0 | 98 |
| C"1 | - | SPLENOCYTES | BSA | BSA | 0 | 0 |
| C"2 | + | SPLENOCYTES | BSA | BSA | 230 | 160 |
| C"3 | + | SPLENOCYTES | CEP | BSA | 0 | 306 |
| C"4 | - | SPLENOCYTES | BSA | CEP | 0 | 0 |
| C"5 | + | SPLENOCYTES | BSA | CEP | 0 | 34 |
| C"6 | + | SPLENOCYTES | CEP | CEP | 0 | 420 |
| D"1 | - | DC | BSA | BSA | 240 | 120 |
| D"2 | + | DC | BSA | BSA | 4000 | 720 |
| D"3 | + | DC | CEP | BSA | 4000 | 920 |
| D"4 | - | DC | BSA | CEP | 0 | 0 |
| **D"5** | + | DC | BSA | CEP | 140 | 170 |
| **D"6** | + | DC | CEP | CEP | 30 | 280 |
| **E"1** | - | NKT | BSA | BSA | 0 | 0 |
| **E"2** | + | NKT | BSA | BSA | 0 | 52 |
| **E"3** | + | NKT | CEP | BSA | 0 | 230 |
| **E"4** | - | NKT | BSA | CEP | 0 | 14 |
| **E"5** | + | NKT | BSA | CEP | 38 | 340 |
| **E"6** | + | NKT | CEP | CEP | 0 | 60 |
| **F"1** | - | NKT+CD4 | BSA | BSA | 0 | 15 |
| **F"2** | + | NKT+CD4 | BSA | BSA | 150 | 0 |
| **F"3** | + | NKT+CD4 | CEP | BSA | 0 | 360 |
| **F"4** | - | NKT + CD4 | BSA | CEP | 29 | 28 |
| **F"5** | + | NKT + CD4 | BSA | CEP | 0 | 0 |
| **F"6** | + | NKT+CD4 | CEP | CEP | 0 | 300 |
| **G"1** | - | NKT+CD8 | BSA | BSA | 18 | 98 |
| **G"2** | + | NKT+CD8 | BSA | BSA | 0 | 12 |
| **G"3** | + | NKT+CD8 | CEP | BSA | 0 | 350 |
| **G"4** | - | NKT + CD8 | BSA | CEP | 0 | 0 |
| **G"5** | + | NKT+CD8 | BSA | CEP | 0 | 0 |
| **G"6** | + | NKT+CD8 | CEP | CEP | 0 | 19 |
| **H"1** | - | NKT+DC | BSA | BSA | 0 | 100 |
| **H"2** | + | NKT+DC | BSA | BSA | 4000 | 270 |
| **H"3** | + | NKT+DC | CEP | BSA | 0 | 98 |
| **H"4** | - | NKT+DC | BSA | CEP | 0 | 0 |
| **H"5** | + | NKT+DC | BSA | CEP | 0 | 0 |
| **H"6** | + | NKT+DC | CEP | CEP | 44 | 80 |

| | | | | | | |
|---|---|---|---|---|---|---|
| BSA: Bovine Serum Albumin CEP: Colitis Extracted Protein TNBS: 2,4,6,-Trinitrobenezene Sulfonic Acid DC: Dendritic Cells | | | | | | |

The examples of the present invention have shown that adoptive transfer of tolerized splenocytes into naive mice induced tolerance, since it is assumed that Th2 specific memory cells were transferred. In contrast adoptive transfer of lymphocytes from NK1.1 depleted CEP fed mice, failed to transfer the tolerance, and upregulated the inflammatory Th1 mediated response. It was found that NK1.1 + T cells rapidly produce IL4, and play a regulatory role in autoimmune response in the experimental allergic encephalomyelitis and in the diabetic NOD mice models [Bendelac, A., et al., Annu Rev Immunol 15: 535-562 (1997); Sakamoto, A., et al., J Allergy Clin Immunol 103(5 pt 2): s445-51 (1999); Seki, S., et al., J Immunol 147:1214-1221 (1991)]. However depletion of NK1.1 T cells at termination of oral tolerance induction affected the type of cytokine secretion decreasing the CD4 + IL4 + /CD4 +IFNγ ratio compared with tolerized non-depleted NK1.1 T cells mice. The results of the present invention suggest that NK1.1 T cells may influence the Th1/Th2 profile of immune responses via IFNγ pro-inflammatory or via IL4 anti-inflammatory cytokine secretion. In both conditions, their impact is far greater than that by conventional CD4+ T cells [Chen, H. et al., J Immunol 159:2240-2249 (1997)].

Furthermore, the present inventors have further showed that *ex vivo* education of NKT cells is feasible. Since exposure of NKT cells *in vitro* to the disease target antigen enabled education of these cells towards the anti-inflammtory IL10 secretion pattern.

In conclusion, NK1.1 + lymphocytes play a dual role in immune modulation and in switching the immune response in the immunogenic or tolerogenic directions. The environment in which they become activated, different types of stimulations, or signaling receptors may determine their function. It is noteworthy that NK1.1 + T cells which are involved in distinct immunoregulatory mechanisms, and modulate the type of effector cells and the Th1/Th2 paradigm in immune-mediated disorders.

In conclusion, NK1.1 + lymphocytes play a dual role in immune modulation and in switching the immune response in the immunogenic or tolerogenic directions. The environment in which they become activated, different types of stimulations, or signaling receptors may determine their function. It is noteworthy that NK1.1 + T cells which are involved in distinct immunoregulatory mechanisms, and modulate the type of effector cells and the Th1/Th2 paradigm in immune-mediated disorders.

### II.

### Materials and Methods

### Animals

Female immunocompetent (heterozygous) and athymic Balb/C mice were purchased from Jackson Laboratories, Bar Harbor, Maine. All animals were kept in laminar flow hoods in sterilized cages and given irradiated food and sterile acidified water. Animal experiments were carried out in accordance with the guidelines of the Hebrew University-Hadassah Institutional Committee for Care and Use of Laboratory Animals, and with the committee's approval.

### Cell Cultures

The HBsAg secreting human hepatoma cell line Hep-3B was grown in culture as monolayers, in a medium supplemented with non-essential amino acids and 10% heat inactivated fetal bovine serum.

### Tumor and Splenocyte Transplantation in Athymic Mice

Athymic mice were conditioned with sub-lethal irradiation (600cGy). Twenty-four hours after irradiation, the mice were injected subcutaneously into the right shoulder with 10⁷ human hepatoma Hep3B cells. Three days following irradiation, splenocytes were harvested from donor immunocompetent mice. Recipient athymic mice were injected intravenously with donor spleen cells at 1 × 10⁷ cells/mouse, establishing a competent immune system in the recipient. The mice were subsequently injected with antigen pulsed NKT cells.

### Isolation of Lymphocytes and Separation of NKT Cells

Splenocytes were prepared from spleens harvested from donor immune-competent Balb/C mice. After the removal of connective tissue from spleens, they were placed in a 10ml dish in cold sterile PBS and crushed through a stainless steel mesh (size 60, Sigma Chemical Co., St. Louis, MO). For lymphocyte isolation, 20ml of histopaque 1077 (Sigma Chemical Co., St. Louis, MO) was placed underneath the cells suspended in 7ml of PBS, in a 50 ml tube. Cells at the interface were collected, diluted in a 50ml tube and washed twice with ice-cold PBS (1250 rpm for 10 minutes). Approximately 1 × 10⁸ cells/mouse were recovered. NKT cell separation was done using Magnetic Cell Sorting (MACS) with specific anti-NK microbeads (Miltenyl Biotec, Germany).

### NKT Cell Education by Pulsing

NKT cells were educated by *ex vivo* pulsing with tumor or viral-associated antigens. 1 × 10⁶ NKT cells in 0.5 ml of PBS were placed in flasks and incubated with HCC lysate (3µg/ml), Hep3B cells or bovine serum albumin (BSA) (1µg/ml), for 72 hours.

### Adoptive Transfer of NKT Cells

Adoptive transfer was performed seven days after the restoration of immune competence. This was carried out by the intravenous injection of NKT cells that were exposed *in vitro* to HCC lysate, Hep3B cells or BSA.

### Evaluation of the Effect of Adoptive Transfer of Ex-vivo Immune-Modulated Regulatory NKT Lymphocytes

The effect of adoptive transfer of *ex-vivo* educated NKT lymphocytes was evaluated by monitoring the following parameters:

### Follow-up of tumor gro wth

Recipient mice were followed at bi-weekly intervals for six weeks. Survival, body weight and tumor volume (using calipers) were assessed. Mice that showed signs of distress and mice with excessive weight loss (more than 10% between measurements or more than 25% of initial body weight) were sacrificed.

### Cytokine Secretion

During the fourth week, blood was drawn from mice in all groups and centrifuged at 14,000 rpm. Serum cytokine levels were measured by "sandwich" ELISA using Genzyme Diagnostics kits (Genzyme Diagnostics, MA, USA).

### Isolation of liver and spleen lymphocytes and flow cytometry determination of CD4+, CD8 + and NKT cells

Isolation of lymphocytes from liver and spleen was performed as described above and triplicates of 2 x 10⁴ cells/500µl PBS were placed into Falcon 2052 tubes. The cells were resuspended in 10µl fetal calf serum (FCS) with 1:20 FITC anti-mouse NK1.1 antibody (NKR-P1C, Pharmigen, U.S.A.) and mixed every ten minutes for thirty minutes. The cells were washed twice in 1 % BSA and kept in 4° C until reading. Analytical cell sorting was performed on 1 × 10⁴ cells from each group with a fluorescence activated cell sorter (FACStar^{plus}, Becton Dickinson, CA). Only live cells were counted, and background fluorescence from non-antibody treated lymphocytes was deducted from the levels obtained. Data was analyzed with Consort 30 two-color countour plot program (Beckton Dickinson, CA).

### Western blot analysis for STA T 1-6

For the determination of STAT protein expression, splenocytes were obtained from mice in all groups. Tissue homogenates (200 mg/ml) were prepared in 0.25M sucrose/10 mM Tris-HCl, pH 7.4 using a glass homogenizer fitted with a motor-driven Teflon pestle. Proteins (100 µg/lane) were resolved by electrophoresis on SDS-polyacrylamide (7.5%) gels and electroblotted to nitrocellulose membranes, For the detection of the STAT proteins, the membranes were probed with a polyclonal rabbit anti-mice antibody directed at the different STAT proteins, followed by alkaline phosphatase-coupled goat anti-rabbit IgG (Bethyl Lab., Montgomery, TX).

### Example 1

### The Effect of Adoptive Transfer of Ex-vivo Immune-Modulated Regulatory NKT Lymphocytes

To evaluate the *in vivo* anti tumor effect of the adoptive transfer of educated NKT cells, four groups of athymic Balb/C mice (Groups A-D), consisting of 10 animals each, were studied (Table 1). All the mice were sublethally irradiated and transplanted with human Hep3B HCC. NKT cells prepared from immunocompetent Balb/C mice were pulsed *ex-vivo* with HCC-derived antigens (HCC lysate), Hep3B cells, and BSA. 1 × 10⁶ of educated NKT cells were subsequently injected into each HCC harboring mouse by adoptive transfer. Group A received NKT cells pulsed with HCC lysate; Group B received NKT cells pulsed with Hep3B cells; Group C received NKT cells pulsed with BSA; and Group D mice did not undergo NKT transplantation.

**Table 1:**

| **Experimental and Control Groups** | | |
|---|---|---|
| **Group** | **Transplanted cells** | **Pulsing antigen** |
| **A** | NKT | HCC lysate |
| **B** | NKT | Hep3B cells |
| **C** | NKT | BSA |
| **D** | None | HCC lysate |

To determine the mechanism of anti-tumor effect, intrasplenic lymphocyte populations were analyzed by FACS for NKT, CD4 and CD8 markers. Tumor size and weight, serum cytokine levels and splenocyte STAT 1-6 protein expression were also assessed.

### Results

Adoptive transfer of NKT cells pulsed with HCC-derived antigens (Group A) resulted in the complete disappearance of tumors within four weeks, and attenuated weight loss (6.5%). In contrast, mice in Group B, Group C and Group D. developed large, necrotic tumors and severe weight loss (21%, 17%, and 23% weight loss in Group B, Group C and Group D, respectively, p<0.05); consequently, survival could not be assessed.

NKT/CD4 and CD8/CD4 ratios were significantly increased in Group A (12.3 vs. 6.4, 4.8 and 5.6 in Group B, Group C and Group D, for NKT/CD4 ratio, respectively, p<0.05). Expression of the transcription factor STAT4 was significantly increased in Group A, But not in Groups B-D. Serum levels of IFNγ were increased in Group A compared with Groups B, C and D (3.24, 1.77 and 1.38 timesfold, respectively, p<0.05)

Adoptive transfer of NKT lymphocytes pulsed *ex-vivo* with HCC-derived antigens lead to suppression of HCC in mice. NKT mediated anti-tumor activity was associated with enhanced Th1 immunity , manifested by increased anti-tumor NKT and CD8 lymphocyte numbers, increased expression of STAT 4, a marker for IL-2 activity, and elevated serum pro-inflammatory cytokine levels. *Ex-vivo* modulation of NKT lymphocytes holds promise as a novel mode of immune therapy for HCC.

### III.

### Materials and Methods

### Reagents

Concanavalin A was purchased from Worthington Biochemical Corporation, USA.

Anti-HBV vaccine (Bio Hep B) was purchased from Bio Technological General Corporation, USA.

### Animals

Experimental protocols were approved by the Animal studies committee of the Jerusalem Hebrew University Medical School. Ten-week-old male leptin-deficient C57BL/6J mice and their lean littermates (+/?) were purchased from the Harlan laboratories. The animals were housed at controlled temperature at the animal Core of the Hadassah-Hebrew University Medical School. Mice were kept on regular 1 2 hour light-dark cycles, fed standard mice chow and had access to tap water from bottles. Mice were weighed and food intake recorded every two days. At the end of the experiment mice were sacrificed by cervical decapitation under isoflurane anesthesia.

### Preparation and Administration of the Oral Antigen

Livers were removed from the relevant mice, cut into small pieces, and mechanically homogenized. After filtration through a 40 mm nylon cell strainer, intact cells were spun down and removed. Proteins were quantified by using a protein assay kit (Biorad, Munich, Germany), and were introduced into the experimental groups described below by using a feeding atraumatic-needle every other day for 30 days (a total of 15 doses).

### Transaminase and Trigliceride Measurement

Serum ALT and AST plasma activity were measured using an automated procedure. Serum 220cc blood samples were processed using the Roche Trigliceride GPO-PAP enzymatic essay kit. Samples' trigliceride levels were measured with the Cobas DP-25 spectrophotometer, using a wavelength of 550nm.

### Glucose Tolerance Measurement

For the measurement of glucose tolerance, mice were fed with glucose in an amount of 1 gram per kilogram weight. Following the oral feeding blood was drown from the tail under isoflurane anesthesia at time 0 and then every fifteen minutes for a total period of three hours. Glucose levels were measured with the Elite glucose test strips and glucometer.

### Splenic and Hepatic Lymphocyte Isolation

Splenocytes were isolated and red blood cells removed as previously described [Vicari, A.P., et al., Immunology Today 17(2):71 (1996)]. Intrahepatic lymphocytes were isolated from all groups of mice at the end of the study, as previously described, with some modifications [Vicari *et al.,* (1996) *ibid*.; Bleicher, P.A., et al., Science 250:679-682 (1990)]. The inferior vena cava was cut above the diaphragm and the liver was flushed with 5 ml of cold PBS until it became pale. The connective tissue and the gall bladder were removed, and livers were place in a 10-ml dish in cold sterile PBS. Livers and spleens were crushed through a stainless mesh (size 60, Sigma Chemical Co., St. Louis MO). Cell suspension was placed in a 50 ml tube for 3 minutes and washed twice in cold PBS (1,250×rpm for 10 minutes), and debris was removed. Cells were re-suspended in PBS, cell suspension was placed through a nylon mesh presoaked in PBS, and unbound cells were collected. Cells were washed twice in 45 ml PBS (1,250xrpm in room temperature). For liver and spleen lymphocyte isolation 20 ml of histopague 1077 (Sigma Diagnostics, St. Louis, MO) were slowly placed underneath the cells suspended in 7 ml of PBS, in a 50-ml tube. The tube was centrifuged at 1;640 rpm for 15 minutes at room temperature. Cells at the interface were collected, diluted in a 50-ml tube, and washed twice with ice-cold PBS (1,250 rpm for 10 minutes). Approximately 1×10⁶ cells/mouse liver were recovered. The viability by trypan blue staining was more than 95%. Both splenocytes and liver-associated lymphocytes were isolated from all animals in all experimental groups.

### Adoptive Transfer of Lymphocytes

Donor mice of all relevant groups were sacrificed at day 1 of the adoptive transfer experiments and single suspensions of lymphocytes derived from spleens were prepared as described [Weiner, H., et al., Annu Rev Immunol 12:809-837 (1994)]. Cells were re-suspended in PBS. before transplantation. Splenic lymphocytes from all groups were transplanted into naive recipient mice, without prior irradiation.

### Cytokine Measurement

1. Serum cytokines: cytokine levels were measured for all mice from all tolerized and non-tolerized groups. IFNγ, TGF-ß, TNF, IL4, IL6, & IL10 levels were measured by a "sandwich" ELISA, using Genzyme Diagnostics kits (Genzyme Diagnostics, MA, USA) according to the manufacturer's instructions.
2. Splenic cytokines: 1 million/ml splenocytes were collected as described above. IFNγ and IL10 levels were then calculated, using the Elispot method (Diaclone Research, USA).

### Flow Cytometry Analysis for Determination of the NKT1.1 lymphocyte Population

Immediately following lymphocyte isolation, triplicates of 2-5x10⁴ cells/500µl PBS were put into Falcon 2052 tubes incubated with 4 ml of 1% BSA for 10 minutes, and centrifuged at 1400 rpm for 5 minutes. Cells were resuspended in 10µl FCS with 1 :20 FITC-anti mouse NK1.1 antibody (NKR-P1C, Pharmingen, USA), and mixed every 10 minutes for 30 minutes. Cells were washed twice in 1 % BSA, and kept in 4°C until reading. For the control group, only 5µl of 1 % BSA was added. Analytical cell sorting was performed on 1x10⁴ cells from each group with a fluorescence-activated cell sorter (FACSTAR plus, Becton Dickinson). Only live cells were counted, and background fluorescence from non-antibody-treated lymphocytes were deducted from the levels obtained. Gates were set on forward-scatters and side-scatters to exclude dead cells and red blood cells. The data was analyzed with Consort 30 two-color contour plot program (Becton Dickinson, Oxnard, CA), or the CELLQuest program.

### Stat Western Blot Analysis

Stat 1,3,4,6 levels were estimated using a western blot analysis kit.

### Hepatic MRI Measurement of Fat Content

The hepatic fat content was measured using the technique of double-echo chemical shift gradient-echo magnetic resonance imaging (MRI) sequence that provides in-phase and opposed-phase images in a single acquisition for assessment/quantification of fat in the mice livers. The T1-weighted opposed-phase MRI technique is sensitive for the detection of relatively small proportions of fat in tissues. All MRIs were performed with a 1.5-T system (Signa LX;GE, Milwaukee, USA). Double-echo MR imaging was performed with a repetition time (TR) of 125 msec, double echo times (TEs) of 4 and 6.5 msec, and flip angle of 80°. Imaging parameters included section thickness of 3mm, 13-cm field of view, 256 * 160 matrix, and one signal acquired, with use of a knee coil. Transverse (axial) and coronal images were acquired at the level of the liver with a 3mm section thickness and no intersection gap. Quantitative assessment of signal intensity (SI) measurements of SI changes between in-phase and opposed-phase images was computed as described in previous reports (Mitchell DG et al., Invest. Radiol 26:1041-1052 (1991); Tomohiro N et al., Radiology 218:642-646 (2001)). The SI index was calculated as follows: SI index = (Slᵢₚ-Siₒₚ)/Slᵢₚ, where Slip is SI on in-phase images and Slₒₚ is SI on opposed-phase images. The SI index reflects the fraction of SI loss on opposed phase images compared with the SI on in-phase images.

### Hepatic Histology Examination

For each mouse a single liver segment was fixed in 10% buffered formaldehyde and embedded in paraffin for histologic analysis. Sections (µm) were stained with hematoxylin/eosin and histologic scoring performed.

### Statistical Analysis

Data are expressed as means +/- SEM. Statistical significance between the different groups in regard to the above parameters was calculated using the unpaired student t test. A p-value of less than 0.05 was considered to indicate a statistically significant difference.

### Example 1

### The Effect of NKT1.1 Lympocyte Adoptive Transfer on Glucose Tolerance

To assess the role of NKT1.1 lymphocytes in the metabolic and hepatic derangements observed in the ob/ob NASH model, mice were divided into 5 groups, as shown in Table 1. On day 12 of the experiment, glucose tolerance tests were performed in all mice groups, as described above.

**Table 1**

| **Adoptive Transfer Groups** | |
|---|---|
| Group A | Adoptive transfer of wildtype NKT1.1 lymphocytes to ob/ob mice. |
| Group B | Adoptive transfer of ob/ob NKT1.1 lymphocytes to ob/ob mice. |
| Group C | Adoptive transfer of wildtype splenocytes to ob/ob mice. |
| Group D | No adoptive transfer to ob/ob mice |
| Group E | Adoptive transfer of ob/ob splenocytes to ob/ob mice. |

As shown in our previous experiments, ob/ob mice that were not subjected to immunologic manipulation (Group C) had a severely disturbed glucose tolerance test (see Table 2, and Figure 1). Similarly, ob/ob mice undergoing adoptive transfer with regular ob/ob splenocytes had significantly elevated glucose levels throughout the glucose tolerance test.

**Table 2**

| **Glucose Tolerance Test Results** | | | | | |
|---|---|---|---|---|---|
| | A | B | C | D | E |
| Glucose 0 min. | 146.7 +\-28.59 | 125.8 +\-19.98 | 151.7 +\-104.86 | 211.7 +\-37.49 | 91.3+\-38.30 |
| Glucose 15 min. | 188.9 +\-46.79 | 205.1 +\-37.60 | 232.6 +\-99.15 | 288.2 +\-61.25 | 274 +\-50.24 |
| Glucose 30 min. | 175.4 +\-37.13 | 216 +\-46.44 | 237.3 +\-125.49 | 289.2 +\-79.12 | 289.1 +\-43.64 |
| Glucose 60 min. | 161.1 +\-26.66 | 159.3 +\-36.07 | 201.8 +\-141.82 | 281.6 +\-74.82 | 285.8 +\-85.06 |
| Glucose 90 min. | 165.7 +\-28.48 | 165.9 +\-20.73 | 212.1 +\-130.68 | 288 +\-57.83 | 224.3 +\-62.33 |
| Glucose 120 min. | 156.1 +\-29.68 | 151.5 +\-21.23 | 186.9 +\-119.99 | 247.3 +\-69.06 | 199.6 +\-28.17 |
| Glucose 180 min. | 165.2 +\-49.98 | 130.6 +\-30.11 | 169 +\-105.82 | 190.7 +\-70.04 | 176.9 +\-65.87 |

In contrast, mice injected with either wildtype (Group A) or ob/ob (Group B) NKT1.1 lymphocytes featured a significantly improved glucose tolerance test compared to Group D (P<0.001 for all time periods up to the 120 minute time period). In fact, differences in glucose levels during the glucose tolerance test between each of the ob/ob groups and their lean littermates were not statistically different (P value ranging between 0.20-0.63 throughout the glucose tolerance test). The glucose levels of Group C mice, which were implanted with wildtype splenocytes, were midway between the significantly elevated levels of Group D and Group E mice and the virtually normal levels of Group A and Group B mice.

These results suggest that a decreased number and/or a disturbed function of ob/ob NKT1.1 lymphocytes plays a major role in the development of the glucose intolerance in ob/ob mice. By the replenishing of either wildtype or ob/ob NKT lymphocytes one is able to correct the glucose intolerance in these mice.

### Example 2

### The Effect of NKT1.1 Lympocyte Adoptive Transfer on the Hepatic Fat Content

To assess the effect of NKT1.1 lymphocyte adoptive transfer on the level of steatosis, mice of all groups (see Table 3) underwent an abdominal MRI at the end of the experiment, and the hepatic fat content was estimated according to the methods described above and presented as the SI index.

**Table 3**

| **Hepatic Fat Content- Adoptive Transfer Experiment** | | | | | | |
|---|---|---|---|---|---|---|
| | **In Phase Images** | **IPSD** | **Opposite Phase Images** | **OPSD** | **FAT CONTENT (IP-OP)** | **SI INDEX (IP-OP/IP)** |
| A | 1066.87 | 91.29 | 426.92 | 196.62 | 639 | 0.59 |
| B | 1060.57 | 83.07 | 512.99 | 75.53 | 647 | 0.55 |
| C | 1147.36 | 86.17 | 449.28 | 87.95 | 698 | 0.61 |
| D | 1088.1 | 85.92 | 365.93 | 85.72 | 722 | 0.66 |
| E | 1119.45 | 90.68 | 401.04 | 83.99 | 718 | 0.64 |

Both mice receiving adoptive transfer of wildtype (Group A) and ob/ob (Group B) NKT1.1 lymphocytes showed decreased levels of hepatic fat content by MRI, as shown in Figures 2a and 2b. Although only a small period of time elapsed between the adoptive transfer and the MRI recording (12 days), these differences approached statistical significance, (P=0.063, P=0.008, respectively). This suggests that NKT1.1 lymphocyte depletion or malfunction is involved in the pathogenesis of non-alcoholic steatohepatitis in the ob/ob model, and that their replenishment may correct the steatosis.

### Example 3

### The Effect of NKT1.1 Lymphocyte Adoptive Transfer on Susceptibility to Concanavalin-A Hepatitis

In order to assess the role of NKT1.1 lymphocytes on the previously known resistance of ob/ob mice to Con-A induced hepatitis, mice of all groups (Table 4) received, on day 1 2 of the experiment, in a vein of their tail, an intravenous injection of 200µg of Con-A, dissolved in pyrogen-free saline, for a total volume of 0.1 cc. Twenty-four hours later, all the mice were sacrificed and serum transaminase levels and the histological degree of hepatic inflammation were determined. As shown in Table 4 and Figures 3a and 3b, Group D and Group E mice developed only a mild elevation in hepatic transaminases over their baseline levels in response to Con-A challenge (AST 1.48 & 1.40 times their baseline levels, respectively; and ALT 1.52 & 1.79 times their baseline levels, respectively). In contrast, groups A & B developed a significant elevation in both AST (3.6 & 2.44 times their baseline levels) and ALT (5.2 & 3.46 times their baseline levels) in response to Con A. Group C mice, implanted with wildtype splenocytes, featured a modest elevation of hepatic transaminases midway between those of Group A and Group B, and those of Group D and Group E. The results in this experiment suggest that adoptive transfer of NKT lymphocytes may render ob/ob mice more vulnerable to Con-A hepatitis, possibly by the activation of CD4 lymphocytes involved in Con-A hepatitis.

**Table 4**

| **Average Transaminase Levels in the Adoptive Transfer Groups** | | |
|---|---|---|
| | **AST** | **ALT** |
| **A** | 819 +/-269 | 1077 +/- 468 |
| **B** | 556 +/- 143 | 626 +/- 123 |
| **C** | 524 +/-221 | 530 +/- 166 |
| **D** | 338 +/- 90 | 315 +/-66 |
| **E** | 320 +/- 89 | 371 +/- 135 |

### Example 4

### The Effect of Oral Immune Regulation (by Liver Extract Feeding) on Glucose Tolerance

To evaluate the effect of oral immune regulation on the various metabolic and immunologic components of the NASH model, mice were divided into six groups consisting of fourteen mice each, as depicted in Table 5. Every other day, each group was given either 50µg/mouse of ob/op liver extract, 50µg/mouse of regular littermate liver extract or 50µg/mouse of bovine serum albumin.

**Table 5**

| **Oral Immune Regulation in Mice Groups** | |
|---|---|
| Group A | ob/ob mice fed with c57bl/6 mouse liver extract |
| Group B | ob/ob mice fed with ob/ob mouse liver extract |
| Group C | ob/ob mice fed with bovine serum albumin |
| Group D | C57bl/6 mice fed with c57bl/6 mouse liver extract |
| Group E | C57bl/6 mice fed with ob/ob mouse liver extract |
| Group F | C57bl/6 mice fed with bovine serum albumin |

On day 30, all liver extract feedings (totaling fifteen feedings) were terminated. On day 59, glucose tolerance tests were performed on all mice, as described above. As expected, all groups of ob/ob mice had significantly elevated blood glucose levels after a glucose meal as compared to their lean littermate groups (P<0.001, Table 6). However, Group B and Group C ob/ob mice, which were fed with wildtype and ob/ob liver extract, respectively, developed significantly lower glucose levels in response to oral glucose loading compared to Group C ob/ob mice fed with BSA (P< 0.001), as depicted in Figure 4.

This suggests that immune modulation through oral immune regulation induction alters the metabolic profile of ob/ob mice, improving their glucose tolerance results and rendering them less diabetic.

**Table 6**

| **Average Glucose Levels After Oral Glucose Tolerance Test** | | | | | | |
|---|---|---|---|---|---|---|
| | A | B | C | D | E | F |
| Glucose 0 min. | 87.4 +\-10.3 | 103.8 +\-19.37 | 81 +\-29.74 | 85.6 +\-7.75 | 131.2 +\-9.00 | 78.3 +\-12.74 |
| Glucose 15 min. | 145 +\-22.05 | 150.2 +\-27.13 | 202.7 +\-68.36 | 95.5 +\-7.67 | 101.7+.\-16.87 | 105.9 + \-19.34 |
| Glucose 30 min. | 144.1 +\-23.88 | 144.5 +\-26.18 | 226.9 +\-91.34 | 88.8 +\-11.70 | 86.5 +\-9.44 | 91.7 +\-12.96 |
| Glucose 60 min. | 118.6 +\-28.99 | 120.7 +\-30.82 | 170.2 +\-86.72 | 81.7 +\-10.73 | 81.0 +\-12.44 | 83.1 +\-10.91 |
| Glucose 90 min. | 103.9 +\-20.53 | 98 +\-20.41 | 174.8 +\-66.86 | 83.8 +\-6.73 | 80.7 +\-18.81 | 82.4 +\-16.49 |
| Glucose 120 min. | 96.4 +\-11.49 | 99.6 +\-15.01 | 178.7 +\-74.15 | 80.5 +\-5.58 | 77.5 +\-10.31 | 80.4 +\-10.44 |
| Glucose 1 80 min. | 89.5 +\-15.64 | 94.5 +\-19.14 | 193.7 +\-89.38 | 89.5 +\-9.07 | 84.7 +\-11.94 | 87.5 +\-8.39 |

### Example 5

### The Effect of Oral Immune Regulation Induction by Liver Extract Feeding on the Hepatic Fat Content

To determine the effect of oral immune regulation on hepatic fat content, mice of all six groups underwent an abdominal MRI on day 59 of the experiment (Table 7). Hepatic fat content was determined using the methods described above, and was described as the SI index. All three wildtype mice featured significantly lower hepatic fat content than the ob/ob mice groups (P< 0.001 in all groups), with no effect noted of liver extract feedings (figure 2a). Group A and Group B mice, which were given wildtype and ob/ob liver extract, respectively, showed a significant reduction in the hepatic fat content compared to Group C mice (P=0.03 and P=0.019, respectively). These results are shown in Figure 5a and Figure 5b.

This suggests that oral immune regulation induction through liver extract feedings alters the metabolic profile in a way which results in a reduction in the rate of fat accumulation and NASH in the livers of susceptible mammals.

**Table 7**

| **Calculated MRI Hepatic Fat Content of the Six Mice Groups** | | | | | | |
|---|---|---|---|---|---|---|
| | **In Phase Images** | **SDIP** | **Opposite Phase Images** | **SDOP** | **FAT CONTENT (IP-OP)** | **SI INDEX (IP-OP/IP)** |
| A | 1292.89 | 94.47 | 625.28 | 88.53 | 653 | 0.50 |
| B | 1154.05 | 80.02 | 604.88 | 100.87 | 549 | 0.48 |
| C | 1131.43 | 89.81 | 422.91 | 96.58 | 708 | 0.62 |
| D | 996.1 | 69.96 | 900.93 | 69.11 | 95 | 0.09 |
| E | 1105.29 | 72.12 | 982.05 | 85.25 | 123 | 0.11 |
| F | 1071.65 | 76.88 | 955.26 | 77.68 | 116 | 0.10 |

### Example 6

### The Effect of Oral Immune Regulation Induction by Liver Extract Feeding on Susceptibility to Concanavalin-A Hepatitis

In contrast to wildtype mice, ob/ob (leptin-deficient fatty mice) mice have been previously shown to present a unique resistance to Concanavalin-A induced hepatitis. This was determined from the quantitative and qualitative alteration in the NKT1.1 lymphocyte population in these mice. To determine the effect of oral immune regulation induction by liver extract feeding on the susceptibility to Con-A hepatitis, on the 60^{th} day of the experiment, blood was drawn from the retro orbital plexus of all mice under isoflurane anesthesia, and serum transaminase levels were measured. On the same day, all the mice received an intravenous injection in their tail vein of 200µg of Con-A dissolved in pyrogen-free saline to a total volume of 0.1cc. Twenty-four hours later, all the mice were sacrificed, and serum transaminase levels and histologic degrees of hepatic inflammation were determined.

As previously described, ob/ob mice suffered spontaneous hepatitis (average AST = 227, average ALT = 204) whereas their lean littermates did not (average AST = 37, average ALT = 102). When Group C and Group F mice (ob/ob mice and their lean littermates, given BSA) were subjected to an injection of. 200µg of Con-A, ob/ob mice had a significantly milder increase in AST (average AST increasing to 1.83 times its baseline value) as compared to their lean littermates (average AST increasing to 5.94 times its baseline value), in concert with the previous observations suggesting that ob/ob mice were relatively resistant to Con-A hepatitis. For an unknown reason, ALT levels increased comparably in the ob/ob and lean littermate groups (2.75 and 2.098 times their baseline values, respectively.) There was no effect of liver extract feeding on the degree of transaminase elevation in wildtype mice in response to Con-A administration (AST increasing to 5.56 and 6.67 times its baseline value in Group E and Group F, respectively). This is shown in Figure 6a and Figure 6b.

In contrast, Group A and Group B ob/ob mice, which were fed with wildtype and ob/ob liver extracts, developed a significantly more pronounced elevation in AST (AST increasing to 2.71 and 2.89 times their baseline values, respectively) than ob/ob who were fed with BSA. ALT showed a more modest effect (ALT increasing to 3.1 and 3.72 times their baseline values in Group A and Group B, respectively, in comparison to 2.75 its normal value in Group C). This difference in the degree of susceptibility to Con-A hepatitis is caused by immune modulation induced by liver extract feedings in Group A and Group B, which caused a shift towards more pronounced CD4 lymphocyte mediated hepatic damage in these mice.

### Example 7

### The Response to Hepatitis B Vaccination

In order to verify the profound difference existing between ob/ob mice and their lean littermates in relation to the immunological T cell mediated response to external stimuli, ten ob/ob mice and ten lean littermates were immunized against hepatitis B. 0.4µgrams of bio Hep B vaccine were injected intraperitonealy into all mice on days 1, 14, 21, 25, and 30 of the experiment. On day 30, all the mice were sacrificed and the Anti-HBS antibody titer was measured using standard equipment. In comparison to their lean littermates, ob/ob mice showed an attenuated immune response, featuring significantly lower anti-HBS antibody titers (P=0.027). This is depicted in Table 8 and Figure 7. This validates the impression from previous experiments that ob/ob mice have a profoundly impaired T cell immune response, which may play a major part in the development of the metabolic and immunological phenomena described above in ob/ob mice. Oral tolerance with liver extract and NKT cell adoptive transfer may correct this response.

**Table 8**

| **Average Anti-HBS Titers After HBV Vaccination (Miu/ml)** | |
|---|---|
| **OB/OB** | 105 +/-115 |
| **WILDTYPE** | 303 +/- 364 |

### IV.

### Materials and Methods

In order to assess the putative protective role of NKT cells in GVHD, adoptive transfer of increasing numbers of NKT lymphocytes (0 - 4.5 X 10⁶ cells) to mice transplanted with NKT-depleted splenocytes was performed. Recipient mice were followed for histological parameters of GVHD-associated liver, bowel and cutaneous injury. To determine the mechanism of NKT cell-mediated immune modulation and the role of the liver in tolerance induction, intrahepatic and intrasplenic lymphocytes were isolated and analyzed by FACS for CD4+ and CD8 + subpopulations, and serum cytokine levels were determined.

### Animals

Donor mice were 12-week-old C57BL/6 males, obtained from Jackson Laboratories (Anne Harbor, ME). Recipients were (C57BL/6xBalb/c)F1 female mice. The mice were kept in 1 2 hour light/dark cycles in the Animal Core of the Hadassah-Hebrew University Medical School. All animals were fed regular laboratory chow and imbibed water ad libitum. All animal experiments were approved by the Hebrew-University-Hadassah Institutional Committee for the Care and Use of Laboratory Animals. Following irradiation and splenocyte transplantation, the mice were maintained in laminar flow isolators.

### Lymphocyte Isolation and Separation of NKT Cells

Splenocytes were prepared from spleens harvested from donor C57BL mice. After removal of connective tissue, spleens were placed in a 10 ml dish in cold sterile PBS and crushed through a stainless mesh (size 60, Sigma Chemical Co., St. Louis, MO). For lymphocyte isolation, 20ml of histopaque 1077 (Sigma Diagnostics, St. Louis, MO) was placed underneath the cells suspended in 7ml of PBS, in a 50 ml tube. Cells at the interface were collected, diluted in a 50ml tube and washed twice with ice-cold PBS (1250 rpm for 10 minutes). Approximately 1x10⁸ cells per mouse were recovered. NKT cell separation was done using Magnetic Cell Sorting (MACS) with specific anti-DX5 microbeads (Miltenyl Biotec, Germany).

### Splenocyte Transplantation

To induce GVHD, 2x10⁷ spleen cells from C57BL/6 donor mice were injected intravenously into (C57BL/6xBalb/c)F1 recipient mice. Mice were given ⁶⁰Co whole-body irradiation (7 Gy) prior to transplantation.

### Experimental Groups (n = 8 mice/group)

To assess the putative protective role of NKT cells in GVHD, adoptive transfer of increasing numbers of NKT lymphocytes was performed. Group A mice were transplanted with whole splenocytes, Group B mice were transplanted with NKT depleted splenocytes; Group C, Group D and Group E mice were transplanted with NKT depleted splenocytes to which 0.5, 2.5 and 4.5 X 10⁶ NKT cells were added, respectively. Group F mice did not undergo splenocyte transplantation.

### Grading of Histologic Changes of GVHD

For evaluation of the degree of dermal, hepatic, and intestinal inflammation, tissues were removed from mice in all groups and kept in 10% formaldehyde. Five tissue sections from each mouse were embedded in paraffin, sectioned and stained with hematoxylin-eosin by standard procedure, and examined by experienced pathologists who were unaware of the experimental conditions.

### Flow Cytometery Analysis for the Determination of CD4 + , CD8 + T lymphocytes

Immediately following lymphocyte isolation, triplicates of 2 - 5x10⁶ cells/500µλ PBS were put into Falcon 2052 tubes, incubated with 4 ml of 1% BSA for 10 minutes, and centrifuged at 1400 rpm for 5 minutes. Cells were resuspended in 10µλ FCS with 1:20 FITC-anti mouse CD4 and CD8 antibodies (Pharmingen, USA) and mixed every 10 minutes for 30 minutes. Cells were washed twice in 1% BSA, and kept in 4°C until reading. Analytical cell sorting was performed on 1x10⁴ cells from each group with a fluorescence-activated cell sorter (FACSTAR plus, Becton Dickinson). Only live cells were counted, and background fluorescence from non-antibody-treated lymphocytes was subtracted from the levels obtained. Gates were set on forward-scatters and side-scatters to exclude dead cells and red blood cells. Data was analyzed with either the Consort 30 two-color contour plot program (Becton Dickinson, Oxnard, CA), or the CELLQuest 25 program.

### Measurement of Cytokine Levels

Blood was drawn from mice in all groups and centrifuged at 14,000 rpm. Serum IFNγ, TNFα, IL-10 and IL-12 levels were measured by "sandwich" ELISA using Genzyme Diagnostics kits (Genzyme Diagnostics, MA, USA).

### Statistical Analysis

The results were analyzed by the *Student t test* (two-tailed).

**Effect of adoptive transfer of NKT cells on GVHD associated tissue injury:**

### Example 1

### Alleviation of GVHD of the Skin

Skin biopsies were performed in mice from all groups. The epidermis in GVHD showed diffuse vacuolization of the basal cell layer, spongiosis and dyskeratotic keratinocytes and subepidermal cleft formation; morphological changes characterizing grade II of acute GVHD. In some, subepidermal cleft formation with focal complete loss of the epidermis were observed, compatible with grade III-IV of acute GVHD. Adoptive transfer of NKT cells ameliorated these changes.

### Example 2

### Alleviation of Small Bowel GVHD

Small bowel biopsies were performed in mice from all groups. Significant attenuation of all GVHD-related histologic parameters was observed in mice transplanted with NKT cells. In controls, apoptotic bodies (single cell necrosis), characteristic of grade I acute GVHD, were seen in many crypts. In some specimens, necrotic debris was present in bowel crypts, compatible with grade II acute GVHD.

### Example 3

### Amelioration of GVHD Associated Liver Disease

In mice transplanted with NKT cells, mild degrees of portal inflammation, lymphocyte infiltration and disruption of intrahepatic bile ducts were noted. In contrast, NKT depleted mice developed severe non-suppurative cholangitis accompanied by endothelialitis; the latter was evident as damage to the venous endothelium, lymphocytic infiltration and sloughing.

### Example 4

### Effect of Adoptive Transfer of NKT Cells on GVHD Associated Mortality

Adoptive transfer of 4.5 X 10⁶ NKT cells significantly improved survival (85% survival on the 28^{th} day). In contrast, depletion of NKT cells led to a high rate of mortality (100% mortality on the 14^{th} day). A direct correlation with the number of transplanted NKT cells was noted (maximum effect with transplantation of 4.5 X 10⁶ NKT cells). These results are shown in Figure 8.

### Example 5

### Effect of Adoptive Transfer of NKT Cells on Intrahepatic CD8 Lymphocyte Trapping as a Measure of Peripheral Tolerance Induction

Tolerance induction was associated with intrahepatic trapping of CD8 lymphocytes, manifested by a 2.26 time fold increase in the peripheral CD4/CD8 ratio, and a simultaneous 1 6 time fold decrease in the intrahepatic CD4/CD8 ratio in mice transplanted with 4.5 X 10⁶ NKT cells compared with NKT-cell depleted mice (p <0.05). These results are shown in Figure 9 and Figure 10.

### Example 6

### Effect of Adoptive Transfer of NKT Cells on Serum Cytokines

Serum IL-12 level was significantly lower (52 pg/ml vs. 735 pg/ml) and serum IL-10 levels significantly higher (112 pg/ml vs. 50 pg/ml) in tolerized mice transplanted with 4.5 X 10⁶ NKT cells compared with NKT-cell depleted animals (p<0.05). There was no significant difference in serum IFNγ and TNFα levels between the groups. These results are depicted in Figure 11 and Figure 12.

### Conclusions

Adoptive transfer of 4.5 X 10⁶ NKT cells significantly alleviated GVHD-related hepatic, bowel, and cutaneous injury. In contrast, depletion of NKT cells led to severe GVHD-associated multi organ injury.

The transplantation of small numbers of regulatory NKT cells led to amelioration of GVHD-related liver, bowel, and skin injury by facilitating the development of graft-host tolerance. This effect was associated with modulation of effector cell subsets and serum cytokines towards a Th2 type immune response. The liver plays an important role in tolerance induction via lymphocyte trapping. Transplantation of NKT cells holds promise as a novel therapeutic measure for GVHD.

## Claims

1. The use of a therapeutic composition that manipulates the NKT cell population in a subject for the treatment of immune-related or immune-mediated disorders in a mammalian subject in need of such treatment, wherein the result of administration of said composition comprises the modulation of the Th1/Th2 cell balance towards an inflammatory response, said modulation being mediated by different components, cells, tissues or organs of said subject or another subject.

2. The use of a therapeutic composition that manipulates the NKT cell population in a subject for the treatment of immune-related or immune-mediated disorders in a mammalian subject in need of such treatment, wherein the result of administration of said composition comprises the modulation of the Th1/Th2 cell balance towards an anti-inflammatory or pro-inflammatory response, said modulation being mediated by different components, cells, tissues or organs of said subject' s or another subject' s immune system.

3. The use of a therapeutic composition that manipulates the NKT cell population in a subject for the treatment of immune-related or immune-mediated disorders in a mammalian subject in need of such treatment, wherein the result of administration of said composition comprises the modulation of the Th1/Th2 cell balance toward anti-inflammatory cytokine producing cells, said modulation being mediated by different components, cells, tissues or organs of said subject's or another subject's immune system.

4. The use of claim 1, 2 or 3, wherein said components comprise cellular immune reaction elements, humoral immune reaction elements and cytokines.

5. The use of claim 3, wherein said manipulation is performed by depletion of said NKT cell population.

6. The use of claim 3, wherein the therapeutic composition comprises educated NKT cells obtained from a subject, that were ex vivo educated such that the resulting educated NKT cells may modulate the Th1/Th2 cell balance toward pro-inflammatory cytokine producing cells; and that when the educated NKT cells are reintroduced to said subject, the composition may modulate the Th1/Th2 cell balance toward pro-inflammatory cytokine producing cells, resulting in a decrease in the quantitative ratio between any one of IL4 and IL10 to IFNy.

7. The use of claim 6, wherein the ex vivo education step is performed by culturing said NKT cells in the presence of any one of:
a. antigens or epitopes associated with said immune-related or immune-mediated disorder or disease to be treated, antigens or epitopes associated with the immune-mediated inflammatory response, or any combination thereof;
b. at least one liver-associated cell of tolerized or non-tolerized subjects suffering from said immune-related or immune-mediated disorder or of said subject;
c. at least one cytokine, or adhesion molecule, or any combination thereof; and
d. a combination of any of (a), (b), (c).

8. The use of claim 7, wherein said ex vivo education is performed by culturing said NKT cell in the presence of antigens associated with said immune-related or immune-mediated disorder.

9. The use of claim 8 wherein said antigens comprise allogeneic antigens from donors suffering from said immune-related or immune-mediated disorder or disease, xenogeneic antigens, syngeneic antigens, autologous antigens, non-autologous antigens, or recombinantly prepared antigens, or any combination thereof.

10. The use of claim 7, wherein said liver-associated cells comprise Kupffer cells, Stellate cells, liver endothelial cells, liver-associated stem cells, an apolipoprotein, or any other liver-related lymphocytes.

11. The use of claim 7, wherein said cytokines comprise IL4, IL 10, TGFB, IFNy, IL2, IL18, IL12 or IL15.

12. The use of claim 7, wherein said adhesion molecules comprise integrins, Selectin or ICAM.

13. The use of claim 6, wherein said educated NKT cells are re-introduced to said subject by adoptive transfer.

14. The use of claim 6 or 7, further comprising the step of eliciting in said subject immune modulation of the immune-related or immune-mediated disorder by administering to said subject components, cells, tissues and/or organs derived from any allogeneic donor suffering from said immune-related or immune-mediated disorder, xenogeneic sources, syngeneic sources, autologous sources, non-autologous sources, or immunologically functional equivalents, or any combination thereof.

15. The use of claim 14, wherein said components, cells, tissues or organs are administered orally.
